(19) Europäisches Patentamt
European Patent Office
Office européen des brevets

(11) **EP 3 936 124 A1**

(12) **EUROPEAN PATENT APPLICATION**
published in accordance with Art. 153(4) EPC


(43) Date of publication:
**12.01.2022 Bulletin 2022/02**

(21) Application number: **20762380.2**

(22) Date of filing: **28.02.2020**

(51) Int Cl.:
***A61K 31/352*** *(2006.01)* ***A61P 25/00*** *(2006.01)*

(86) International application number:
**PCT/KR2020/002907**

(87) International publication number:
**WO 2020/175962 (03.09.2020 Gazette 2020/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.02.2019 KR 20190024024**



(71) Applicant: **Glaceum, Inc.**
**Gyeonggi-do 16675 (KR)**

(72) Inventors:
- **YOO, Sang Ku**
**Suwon-si Gyeonggi-do 16223 (KR)**
- **KIM, Ji Young**
**Seoul 06547 (KR)**
- **JO, In Geun**
**Cheonan-si Chungcheongnam-do 31099 (KR)**
- **KIM, Kyung Il**
**Suwon-si Gyeonggi-do 16512 (KR)**
- **PARK, Yun Sun**
**Suwon-si Gyeonggi-do 16674 (KR)**

(74) Representative: **Nony**
**11 rue Saint-Georges**
**75009 Paris (FR)**


(54) **PHARMACEUTICAL COMPOSITION FOR PREVENTING OR TREATING NEUROLOGICAL DISEASES**


(57) The present disclosure relates to a pharmaceutical composition for preventing or treating neurological disease containing a pyrano[2,3-f]chromene derivative compound, a pharmaceutically acceptable salt thereof, or a solvate thereof. The pharmaceutical composition is capable of enhancing or protecting the functions of various enzymes including paraoxonase.


[Figure 1]

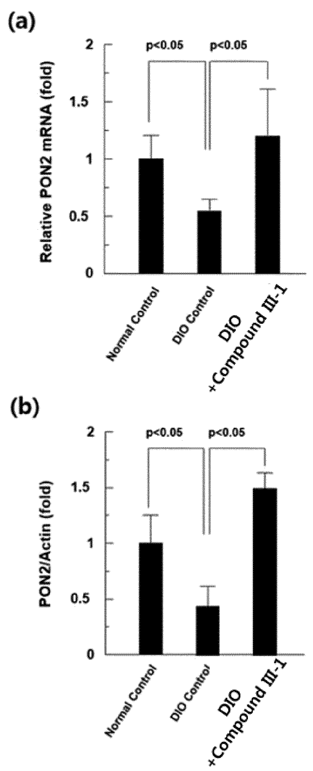


EP 3 936 124 A1

Printed by Jouve, 75001 PARIS (FR)

## Description

### Technical Field

The present disclosure relates to a pharmaceutical composition for preventing or treating neurological disease, and particularly, to a pharmaceutical composition for preventing or treating neurological disease containing a pyrano[2,3-f]chromene derivative compound.

### Background Art

The nervous system is an organ that receives stimuli and signals from the internal and external environments of the body, transmits them to other parts of the body, and produces a response. The nervous system plays a role in regulating and controlling bodily activities according to the situation, and consists of the central nervous system consisting of the brain and spinal cord, and the peripheral nervous system consisting of the peripheral nerves. If a problem arises in the nervous system, it can lead to a fatal disease. Neurological diseases are usually difficult to cure, and hence attention is required. Meanwhile, it is widely known that damage to proteins, lipids and DNA due to increased reactive oxygen species and oxidative stress plays a very important role in the onset of acute and chronic neurological diseases.

Several enzymes are involved in the process of repairing damage caused by reactive oxygen species generated in the human body. One of these enzymes is paraoxonase (PON). Three types of paraoxonase are known, including PON1, PON2, and PON3. Among them, PON1 and PON2 are well known to show antioxidant and anti-inflammatory effects by acting as antioxidant enzymes that protect cells from oxidative stress. It has been reported that PON3 also plays a similar role.

When the functions of PON1 and PON2 were reduced, a phenomenon was observed in which oxidative stress in serum or macrophages increase, and a phenomenon was observed in which oxidative stress-induced toxicity occurs. In addition, it has been reported that paraoxonase plays a neuroprotective role in animals in which PON1 and PON2 are knocked out or deficient. Thus, it is well known that PON1 and PON2 are involved in neuroprotection through antioxidant and anti-inflammatory effects.

Accordingly, there is a need for a pharmaceutical composition capable of enhancing or protecting the functions of various enzymes including paraoxonase in order to prevent and treat neurological diseases.

[Prior Art Documents]

[Patent Documents]

Korean Patent Application Publication No. 10-2018-0002539
Korean Patent Application Publication No. 10-2015-0075030

[Non-Patent Documents]

Modulation of PON2 in mouse brain_Neuroprotection mechanism by quercetin (Neurochem Research 2013 (38) 1809-1818)
PON2 in brain and its potential role in neuroprotection (Neurotoxicology 2014 (43) 3-9)
DJ-1 interacts with and regulates Paraoxonase 2 for neuronal survival in response to ROS (PLOS one 2014 (9) e106601)
PON2 has the neuroprotective role in the mouse central nervous system (Toxicology and Applied Pharmacology 2011 (256) 369-378)
PON2 has the potential neuro-protecting effect against oxidative stress *in vivo* and *in vitro* (Neurochemical Research 2013 (38) 1809-1818; Neurotoxicology 2014 (43) 3-9)

### DISCLOSURE

### Technical Problem

A technical problem to be achieved by the present disclosure is to provide a pharmaceutical composition

containing a pyrano[2,3-f]chromene derivative compound having an excellent effect of preventing or treating neurological disease.

However, the problem to be solved by the present disclosure is not limited to the above-mentioned problem, and other problems not mentioned herein will be clearly understood by those skilled in the art from the following description.

## Technical Solution

One embodiment of the present disclosure provides a pharmaceutical composition for preventing or treating neurological disease containing a pyrano[2,3-f]chromene derivative compound represented by the following Formula I, a pharmaceutically acceptable salt thereof, or a solvate thereof:

[Formula I]

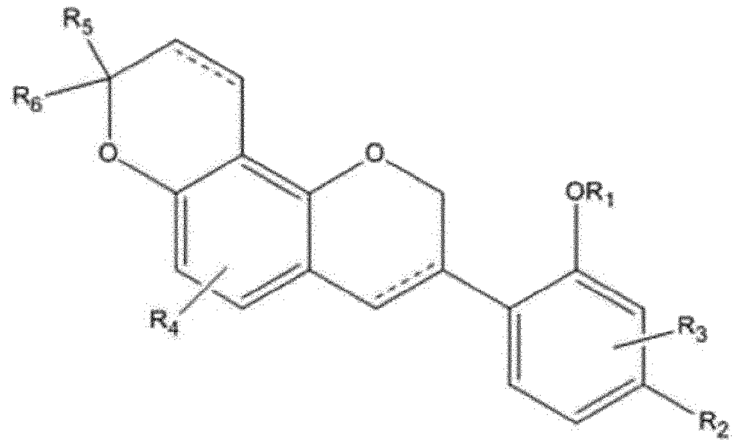

wherein:

- the dotted line is an optional double bond;
- $R_1$ is any one of a hydrogen atom and a substituted or unsubstituted linear or branched $C_1$-$C_6$ alkyl group;
- $R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;
- at least one of $OR_1$ and $R_2$ is a hydroxyl group;
- $R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;
- $R_4$ is a hydrogen atom, methyl, ethyl, methoxy or ethoxy; and
- $R_5$ and $R_6$ are each independently any one of a hydrogen atom and a $C_1$-$C_6$ alkyl group;
- wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

## Advantageous Effects

A pharmaceutical composition containing a pyrano[2,3-f]chromene derivative compound, a pharmaceutically acceptable salt thereof, or a solvate thereof according to one embodiment of the present disclosure has excellent effects of preventing and treating neurological diseases such as Alzheimer's disease, vascular dementia, Parkinson's disease, meningitis, epilepsy, stroke, brain tumor, neuromuscular disease, nervous system infection, hereditary neurological disease, spinal cord disease, and movement disorder.

## Brief Description of Drawings

FIG. 1 depicts graphs showing the relative expression level of PON2 mRNA (FIG. 1a) and the relative expression level of PON2 relative to actin (FIG. 1 b) in each of a normal control group, a DIO control group and a Formula III-1 compound-administered group.
FIG. 2 is a graph showing the cell viability percentage measured according to Experimental Example 3-2.
FIG. 3 is a graph showing the ATP percentage measured according to Experimental Example 3-3.
FIG. 4 is a graph showing the concentration percentage of DCF measured according to Experimental Example 3-4.
FIG. 5 is a graph showing the concentration percentage of Mito-SOX measured according to Experimental Example 3-5.
FIGS. 6a to 6c are graphs showing the expression levels of iNOS and COX-2 in RAW264.7 cells, measured according to Experimental Example 4-2.
FIGS. 7a to 7c are graphs showing the expression levels of iNOS and COX-2 in BV2 cells, measured according to Experimental Example 4-2.
FIGS. 8a to 8c are graphs showing the level of NO production in RAW264.7 cells, measured according to Experimental Example 4-2.
FIGS. 9a to 9c are graphs showing the level of $PGE_2$ production in RAW264.7 cells, measured according to Experimental Example 4-2.
FIGS. 10a to 10c are graphs showing the level of NO production in BV2 cells, measured according to Experimental Example 4-2.
FIGS. 11a to 11c are graphs showing the level of $PGE_2$ production in BV2 cells, measured according to Experimental Example 4-2.
FIGS. 12a to 12c are graphs showing the mRNA expression level of IL-1β in RAW264.7 cells, measured according to Experimental Example 4-3.
FIGS. 13a to 13c are graphs showing the mRNA expression level of IL-6 in RAW264.7 cells, measured according to Experimental Example 4-3.
FIGS. 14a to 14c are graphs showing the mRNA expression level of TNF-α in RAW264.7 cells, measured according to Experimental Example 4-3.
FIGS. 15a to 15c are graphs showing the mRNA expression level of IL-1β in BV2 cells, measured according to Experimental Example 4-3.
FIGS. 16a to 16c are graphs showing the mRNA expression level of IL-6 in BV2 cells, measured according to Experimental Example 4-3.
FIGS. 17a to 17c are graphs showing the mRNA expression level of TNF-α in BV2 cells, measured according to Experimental Example 4-3.
FIGS. 18a to 18c are graphs showing the level of transcription of NF-κB protein into the nucleus of each of p65 and p50 in RAW264.7 cells, measured according to Experimental Example 4-4.
FIGS. 19a to 19c are graphs showing the level of transcription of NF-κB protein into the nucleus of each of p65 and p50 in BV2 cells, measured according to Experimental Example 4-4.

**Best Mode**

All technical terms used herein have the same meaning as commonly understood by those skilled in the art to which the present disclosure pertains, unless otherwise defined. In addition, preferred methods or samples are described herein, but those similar or equivalent thereto are also included within the scope of the present disclosure.

Hereinafter, the present disclosure will be described in more detail.

One embodiment of the present disclosure provides a pharmaceutical composition for preventing or treating neurological disease containing a pyrano[2,3-f]chromene derivative compound represented by the following Formula I, a pharmaceutically acceptable salt thereof, or a solvate thereof:

[Formula I]

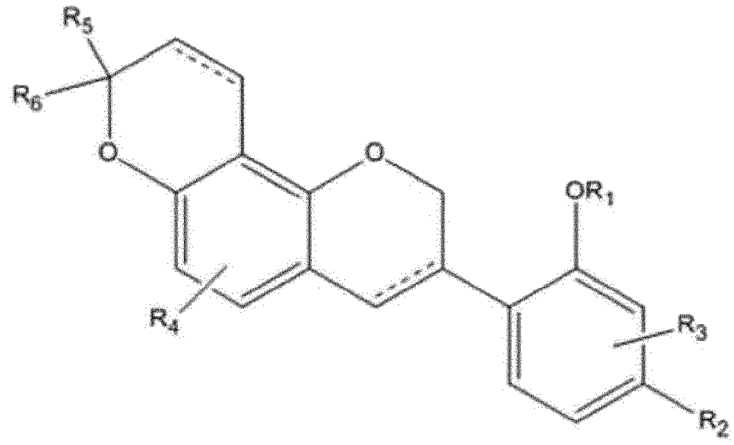

wherein:

the dotted line is an optional double bond;

$R_1$ is any one of a hydrogen atom and a substituted or unsubstituted linear or branched $C_1$-$C_6$ alkyl group;

$R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;

at least one of $OR_1$ and $R_2$ is a hydroxyl group;

$R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;

$R_4$ is a hydrogen atom, methyl, ethyl, methoxy or ethoxy; and

$R_5$ and $R_6$ are each independently any one of a hydrogen atom and a $C_1$-$C_6$ alkyl group;

wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

The term "optional double bond" means that it may be a single bond or a double bond as the case may be.

The pharmaceutical composition containing the pyrano[2,3-f]chromene derivative compound of Formula I, a pharmaceutically acceptable salt thereof, or a solvate thereof according to one embodiment of the present disclosure has excellent effects of preventing and treating neurological diseases, particularly brain disease. Specifically, the pharmaceutical composition has excellent effects of preventing and treating neurodegenerative diseases, neuroinflammation, and mitochondrial dysfunction.

According to one embodiment of the present disclosure, $R_1$ may be a hydrogen atom, a methyl or an ethyl , $R_2$ may be a methyl, an ethyl, an n-propyl, an iso-propyl, an n-butyl, an iso-butyl, a tert-butyl, a methoxy, an ethoxy, an n-propoxy, an iso-propoxy, an n-butoxy, an iso-butoxy, a tert-butoxy, or a hydroxyl group, $R_3$ and $R_4$ may be each a hydrogen atom, and $R_5$ and $R_6$ may be each a methyl. When $R_1$ to $R_6$ are as defined above, the pharmaceutical composition containing the pyrano[2,3-f]chromene derivative compound of Formula I, a pharmaceutically acceptable salt thereof, or a solvate thereof may be chemically stable and may have excellent effects on the prevention and treatment of brain disease.

According to one embodiment of the present disclosure, the pyrano[2,3-f]chromene derivative compound of Formula I contained in the pharmaceutical composition for preventing or treating neurological disease may be a compound of the following Formula II:

[Formula II]

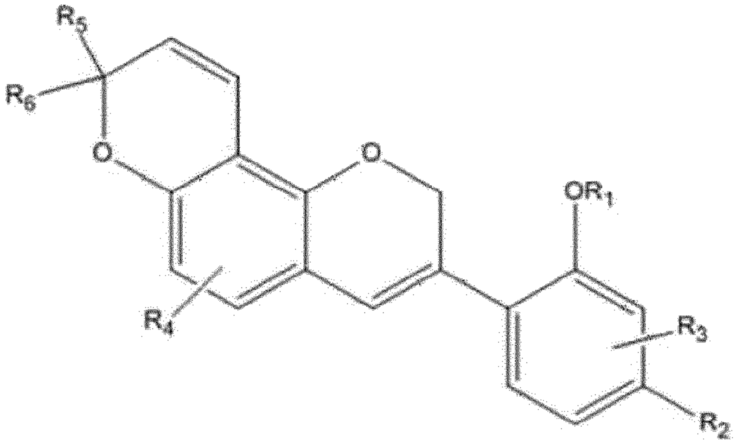

wherein:

$R_1$ is any one of a hydrogen atom and a substituted or unsubstituted linear or branched $C_1$-$C_6$ alkyl group;
$R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;
at least one of $OR_1$ and $R_2$ is a hydroxyl group;
$R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;
$R_4$ is a hydrogen atom, methyl, ethyl, methoxy or ethoxy; and
$R_5$ and $R_6$ are each independently any one of a hydrogen atom and a $C_1$-$C_6$ alkyl group;
wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

According to one embodiment, the compound of Formula II may be any one of the following compounds:

[Formula II-1]

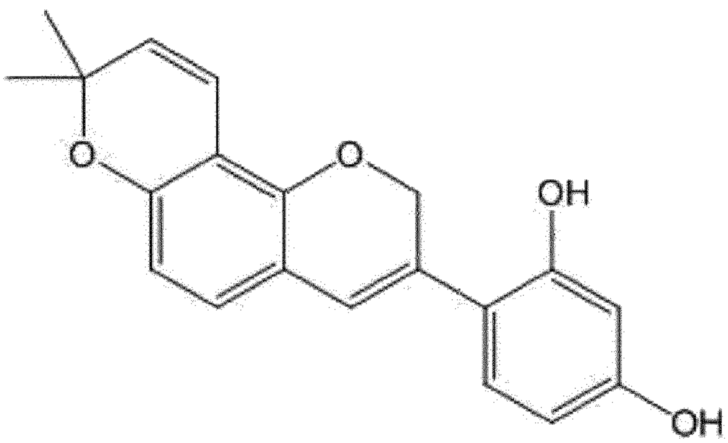

4-(8,8-dimethyl-2,8-dihydropyrano[2,3-f]chromen-3-yl)benzene-1,3-diol;

[Formula II-2]

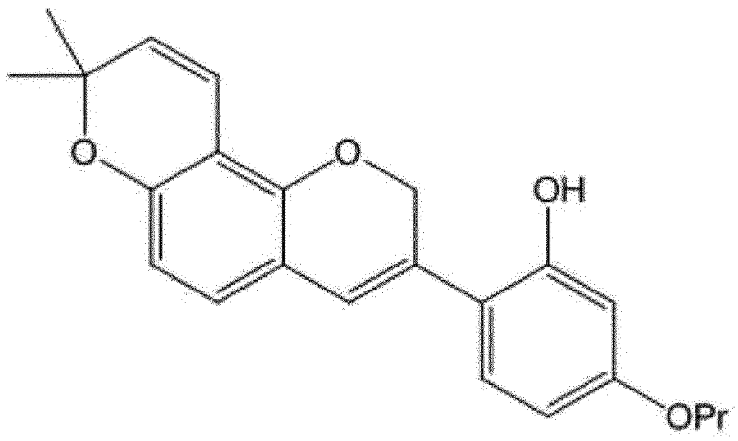

2-(8,8-dimethyl-2,8-dihydropyrano[2,3-f]chromen-3-yl)-5-propoxyphenol.

According to one embodiment of the present disclosure, the pyrano[2,3-f]chromene derivative compound of Formula I may be a compound of the following Formula III:

[Formula III]

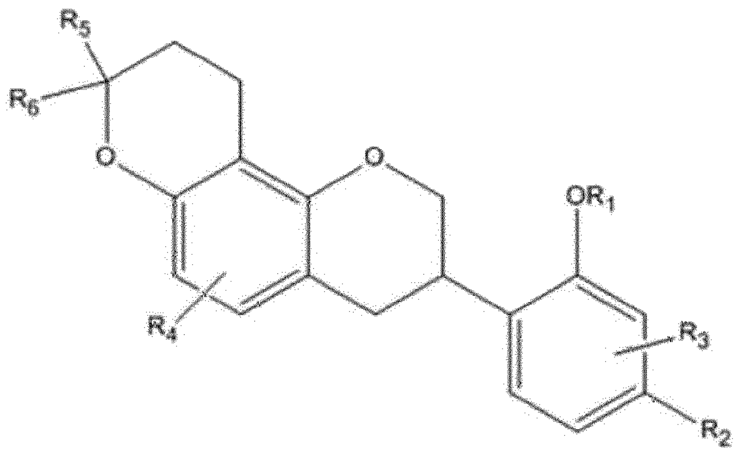

wherein:

$R_1$ is any one of a hydrogen atom and a substituted or unsubstituted linear or branched $C_1$-$C_6$ alkyl group;
$R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;
at least one of $OR_1$ and $R_2$ is a hydroxyl group;
$R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;
$R_4$ is a hydrogen atom, methyl, ethyl, methoxy or ethoxy; and
$R_5$ and $R_6$ are each independently any one of a hydrogen atom and a $C_1$-$C_6$ alkyl group;
wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

According to one embodiment of the present disclosure, the compound of Formula III may be any one of the following compounds:

[Formula III-1]

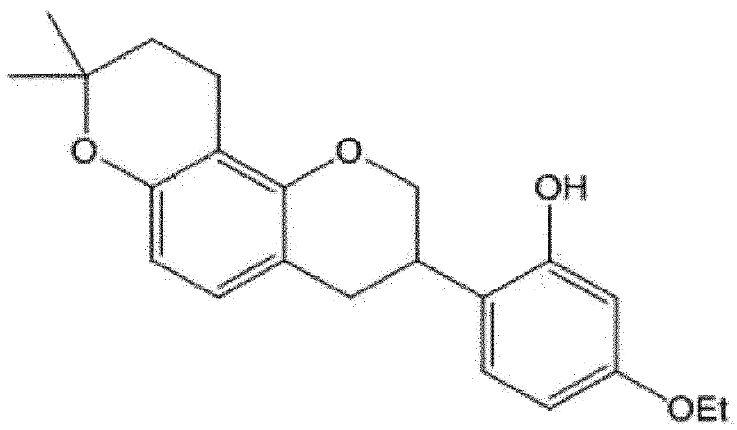

2-(8,8-dimethyl-2,3,4,8,9,0-hexahydropyrano[2,3-f]chromen-3-yl)-5-ethoxyphenol;

[Formula III-2]

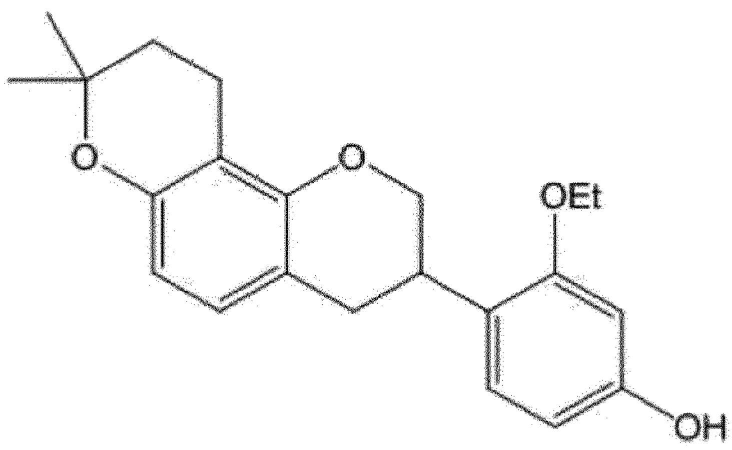

4-(8,8-dimethyl-2,3,4,8,9,10-hexahydropyrano[2,3-f]chromen-3-yl)-3-ethoxyphenol;

[Formula III-3]

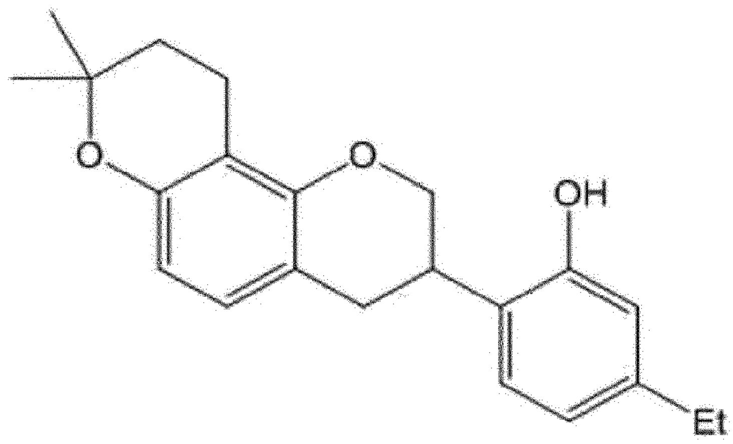

2-(8,8-dimethyl-2,3,4,8,9,10-hexahydropyrano[2,3-f]chromen-3-yl)-5-ethylphenol;

[Formula III-4]

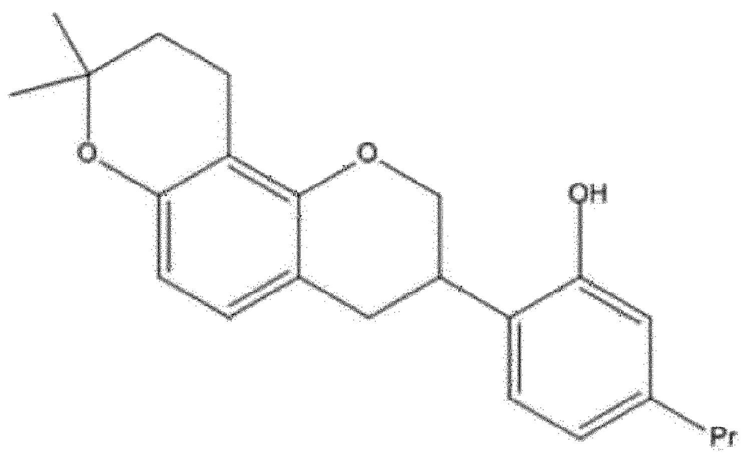

2-(8,8-dimethyl-2,3,4,8,9,10-hexahydropyrano[2,3-f]chromen-3-yl)-5-propylphenol.

According to one embodiment of the present disclosure, the pyrano[2,3-f]chromene derivative compound of Formula I may be a compound of Formula IV:

[Formula IV]

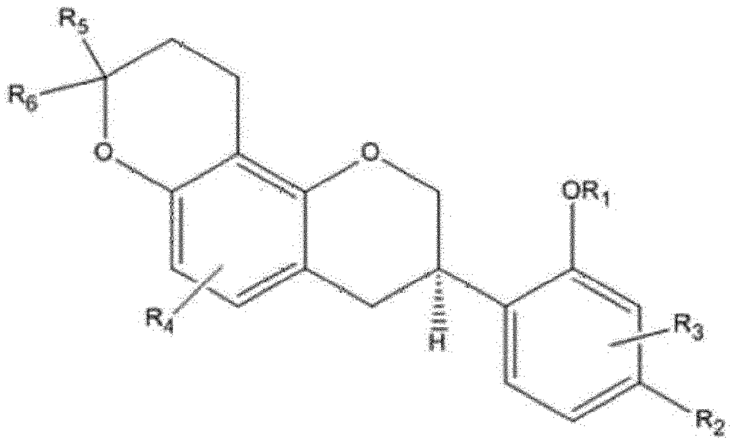

wherein:

$R_1$ is any one of a hydrogen atom and a substituted or unsubstituted linear or branched $C_1$-$C_6$ alkyl group;
$R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;
at least one of $OR_1$ and $R_2$ is a hydroxyl group;
$R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;
$R_4$ is a hydrogen atom, methyl, ethyl, methoxy or ethoxy; and
$R_5$ and $R_6$ are each independently any one of a hydrogen atom and a $C_1$-$C_6$ alkyl group;
wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

According to one embodiment of the present disclosure, the compound of Formula IV may be any one of the following compounds:

[Formula IV-1]

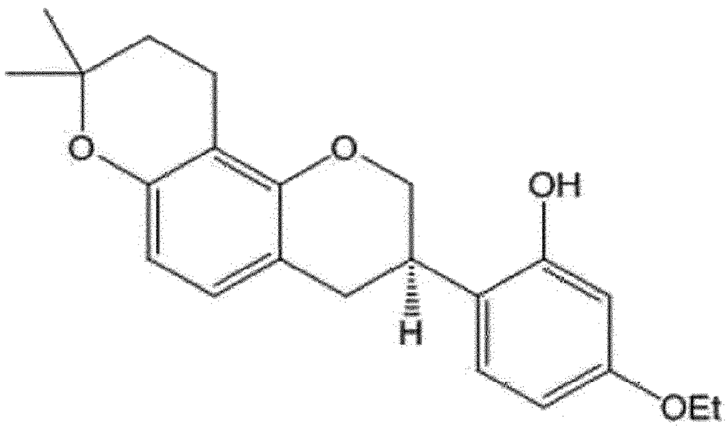

(R)-2-(8,8-dimethyl-2,3,4,8,9,10-hexahydropyrano[2,3-f]chromen-3-yl)-5-ethoxyphenol;

[Formula IV-2]

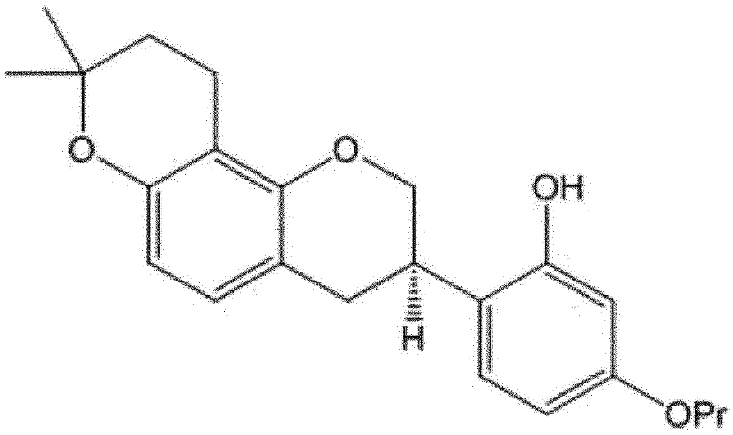

(R)-2-(8,8-dimethyl-2,3,4,8,9,10-hexahydropyrano[2,3-f]chromen-3-yl)-5-propoxyphenol;

[Formula IV-3]

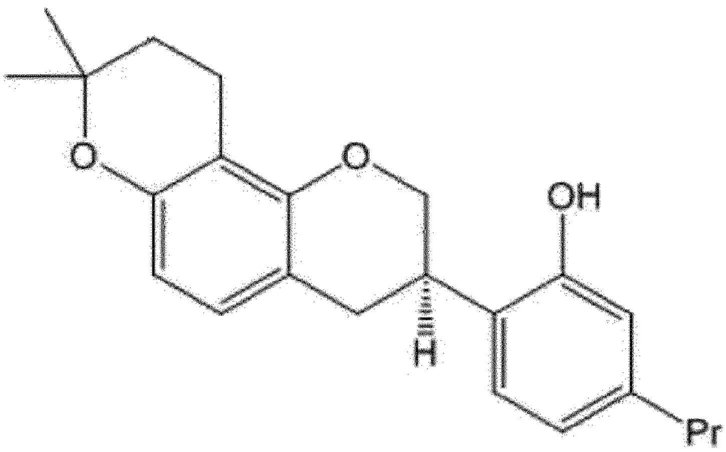

(R)-2-(8,8-dimethyl-2,3,4,8,9,10-hexahydropyrano[2,3-f]chromen-3-yl)-5-propylphenol;

[Formula IV-4]

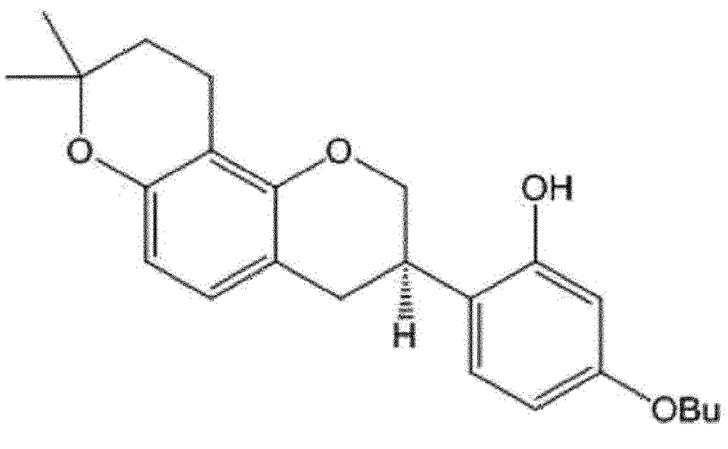

(R)-2-(8,8-dimethyl-2,3,4,8,9,10-hexahydropyrano[2,3-f]chromen-3-yl)-5-butoxyphenol.

According to one embodiment of the present disclosure, the pyrano[2,3-f]chromene derivative compound of Formula I may be a compound of Formula V:

[Formula V]

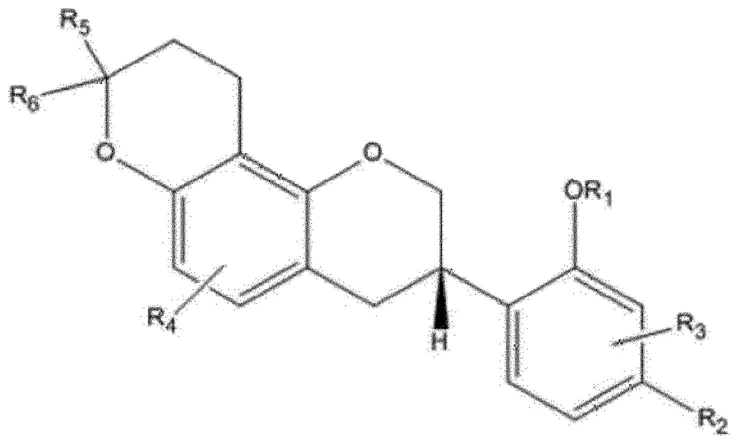

wherein:

$R_1$ is any one of a hydrogen atom and a substituted or unsubstituted linear or branched $C_1$-$C_6$ alkyl group;
$R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;
at least one of $OR_1$ and $R_2$ is a hydroxyl group;
$R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;
$R_4$ is a hydrogen atom, methyl, ethyl, methoxy or ethoxy; and
$R_5$ and $R_6$ are each independently any one of a hydrogen atom and a $C_1$-$C_6$ alkyl group;
wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

According to one of the present disclosure, the compound of Formula V may be any one of the following compounds:

[Formula V-1]

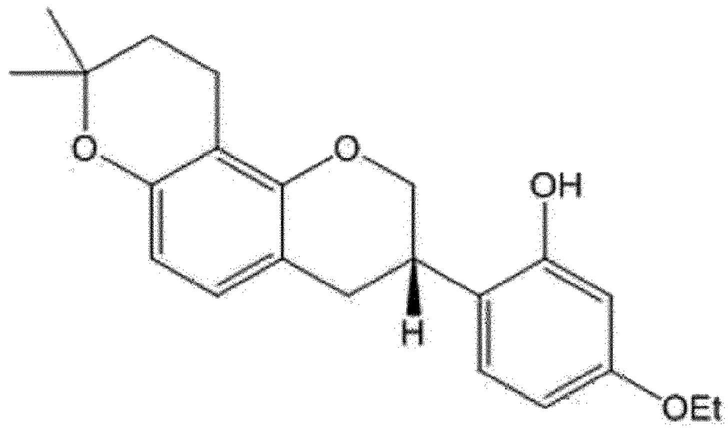

(S)-2-(8,8-dimethyl-2,3,4,8,9,10-hexahydropyrano[2,3-f]chromen-3-yl)-5-ethoxyphenol;

[Formula V-2]

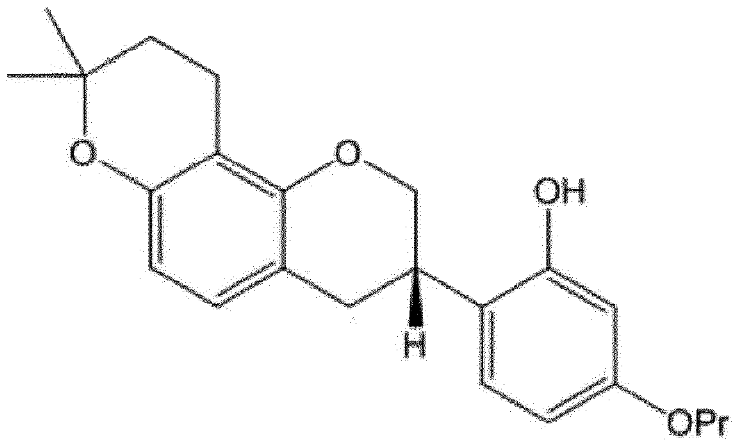

(S)-2-(8,8-dimethyl-2,3,4,8,9,10-hexahydropyrano[2,3-f]chromen-3-yl)-5-propoxyphenol;

[Formula V-3]

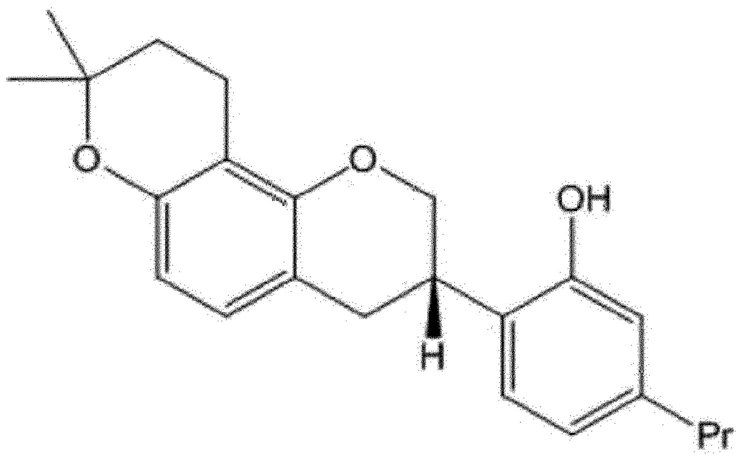

(S)-2-(8,8-dimethyl-2,3,4,8,9,10-hexahydropyrano[2,3-f]chromen-3-yl)-5-propylphenol;

[Formula V-4]

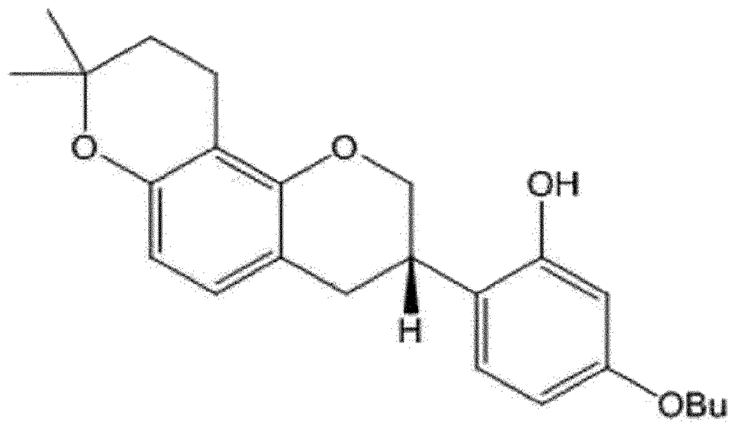

(S) -2- (8, 8-dimethyl-2, 3, 4, 8,9, 10-hexahydropyrano [2, 3-f]chromen-3-yl)-5-butoxyphenol.

According to one embodiment of the present disclosure, the pharmaceutically acceptable salt of the compound of Formula I may mean that the compound of Formula I forms a salt with a free acid and exists as an acid addition salt. The compound of Formula 1 may be formed into a pharmaceutically acceptable acid addition salt according to a conventional method known in the art. As the free acid, an organic acid or an inorganic acid may be used. As the inorganic acid, there may be used hydrochloric acid, bromic acid, sulfuric acid, phosphoric acid, or the like, and as the organic acid, there may be used citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, galacturonic acid, embonic acid, glutamic acid, aspartic acid, or the like.

The pharmaceutical composition according to an embodiment of the present disclosure may contain pharmaceutically acceptable carriers, excipients or diluents, in addition to the pyrano[2,3-f]chromene derivative compound of Formula I, a pharmaceutically acceptable salt thereof, or a solvate thereof. Examples of the pharmaceutically acceptable carriers, excipients or diluents include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil.

In one embodiment of the present disclosure, the pyrano[2,3-f]chromene derivative compound of Formula I may be produced by the same method as described in Korean Patent Application Publication Nos. 10-2015-0075030 and 10-2018-0002539, but is not limited thereto.

In the present disclosure, neurological diseases may be roughly classified into central neurological diseases and peripheral neurological diseases. Examples of central neurological diseases include brain diseases and spinal cord diseases. Neurological disease may be those caused by neurodegeneration, neuroinflammation, mitochondrial dysfunction, or the like.

The pharmaceutical composition according to one embodiment of the present disclosure may be used for the prevention or treatment of neurological diseases, because the compound of Formula I contributes to improving various anti-inflammatory markers and mitochondrial functions by acting as, particularly a PONase activator. The pharmaceutical composition according to one embodiment of the present disclosure may be used for the prevention or treatment of any one neurological disease selected from among, for example, Alzheimer's disease, vascular dementia, Parkinson's disease, meningitis, epilepsy, stroke, brain tumor, neuromuscular disease, nervous system infection, hereditary neurological disease, spinal cord disease, and movement disorder. In addition, it may also be used for the prevention or treatment of headache, dizziness, pain, etc. which are caused by neurological disease.

According to one embodiment of the present disclosure, the pharmaceutical composition may be formulated in conventional pharmaceutical dosage forms known in the art. The pharmaceutical composition may be formulated and administered in any dosage forms including, but not limited to, oral dosage forms, injections, suppositories, transdermal dosage forms and intranasal dosage forms. Preferably, it may be formulated in an oral dosage form or as an injection.

When the pharmaceutical composition is formulated in each of the above-described dosage forms, the formulation may be prepared by adding a pharmaceutically acceptable carrier necessary for the preparation of each dosage form. As used herein, the term "pharmaceutically acceptable carrier" is used to refer to any component other than a

pharmaceutically active ingredient. The term "pharmaceutically acceptable" refers to properties that do not cause any pharmaceutically undesirable change resulting from interaction with other components present in the compositions (for example, interaction between carriers or interaction between a pharmaceutically active ingredient and a carrier). The choice of the pharmaceutically acceptable carrier may depend on factors such as the properties of a particular dosage form, the mode of administration, and the effects of the carrier on solubility and stability.

According to one embodiment of the present disclosure, the pharmaceutically acceptable carrier contained in the pharmaceutical composition for oral administration may be at least one selected from the group consisting of a diluent, a binder, a disintegrant, a glidant (or lubricant), an adsorbent, a stabilizer, a solubilizing agent, a sweetener, a colorant and a flavoring agent, but is not limited thereto.

The term "diluent" refers to any excipient that is added in order to increase the volume of the composition to make a dosage form having a suitable size. The diluent may be at least one selected from among starch (e.g., potato starch, corn starch, wheat starch, pre-gelatinized starch), microcrystalline cellulose (e.g., low-hydration microcrystalline cellulose), lactose (e.g., lactose monohydrate, anhydrous lactose, spray-dried lactose), glucose, sorbitol, mannitol, sucrose, alginate, alkaline earth metal salts, clay, polyethylene glycol, dicalcium phosphate, anhydrous calcium hydrogen phosphate, and silicon dioxide, but is not limited thereto. In the present disclosure, the diluent may be used in an amount of 5 wt% to 50 wt% based on the total weight of the pharmaceutical composition. For example, the diluent may be used in an amount of 10 wt% to 35 wt% based on the total weight of the pharmaceutical composition in order to ensure tabletting and maintain quality.

The term "binder" refers to a material that is used to impart stickiness to a powdery material to make compression easy and improve fluidity. The binder may be at least one selected from among starch, microcrystalline cellulose, highly dispersible silica, mannitol, lactose, polyethylene glycol, polyvinylpyrrolidone, cellulose derivatives (e.g., hydroxypropylmethyl cellulose, hydroxypropyl cellulose, low-substituted hydroxypropyl cellulose), natural gum, synthetic gum, povidone, copovidone, and gelatin, but is not limited thereto. In the present disclosure, the binder may be used in an amount of 2 wt% to 15 wt% based on the total weight of the pharmaceutical composition. For example, the binder may be used in an amount of 1 wt% to 3 wt% based on the total weight of the pharmaceutical composition in order to ensure tabletting and maintain quality.

The term "disintegrant" refers to a material that is added to facilitate the breakdown or disintegration of a solid dosage form after administration *in vivo.* Examples of the disintegrant include, but are not limited to, starch, such as sodium starch glycolate, corn starch, potato starch, or pre-gelatinized starch, or modified starch; clay, such as bentonite, montmorillonite or veegum; cellulose, such as microcrystalline cellulose, hydroxypropyl cellulose or carboxymethyl cellulose; algins, such as sodium alginate or alginic acid; crosslinked cellulose, such as croscarmellose sodium; gum, such as guar gum or xanthan gum; a crosslinked polymer such as crosslinked polyvinylpyrrolidone (crospovidone); or an effervescent agent such as sodium bicarbonate or citric acid, which may be used alone or in combination. In the present disclosure, the disintegrant may be used in an amount of 2 wt% to 15 wt% based on the total weight of the pharmaceutical composition. For example, the disintegrant may be used in an amount of 4 wt% to about 10 wt% based on the total weight of the pharmaceutical composition in order to ensure tabletting and maintain quality.

The term "glidant or lubricant" refers to a material that functions to prevent the adhesion of powder to a compression system and improve the fluidity of granules. Examples of the glidant include, but are not limited to, hard anhydrous silicic acid, talc, stearic acid, a metal salt (magnesium salt or calcium salt) of stearic acid, sodium lauryl sulfate, hydrogenated vegetable oil, sodium benzoate, sodium stearyl fumarate, glyceryl behenate, glyceryl monostearate, and polyethylene glycol, which may be used alone or in combination. In the present disclosure, the glidant may be used in an amount of 0.1 wt% to 5 wt% based on the total weight of the pharmaceutical composition. For example, the glidant may be used in an amount of 1 wt% to 3 wt% in order to ensure tabletting and maintain quality.

Examples of the adsorbent include, but are not limited to, hydrated silicon dioxide, hard anhydrous silicic acid, colloidal silicon dioxide, magnesium aluminometasilicate, microcrystalline cellulose, lactose, and crosslinked polyvinylpyrrolidone, which may be used alone or in combination.

The stabilizer may be at least one selected from among antioxidants such as butylhydroxyanisole, butylhydroxytoluene, carotene, retinol, ascorbic acid, tocopherol, tocopherolpolyethylene glycol succinic acid or propyl gallate; cyclic sugar compounds such as cyclodextrin, carboxyethyl cyclodextrin, hydroxypropyl cyclodextrin, sulfobutyl ether or cyclodextrin; and organic acids such as phosphoric acid, lactic acid, acetic acid, citric acid, tartaric acid, succinic acid, maleic acid, fumaric acid, glycolic acid, propionic acid, gluconic acid or glucuronic acid, but is not limited thereto.

Optionally, the pharmaceutical composition may contain known additives that enhance palatability by stimulating taste. For example, a sweetener such as sucralose, sucrose, fructose, erythritol, potassium acesulfame, sugar alcohol, honey, sorbitol or aspartame may be added to more effectively mask bitterness and maintain the stability and quality of the formulation of the composition. In addition, the composition may contain an acidifier agent such as citric acid or sodium citrate; a natural flavoring agent such as a plum flavor, a lemon flavor, a pineapple flavor or a herb flavor; or a natural pigment such as natural fruit juice, chlorophyllin or a flavonoid.

The pharmaceutical composition for oral administration may be in the form of a solid, semi-liquid or liquid

formulation for oral administration. Examples of the solid formulation for oral administration include, but are not limited to, tablets, pills, hard or soft capsules, powders, fine granules, granules, powders for reconstitution of a solution or suspension, lozenges, wafers, oral strips, dragees, and chewing gum. Examples of the liquid formulation for oral administration include solutions, suspensions, emulsions, syrups, elixirs, alcoholic solutions, aromatic water, lemonade preparations, extracts, infusodecoctions, tinctures, and medicated spirits. Examples of the semi-solid formulation include, but are not limited to, aerosols, creams, gels, and the like.

The pharmaceutical composition according to the present disclosure may be formulated as an injection, and when it is formulated as an injection, it may contain, as a diluent, a non-toxic buffer solution isotonic with blood, for example, a phosphate buffered solution of pH 7.4. The pharmaceutical composition may contain other diluents or additives, in addition to the buffer solution.

The carrier used in the above-mentioned formulation and a method for preparation of the formulation may be selected and prepared according to a method well known in the art, and for example, may be prepared according to the method described in Remington's Pharmaceutical Science (latest edition).

The dosage and timing of administration of the pharmaceutical composition according to the present disclosure may vary depending on the subject's age, sex, condition and body weight, the route of administration, the frequency of administration, and the type of drug. The daily dosage is about 0.1 to 1,000 mg/kg, preferably 1 to 100 mg/kg. The dosage may be appropriately increased or decreased depending on the kind of disease, the degree of progression of the disease, the route of administration, the subject's sex, age and body weight, and the like.

The pharmaceutical composition according to the present disclosure may be arbitrarily administered several times so that the total daily dosage of the compound as an active ingredient is 0.1 to 1,000 mg/kg for an adult, in order to obtain the desired effect.

The pharmaceutical composition according to the present disclosure may contain the compound of formula I according to the present disclosure in an amount of about 0.0001 to 10 wt%, preferably 0.001 to 1 wt%, based on the total weight of the composition.

**Mode for Invention**

Hereinafter, the present disclosure will be described in detail with reference to examples. However, the examples according to the present disclosure may be modified in various different forms, and the scope of the present disclosure is not to be construed as being limited to the examples described below. The examples of the present specification are provided to more completely explain the present disclosure to those of ordinary skill in the art.

**<Experimental Examples>**

In order to examine whether the pyrano[2,3-f]chromene derivative compound of Formula I is effective in preventing or treating neurological disease, the following experiments were conducted.

The following experiments were conducted on compounds of Formulas III-1, IV-1, V-1, IV-2, V-2, II-1, III-4, IV-3, V-3, III-2, IV-4, V-4, II-2 and III-3 produced using the methods described in Korean Patent Application Publication Nos. 10-2015-0075030 and 10-2018-0002539. In addition, glabridin (Daechon Chemical Co., Ltd., Korea) was purchased and used after purification by silica gel column chromatography.

**Experimental Example 1: Experiment for Confirmation of Prevention of Inhibition of PON1 (Paraoxonase 1) Activity under Stress Conditions**

Based on the fact that the activity of PON1 is inhibited when PON1 is treated with oxidized linolenic acid, an experiment was conducted to confirm the prevention of inhibition of PON1 activity. The activity of PON1 was measured using TBBL (5-thiobutyl butyrolactones) as a substrate.

Specifically, after 9.6 μl of 50 mM Tris-HCl containing 1 mM $CaCl_2$ was added to each of 12 Eppendorf tubes, 0.2 μl of each of the compounds of Formulas III-1, IV-1, V-1, IV-2, V-2, II-1, IV-3, V-3, III-2, IV-4, V-4, II-2 and III-3 and glabridine was added to each tube so that the concentration thereof was 30 μM. 0.2 μl of rePON1 (RP-75678, Thermo Corp.) was added to each of the 12 tubes and mixed. Thereafter, each of the mixtures was allowed to react at room temperature for 30 minutes, and 0.5 μl of 1 mM oxidized linolenic acid (OX-LA) dissolved in DMSO was added thereto in order to inhibit rePON1 activity, followed by reaction at room temperature for 2 hours, thus preparing solutions (I).

25 μl of 0.5 mM DTNB dissolved in 50 mM Tris-HCl containing 1 mM $CaCl_2$ was dispensed into each well of a 384-well plate, and then 10 μl of each of the solutions (I) was dispensed into each well, and 20 μl of 2 mM TBBL dissolved in 50 mM Tris-HCl containing 1 mM $CaCl_2$ was added to each well, thus preparing samples. The absorbance of each of the samples at 420 nm was measured using a spectrometer (SpectraMax M2) at 37°C every 2 minutes for 1 hour.

In addition, a negative control was prepared in the same manner as the above-described method, except that,

for the preparation of a solution, 0.2 μl of DMSO was added instead of the compounds of Formulas III-1, IV-1, V-1, IV-2, V-2, II-1, IV-3, V-3, III-2, IV-4, V-4, II-2 and III-3 and glabridin. Furthermore, a normal control was prepared in the same manner as the above-described method, except that, for the preparation of a solution, 0.2 μl of DMSO was added instead of the above-described compounds and glabridin and that oxidized linolenic acid was not added. The absorbance of each of the negative control and the normal control was measured.

Using SoftMax Pro software, the activity of PON1 was calculated from the absorbance using the following Equation 1, and the results are shown in Table 1 below.

[Equation 1]

$$\text{Activity} = (\text{absorbance}_{\text{sample}} - \text{absorbance}_{\text{blank}}) / (\text{absorbance}_{\text{PON1}} - \text{absorbance}_{\text{blank}}) * 100$$

[Table 1]

| | Activity (%) |
|---|---|
| Normal control | 100 |
| Negative control | 73.64 |
| Formula III-1 | 89.03 |
| Formula IV-1 | 95.01 |
| Formula V-1 | 80.71 |
| Formula IV-2 | 93.50 |
| Formula V-2 | 89.40 |
| Formula II-1 | 85.90 |
| Formula IV-3 | 90.60 |
| Formula V-3 | 95.10 |
| Formula III-2 | 83.80 |
| Formula IV-4 | 94.10 |
| Formula V-4 | 94.90 |
| Glabridin | 82.78 |

From Table 1, it can be confirmed that, when oxidized linolenic acid was added to the solutions, each containing the compound of Formula III-1, IV-1, V-1, IV-2, V-2, II-1, IV-3, V-3, III-2, IV-4, V-4, II-2 or III-3 and rePON1, the inhibition of rePON1 activity by the oxidized linolenic acid was prevented. In addition, it can be confirmed that the extent to which the inhibition of the activity of rePON1 was prevented was greater when oxidized linolenic acid was added to the solution containing the compound of Formula III-1, IV-1, V-1, IV-2, V-2, II-1, IV-3, V-3, III-2, IV-4, V-4, II-2 or III-3 and rePON1 than when oxidized linolenic acid was added to the solution containing glabridin and rePON1.

**Experimental Example 2: Experiment for Confirmation of Expression of PON2 (Paraoxonase 2)**

14-Week-old C57BL/6J DIO (Diet Induced Obesity) mice (The Jackson Laboratory, USA) were purchased. The normal control group was fed a 10% fat diet for 3 weeks, and the DIO control group was fed a 60% fat diet for 3 weeks. In addition, the Formula III-1 compound-administered group was fed a 60% fat diet for 3 weeks, and then 100 mg/kg of the compound of Formula III-1 was orally administered thereto once daily for 6 weeks.

The mice of the normal control group, the DIO control group and the Formula III-1 compound-administered group were euthanized, and the liver tissues were extracted therefrom, washed with a PBS solution containing PIC (protease inhibitor cocktail), sectioned to a size of 0.2 to 0.5 g, and stored at -196°C until the experiment.

The following experiment was conducted using the liver tissue sections from the normal control group, the DIO

control group and the Formula III-1 compound-administered group.

**- Analysis of Relative Expression Level of PON2 mRNA**

The relative expression level of PON2 mRNA was analyzed using real-time PCR. Specifically, trizol (Invitrogen, Carlsbad, CA, USA) was added to the liver tissue which was then minced using a homogenizer, and total RNA was extracted therefrom. 100 pmol of oligo Dt17 primer was added to 1 μg of the total RNA and incubated at 74°C for 10 minutes, and 30 U RNasin (RNase inhibitor), 10 U AMV-RT (avian myeloblastosis virus reverse transcriptase) and AMV-RT buffer (Promega, Israel) were added thereto, followed by reaction at 42°C for 2 hours and at 52°C for 1 hour, thereby synthesizing cDNA from the RNA extracted from the liver tissue. The cDNA was amplified using the SYBR Premix Ex Taq kit (Takara Bio Co., Ltd.) and the StepOnePlus Real-time PCR system (Applied Biosystems).

The primer sequences were designed using the Primer Quest software (Integrated DNA Technologies). The primer sequences used are shown in Table 2 below.

[Table 2]

| Primer | Primer sequence | | SEQ ID NO |
|---|---|---|---|
| PON2 | Forward | 5'-GGT AAA CAG GTC TGC GGC CTC G-3' | SEQ ID NO: 1 |
| | Reverse | 5'-CCT AGG AGT CAC TTC CCG CCT CA-3' | SEQ ID NO: 2 |
| GAPDH (glyceraldehyde 3-phosphate dehydrogenase) | Forward | 5'-TGT GTC CGT CGT GGA TCT GA-3' | SEQ ID NO: 3 |
| | Reverse | 5'-CCT GCT TCA CCA CCT TCT TGA T-3' | SEQ ID NO: 4 |

The annealing temperature was set to 60°C. GAPDH is a housekeeping gene that is uniformly expressed in all tissues even under any conditions, and the GAPDH primer was used for the purpose of confirming that the same amount of RNA was used in the experiment.

**- Analysis of Relative Expression Level of PON2 by Western Blot Analysis**

A lysis buffer (CelLytic™ MT Cell Lysis Reagent, Sigma-Aldrich Corp.) containing a protease inhibitor cocktail was added to the liver tissue which was then minced using a homogenizer, incubated at 0°C for 30 minutes, and centrifuged at 14,000 rpm for 15 minutes at 4°C to obtain the supernatant. The protein concentration was quantified using the Bradford method, and then the same amount of protein was separated by electrophoresis on 10% SDS-PAGE and transferred to a polyvinylidene difluoride membrane (PVDF; Pall Corporation, USA) in glycine-methanol buffer. The PVDF membrane was blocked in a blocking solution containing 5% nonfat milk, and then incubated overnight with PON2 antibody (Abcam, UK). Subsequently, the membrane was incubated with a secondary antibody, and then ECL solution (Thermo Fisher Scientific, USA) was applied onto the PVDF membrane to emit light. Next, the PVDF membrane was exposed to X-ray film in the dark and then developed.

Actin, a housekeeping gene, was used for the purpose of confirming that the same amount of RNA was used in the experiment.

FIG. 1 depicts graphs showing the relative expression level of PON2 mRNA (FIG. 1a) and the relative expression level of PON2 relative to actin (FIG. 1b) in each of the normal control group, the DIO control group and the Formula III-1 compound-administered group.

From FIG. 1, it can be confirmed that the expression level of PON2 in the Formula III-1 compound-administered group was higher than that in the DIO control group, and the expression levels of PON2 mRNA and PON2 in the Formula

III-1 compound-administered group were also higher than those in the normal control group.

**Experimental Example 3: Experiment for Confirmation of Restoration of Damaged Mitochondrial Function of Neuroblastoma Cells (SH-SY5Y Cells)**

**3-1. Induction of Mitochondrial Dysfunction and Cell Culture**

Human neuronal SH-SY5Y cells were cultured in a 1:1 DMEM/F12 medium containing 10% FBS (fetal bovine serum) at 37°C under 5% carbon dioxide and 95% air. The cultured cells were transferred to a 96-well cell culture plate at a density of 2 * $10^4$ cells/well and cultured for 24 hours. Thereafter, after replacement with serum-restricted medium (1:1 DMEM/F12 medium containing 0.5% FBS), the cells were cultured for 16 hours. Then, the cultured cells were treated with 1 mM MPP+ (1-methyl-4-phenylpyridinium) and cultured for 24 hours, thus inducing mitochondrial dysfunction in the cells.

Thereafter, the compound of Formula III-1 was added to each well at a concentration of 0.0001, 0.001, 0.01, 0.1, or 1 μM, and the cells were cultured for 24 hours.

**3-2. Confirmation of Inhibitory Effect against Cell Death Resulting from Mitochondrial Damage**

Each of the cultures obtained in Experimental Example 3-1 was treated with 0.2 mg/ml of 3-(4,5-dimethylthiazol-2-yl)-2,5-diphenyl-tetrazolium bromide (MTT; Sigma-Aldrich) and cultured for 4 hours. The MTT formazan precipitate formed by the viable cells was dissolved in 100 μl of 0.04N hydrochloric acid/isopropanol, and the absorbance at 540 nm was measured using an ELISA microplate reader (Molecular Devices).

The absorbances of controls were measured in the same manner as the above-described method, except for using a sample to which DMSO was added (normal control) instead of MMP+ and the compound of Formula III-1 and a sample to which the compound of Formula III-1 was not added (negative control).

The cell viability percentage was calculated from the absorbance.

FIG. 2 is a graph showing the cell viability percentage measured according to Experimental Example 3-2.

As shown in FIG. 2, it can be confirmed that the function of mitochondria damaged by MPP+ was restored when the compound of Formula III-1 was added.

**3-3. Confirmation of Recovery from Decrease in Intracellular ATP Production Caused by Mitochondrial Damage**

100 μl of the cell lysate of the culture obtained in Experimental Example 3-1 was mixed with 100 μl of luciferin-luciferase reaction buffer using an ATP bioluminescent somatic cell assay kit (Sigma-Aldrich), and the mixture was allowed to react at 20°C for 10 minutes. Thereafter, the supernatant was obtained and the fluorescence intensity thereof was measured with an LB 9501 Lumat-Luminometer to confirm recovery from the decrease in intracellular ATP production caused by mitochondrial damage.

The fluorescence intensities of controls were measured in the same manner as the above-described method, except for using a sample to which DMSO was added (normal control) instead of MMP+ and the compound of Formula III-1 and a sample to which the compound of Formula III-1 was not added (negative control).

The intracellular ATP production determined from the fluorescence intensity was expressed as a percentage of the normal control.

FIG. 3 is a graph showing the ATP percentage measured according to Experimental Example 3-3.

As shown in the graph of FIG. 3, it can be confirmed that, when the compound of Formula III-1 was added, recovery from the decrease in ATP production caused by mitochondrial damage due to MPP+ occurred.

**3-4. Confirmation of Effect of Alleviating Reactive Oxygen Species Generation Caused by Mitochondrial Damage**

Based on the principle that 2',7'-dichlorofluorescein diacetate (DCF-DA) is oxidized to fluorescent DCF by reaction with reactive oxygen species, the level of reactive oxygen species generation was measured. 1 μM DCF-DA and 0.05 μM bisbenzimide (Hoechst 33342) were added to the culture obtained in Experimental Example 3-1, and the cells were stained at 37°C for 1 hour. Then, the fluorescence intensity of DCF at an excitation wavelength of 485 nm and an emission wavelength of 535 nm and the fluorescence intensity of bisbenzimide at an excitation wavelength of 335 nm and an emission wavelength of 460 nm were measured using a fluorescence analyzer to determine the level of reactive oxygen species generation.

The fluorescence intensities of controls were measured in the same manner as the above-described method, except for using a sample to which DMSO was added (normal control) instead of MMP+ and the compound of Formula III-1 and a sample to which the compound of Formula III-1 was not added (negative control).

The concentration of DCF determined from the fluorescence intensity was expressed as a percentage of the normal control.

FIG. 4 is a graph showing the concentration percentage of DCF measured according to Experimental Example 3-4.

As shown in the graph of FIG. 4, it can be confirmed that, when the compound of Formula III-1 was added, the level of intracellular reactive oxygen species generation caused by the cytotoxicity induced by the addition of MPP+ decreased.

### 3-5. Confirmation of Effect of Alleviating Reactive Oxygen Species Generated in Mitochondria by Mitochondrial Damage

The level of reactive oxygen species generated in mitochondria was measured using a reduced fluorescent material called MitoSOX™ Red reagent. MitoSOX Red does not fluoresce until it enters cells that breathe, but after it enters the cells and is oxidized, it selectively stains the mitochondria.

Specifically, the culture obtained in Experimental Example 3-1 was treated with 50 μg of MitoSOX Red dissolved in 13 μl DMSO and was incubated at 37°C for 1 hour. After incubation, the cultures were washed twice with PBS, treated with 0.05 μM bisbenzimide (Hoechst 33342) and stained at 37°C for 1 hour. Then, the fluorescence intensity at an excitation wavelength of 510 nm and an emission wavelength of 595 nm was measured with a fluorescence analyzer to determine the level of reactive oxygen species generated in the mitochondria.

The fluorescence intensities of controls were measured in the same manner as the above-described method, except for using a sample to which DMSO was added (normal control) instead of MMP+ and the compound of Formula III-1 and a sample to which the compound of Formula III-1 was not added (negative control).

The concentration of MitoSOX Red determined from the fluorescence intensity was expressed as a percentage of the normal control.

FIG. 5 is a graph showing the concentration percentage of Mito-SOX measured according to Experimental Example 3-5.

As shown in the graph of FIG. 5, it can be confirmed that, when the compound of Formula III-1 was added, the level of reactive oxygen species generated in the mitochondria due to cytotoxicity induced by the addition of MPP+ decreased.

As a result, it can be confirmed from Experimental Example 3 that, when the pyrano[2,3-f]chromene derivative compound of Formula I was included, neuroprotective markers were improved through the enhancement of mitochondrial function.

## Experimental Example 4: Experiment for Confirmation of Anti-inflammatory Effect

### 4-1. Culture and Pretreatment of RAW264.7 Cells and BV2 Cells

RAW264.7 cells or BV2 cells ($5 \times 10^5$ cells/well) were dispensed in DMEM containing 10% heat-inactivated FBS, 10 IU/mL penicillin G, 100 μg/mL streptomycin and 2 mM L-glutamine and cultured in a 5% $CO_2$ incubator at a temperature of 37°C.

The compound of Formula III-3, III-4, V-3, III-1, IV-1, V-1, IV-2, V-2 or II-2 was added to the RAW264.7 cells or BV2 cells, and the toxicities of the compounds were determined by MTT assay. The concentration that did not show cytotoxicity was determined to be the testable concentration, and the following experiment was performed.

In the experiment for conformation of the anti-inflammatory effect, Butein as an anti-inflammatory substance was used for reference.

First, each compound was added to the cultured RAW264.7 cells or BV2 cells as shown in Table 3 below, and the cells were pretreated for 3 hours.

[Table 3]

| | Amount (μM) added to RAW264.7 cells | | | Amount (μM) added to BV2 cells | | |
|---|---|---|---|---|---|---|
| Formula III-3 | 5 | 10 | 20 | 2.5 | 5 | 10 |
| Formula III-4 | 2.5 | 5 | 10 | 2.5 | 5 | 10 |
| Formula V-3 | 1.25 | 2.5 | 5 | 0.625 | 1.25 | 2.5 |
| Formula III-1 | 5 | 10 | 20 | 2.5 | 5 | 10 |

(continued)

| | Amount ($\mu$M) added to RAW264.7 cells | | | Amount ($\mu$M) added to BV2 cells | | |
|---|---|---|---|---|---|---|
| Formula IV-1 | 5 | 10 | 20 | 5 | 10 | 20 |
| Formula V-1 | 5 | 10 | 20 | 1.25 | 2.5 | 5 |
| Formula IV-2 | 5 | 10 | 20 | 5 | 10 | 20 |
| Formula V-2 | 2.5 | 5 | 10 | 1.25 | 2.5 | 5 |
| Formula II-2 | 2.5 | 5 | 10 | 5 | 10 | 20 |
| Glabridin | 10 | 20 | 40 | 5 | 10 | 20 |

### 4-2. Analysis of Expression Levels of iNOS and COX-2 Proteins and Production of NO and $PGE_2$

The pretreated cells of Experimental Example 4-1 were treated with 1 $\mu$g/ml of LPS for 24 hours, and then the expression levels of iNOS and COX-2 were observed through Western blot, and the production of NO and $PGE_2$ was measured.

FIGS. 6a to 6c are graphs showing the expression levels of iNOS and COX-2 in RAW264.7 cells, measured according to Experimental Example 4-2, and FIGS. 7a to 7c are graphs showing the expression levels of iNOS and COX-2 in BV2 cells, measured according to Experimental Example 4-2.

FIGS. 8a to 8c are graphs showing the level of NO production in RAW264.7 cells, measured according to Experimental Example 4-2, and FIGS. 9a to 9c are graphs showing the level of $PGE_2$ production in RAW264.7 cells, measured according to Experimental Example 4-2.

FIGS. 10a to 10c are graphs showing the level of NO production in BV2 cells, measured according to Experimental Example 4-2, and FIGS. 11a to 11c are graphs showing the level of $PGE_2$ production in BV2 cells, measured according to Experimental Example 4-2.

### 4-3. Analysis of Expression Levels of IL-1$\beta$, IL-6 and TNF-$\alpha$

The pretreated cells of Experimental Example 4-1 were treated with 1 $\mu$g/ml of LPS for 6 hours, and then the mRNA expression level of the cytokine IL-1$\beta$, IL-6 or TNF-$\alpha$ produced in the inflammatory response was analyzed using RT-PCR.

FIGS. 12a to 12c are graphs showing the mRNA expression level of IL-1$\beta$ in RAW264.7 cells, measured according to Experimental Example 4-3; FIGS. 13a to 13c are graphs showing the mRNA expression level of IL-6 in RAW264.7 cells, measured according to Experimental Example 4-3; and FIGS. 14a to 14c are graphs showing the mRNA expression level of TNF-$\alpha$ in RAW264.7 cells, measured according to Experimental Example 4-3.

FIGS. 15a to 15c are graphs showing the mRNA expression level of IL-1$\beta$ in BV2 cells, measured according to Experimental Example 4-3; FIGS. 16a to 16c are graphs showing the mRNA expression level of IL-6 in BV2 cells, measured according to Experimental Example 4-3; and FIGS. 17a to 17c are graphs showing the mRNA expression level of TNF-$\alpha$ in BV2 cells, measured according to Experimental Example 4-3.

### 4-4. Confirmation of Inhibition of Transcription of NF-$\kappa$B Protein into Nucleus

The pretreated cells of Experimental Example 4-1 were treated with 1 $\mu$g/ml of LPS for 1 hour, and then the level of transcription of NF-$\kappa$B protein into the nucleus of each of p65 and p50 was analyzed through Western blot.

FIGS. 18a to 18c are graphs showing the level of transcription of NF-$\kappa$B protein into the nucleus of each of p65 and p50 in RAW264.7 cells, measured according to Experimental Example 4-4, and FIGS. 19a to 19c are graphs showing the level of transcription of NF-KB protein into the nucleus of each of p65 and p50 in BV2 cells, measured according to Experimental Example 4-4.

From FIG. 6a through FIG. 19c, it can be confirmed that the compound of Formula III-3, III-4, V-3, III-1, IV-1, V-1, IV-2, V-2 or II-2 concentration-dependently decreased the expression levels of iNOS and COX-2, the production of NO and $PGE_2$, the mRNA expression levels of IL-6, IL-1$\beta$ and TNF-$\alpha$, and the levels of transcription of NF-$\kappa$B protein into the nuclei of p65 and p50, in RAW264.7 cells or BV2 cells. In contrast, it can be confirmed that, when glabridin was added, the expression levels of iNOS and COX-2, the production of NO and $PGE_2$, the mRNA expression levels of IL-6, IL-1$\beta$ and TNF-$\alpha$, and the levels of transcription of NF-$\kappa$B protein into the nuclei of p65 and p50, in RAW264.7 cells or BV2 cells, did not decrease compared to when the pyrano[2,3-f]chromene derivative compound of Formula I was

added. In addition, it can be confirmed that, when the pyrano[2,3-f]chromene derivative compound of Formula I was added, it exhibited an anti-inflammatory effect equal to or higher than glabridine at a lower concentration than that of glabridine added.

As a result, it can be confirmed that the pyrano[2,3-f]chromene derivative compound of Formula I acts as a PONase activator and contributes to improving various anti-inflammatory markers and mitochondrial function.

## Claims

1. A pharmaceutical composition for preventing or treating neurological disease containing a pyrano[2,3-f]chromene derivative compound of the following Formula I, a pharmaceutically acceptable salt thereof, or a solvate thereof:

[Formula I]

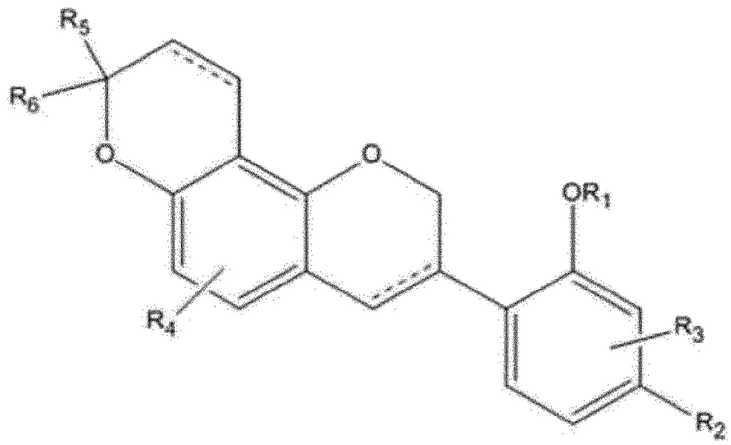

wherein:

the dotted line is an optional double bond;
$R_1$ is any one of a hydrogen atom and a substituted or unsubstituted linear or branched $C_1$-$C_6$ alkyl group;
$R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;
at least one of $OR_1$ and $R_2$ is a hydroxyl group;
$R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;
$R_4$ is a hydrogen atom, methyl, ethyl, methoxy or ethoxy; and
$R_5$ and $R_6$ are each independently any one of a hydrogen atom and a $C_1$-$C_6$ alkyl group;
wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

2. The pharmaceutical composition of claim 1, wherein:

$R_1$ is a hydrogen atom, a methyl group or an ethyl group;
$R_2$ is a methyl, an ethyl, an n-propyl, an iso-propyl, an n-butyl, an iso-butyl, a tert-butyl, a methoxy, an ethoxy, an n-propoxy, an iso-propoxy, an n-butoxy, an iso-butoxy, a tert-butoxy, or a hydroxyl;
$R_3$ and $R_4$ are each a hydrogen atom; and
$R_5$ and $R_6$ are each a methyl.

3. The pharmaceutical composition of claim 1, wherein the pyrano[2,3-f]chromene derivative compound of Formula I is a compound of the following Formula II:

[Formula II]

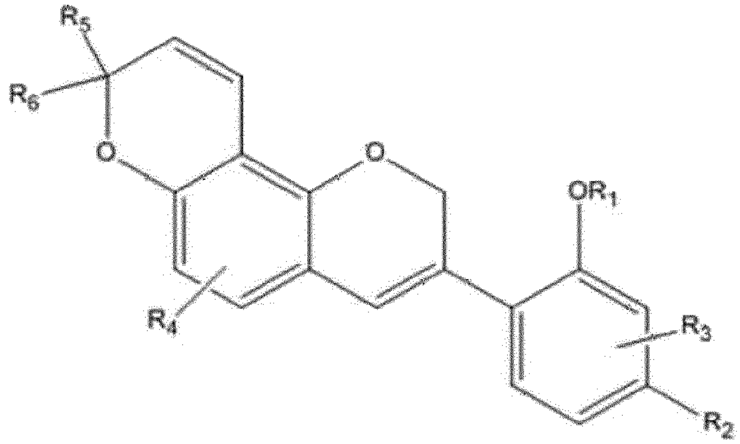

wherein:

$R_1$ is any one of a hydrogen atom and a substituted or unsubstituted linear or branched $C_1$-$C_6$ alkyl group;
$R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;
at least one of $OR_1$ and $R_2$ is a hydroxyl group;
$R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;
$R_4$ is a hydrogen atom, methyl, ethyl, methoxy or ethoxy; and
$R_5$ and $R_6$ are each independently any one of a hydrogen atom and a $C_1$-$C_6$ alkyl group;
wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

**4.** The pharmaceutical composition of claim 3, wherein the compound of Formula II is any one of the following compounds:

[Formula II-1]

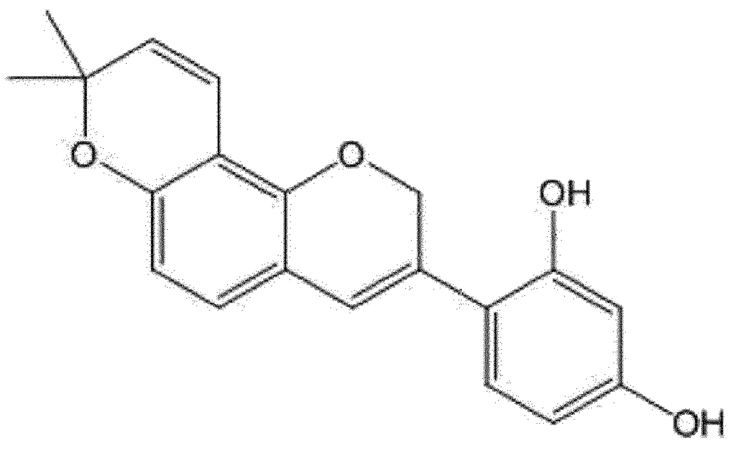

[Formula II-2]

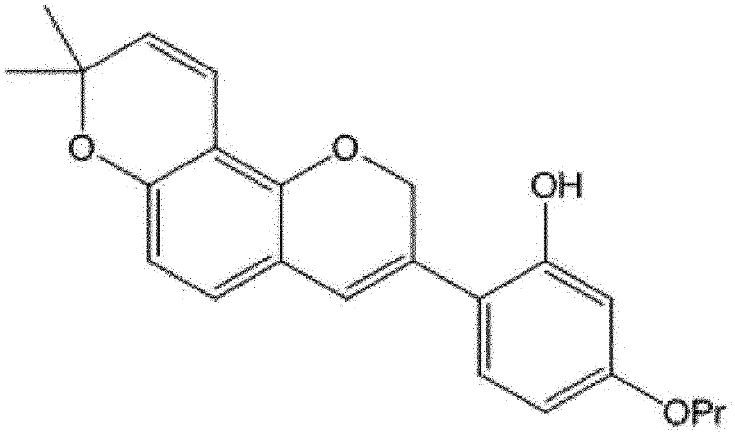

5. The pharmaceutical composition of claim 1, wherein the pyrano[2,3-f]chromene derivative compound of Formula I is a compound of the following Formula III:

[Formula III]

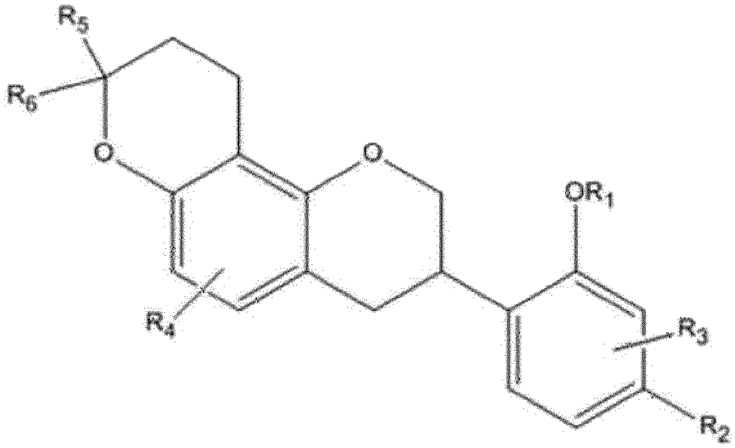

wherein:

$R_1$ is any one of a hydrogen atom and a substituted or unsubstituted linear or branched $C_1$-$C_6$ alkyl group;
$R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;
at least one of $OR_1$ and $R_2$ is a hydroxyl group;
$R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;
$R_4$ is a hydrogen atom, methyl, ethyl, methoxy or ethoxy; and
$R_5$ and $R_6$ are each independently any one of a hydrogen atom and a $C_1$-$C_6$ alkyl group;
wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

6. The pharmaceutical composition of claim 5, wherein the compound of Formula III is any one of the following compounds:

[Formula III-1]

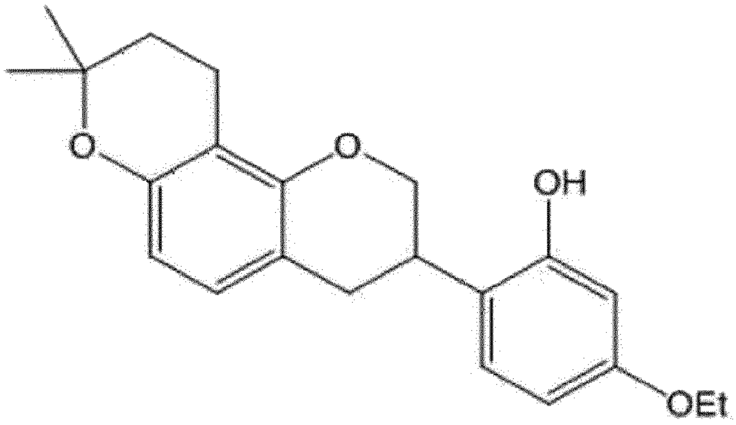

[Formula III-2]

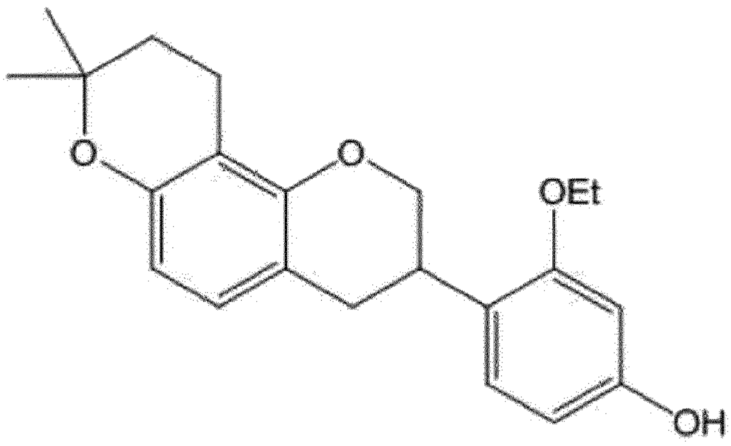

[Formula III-3]

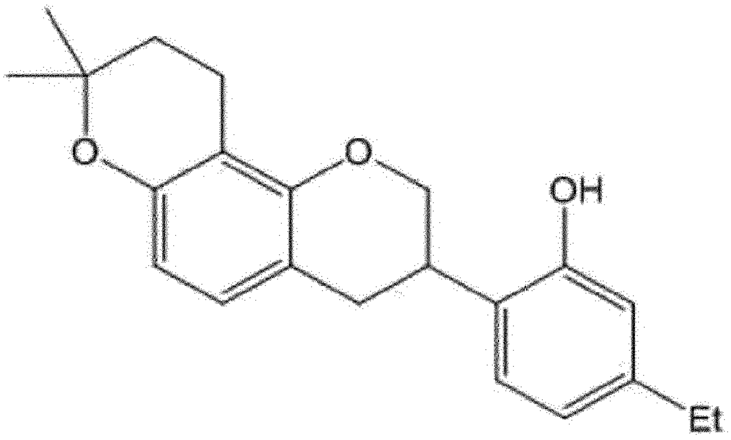

[Formula III-4]

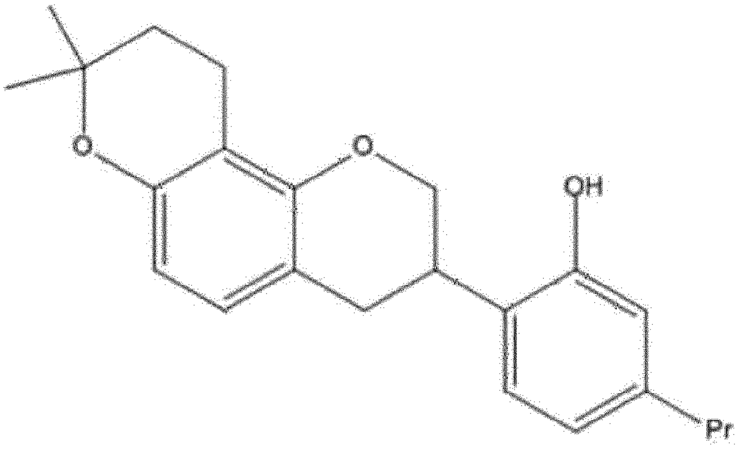

7. The pharmaceutical composition of claim 1, wherein the pyrano[2,3-f]chromene derivative compound of Formula I is a compound of Formula IV:

[Formula IV]

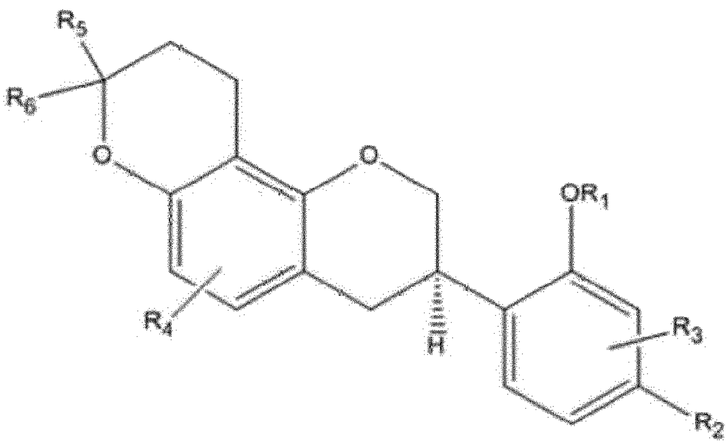

wherein:

$R_1$ is any one of a hydrogen atom and a substituted or unsubstituted linear or branched $C_1$-$C_6$ alkyl group;
$R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;
at least one of $OR_1$ and $R_2$ is a hydroxyl group;
$R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;
$R_4$ is a hydrogen atom, methyl, ethyl, methoxy or ethoxy; and
$R_5$ and $R_6$ are each independently any one of a hydrogen atom and a $C_1$-$C_6$ alkyl group;
wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

8. The pharmaceutical composition of claim 7, wherein the compound of Formula IV is any one of the following com-

pounds:

[Formula IV-1]

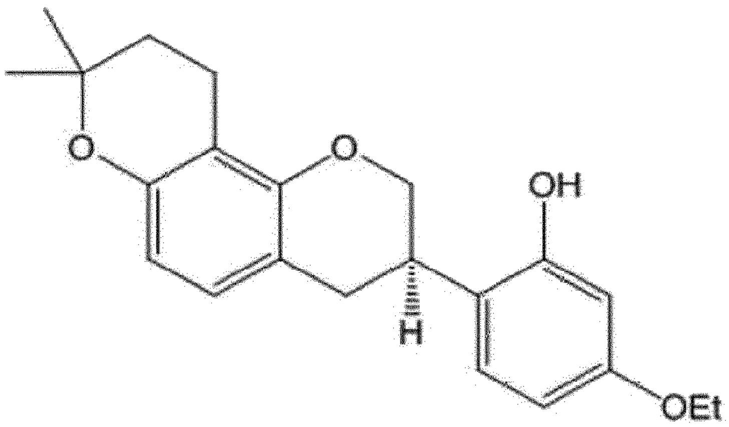

[Formula IV-2]

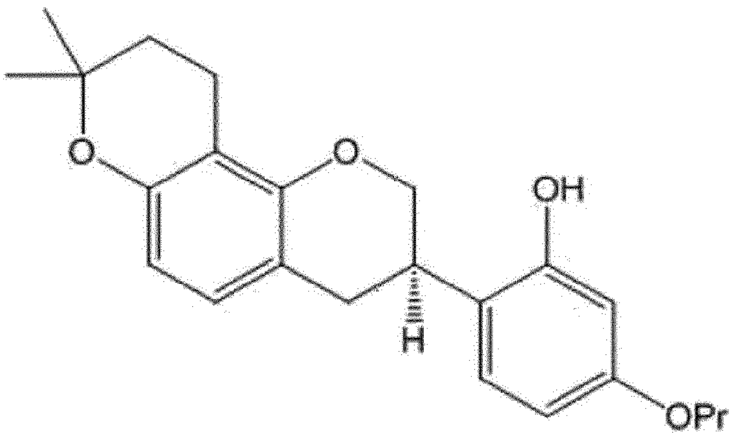

[Formula IV-3]

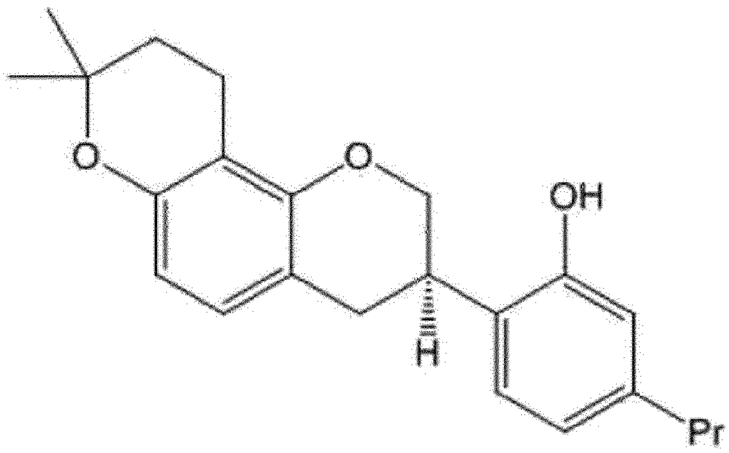

[Formula IV-4]

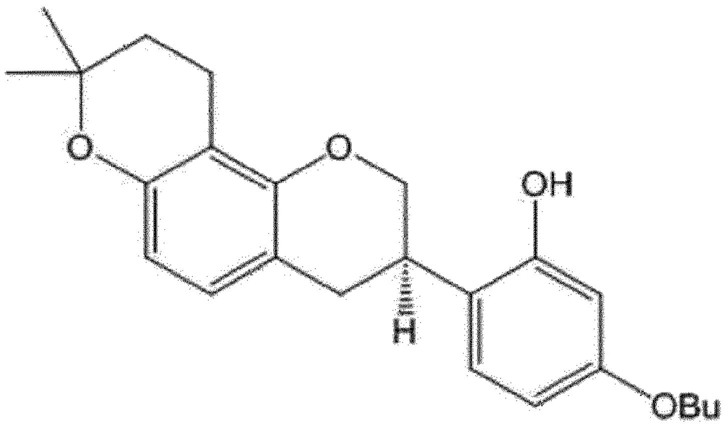

9. The pharmaceutical composition of claim 1, wherein the pyrano[2,3-f]chromene derivative compound of Formula I is a compound of Formula V:

[Formula V]

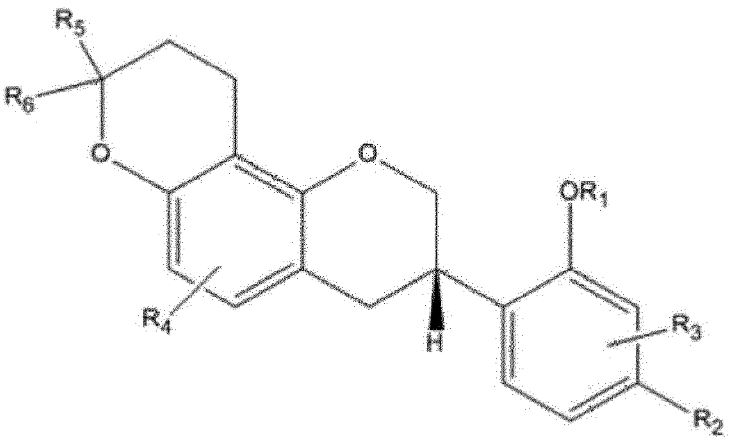

wherein:

$R_1$ is any one of a hydrogen atom and a substituted or unsubstituted linear or branched $C_1$-$C_6$ alkyl group;
$R_2$ is any one of a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, and a hydroxyl group;
at least one of $OR_1$ and $R_2$ is a hydroxyl group;
$R_3$ is any one of a hydrogen atom, a substituted or unsubstituted linear or branched $C_1$-$C_6$ alkyl group, a substituted or unsubstituted linear or branched $C_1$-$C_6$ alkoxy group, a substituted or unsubstituted linear or branched $C_3$-$C_6$ allyloxy group, a substituted or unsubstituted $C_6$-$C_{12}$ aryloxy group, a substituted or unsubstituted linear or branched $C_1$-$C_4$ thioalkyl group, and a halogen atom;
$R_4$ is a hydrogen atom, methyl, ethyl, methoxy or ethoxy; and
$R_5$ and $R_6$ are each independently any one of a hydrogen atom and a $C_1$-$C_6$ alkyl group;
wherein the substituent of each of the substituted alkyl group, the substituted alkoxy group, the substituted allyloxy group, the substituted aryloxy group and the substituted thioalkyl group is any one of a halogen atom, a linear or branched $C_1$-$C_5$ alkyl group, a linear or branched $C_1$-$C_5$ alkoxy group, and a linear or branched $C_1$-$C_3$ thioalkyl group.

10. The pharmaceutical composition of claim 9, wherein the compound of Formula V is any one of the following compounds:

[Formula V-1]

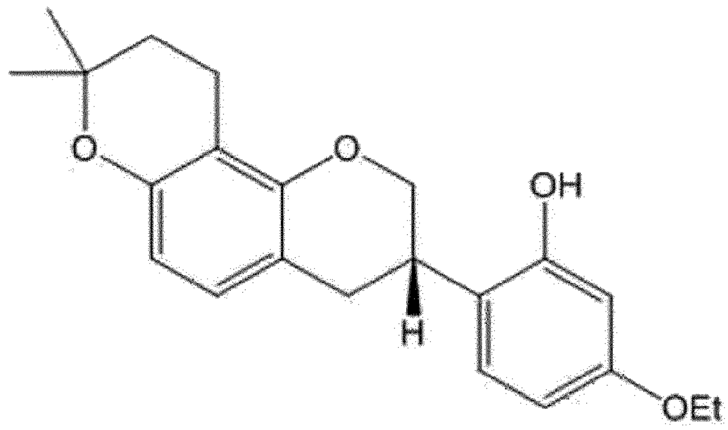

[Formula V-2]

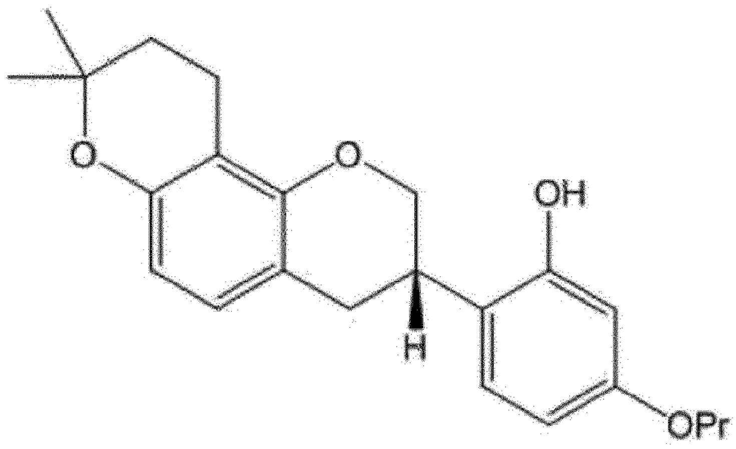

[Formula V-3]

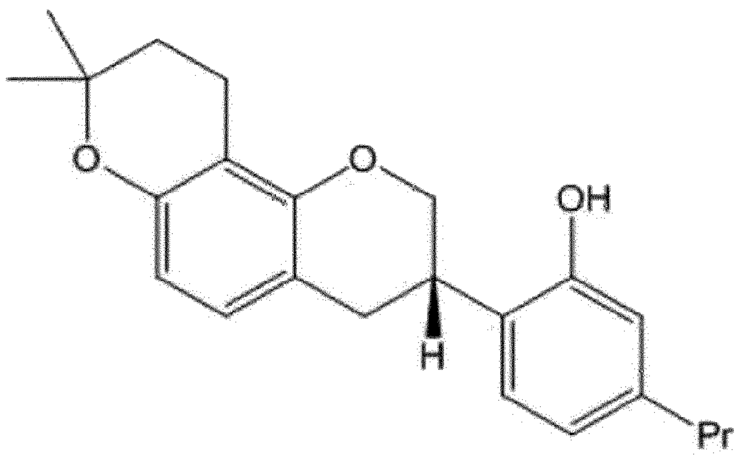

[Formula V-4]

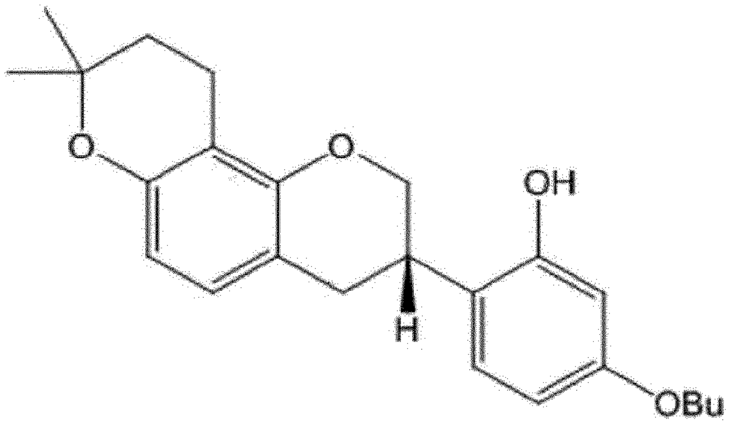

**11.** The pharmaceutical composition of claim 1, wherein the neurological disease is any one of Alzheimer's disease, vascular dementia, Parkinson's disease, meningitis, epilepsy, stroke, brain tumor, neuromuscular disease, nervous system infection, hereditary neurological disease, spinal cord disease, and movement disorder.

[Figure 1]

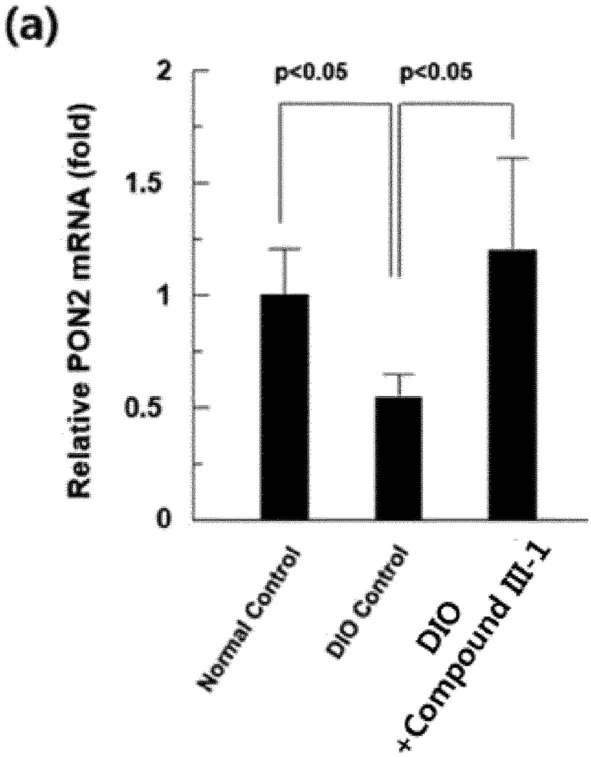
(a)
2
1.5
1
0.5
0
Relative PON2 mRNA (fold)
p<0.05
p<0.05
Normal Control
DIO Control
DIO
+Compound III-1

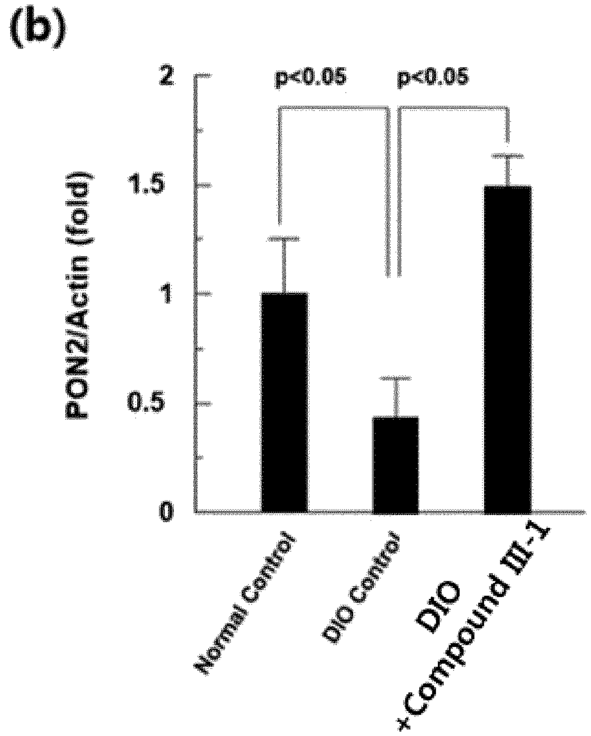
(b)
2
1.5
1
0.5
0
PON2/Actin (fold)
p<0.05
p<0.05
Normal Control
DIO Control
DIO
+Compound III-1

[Figure 2]

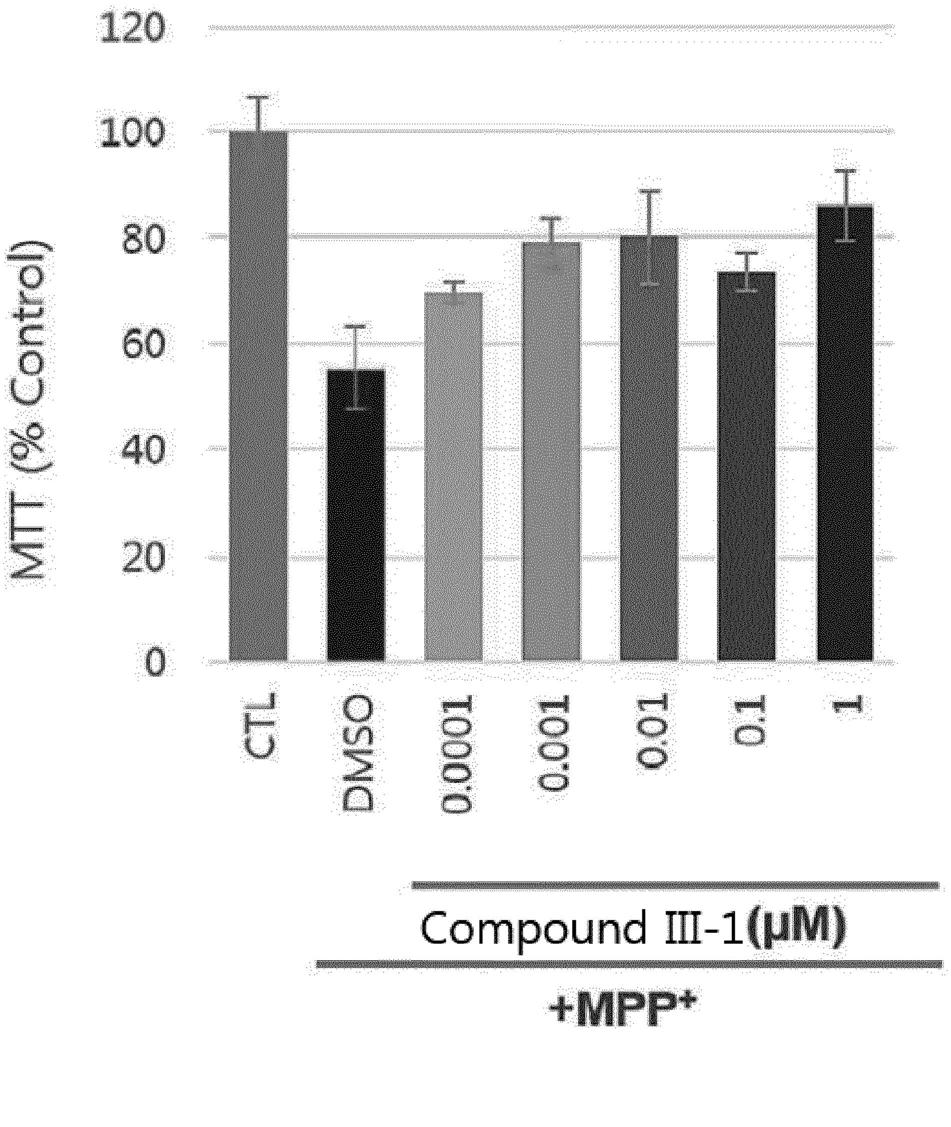
120
100
80
60
40
20
0
MTT (% Control)
CTL
DMSO
0.0001
0.001
0.01
0.1
1
Compound III-1(μM)
+MPP+

[Figure 3]

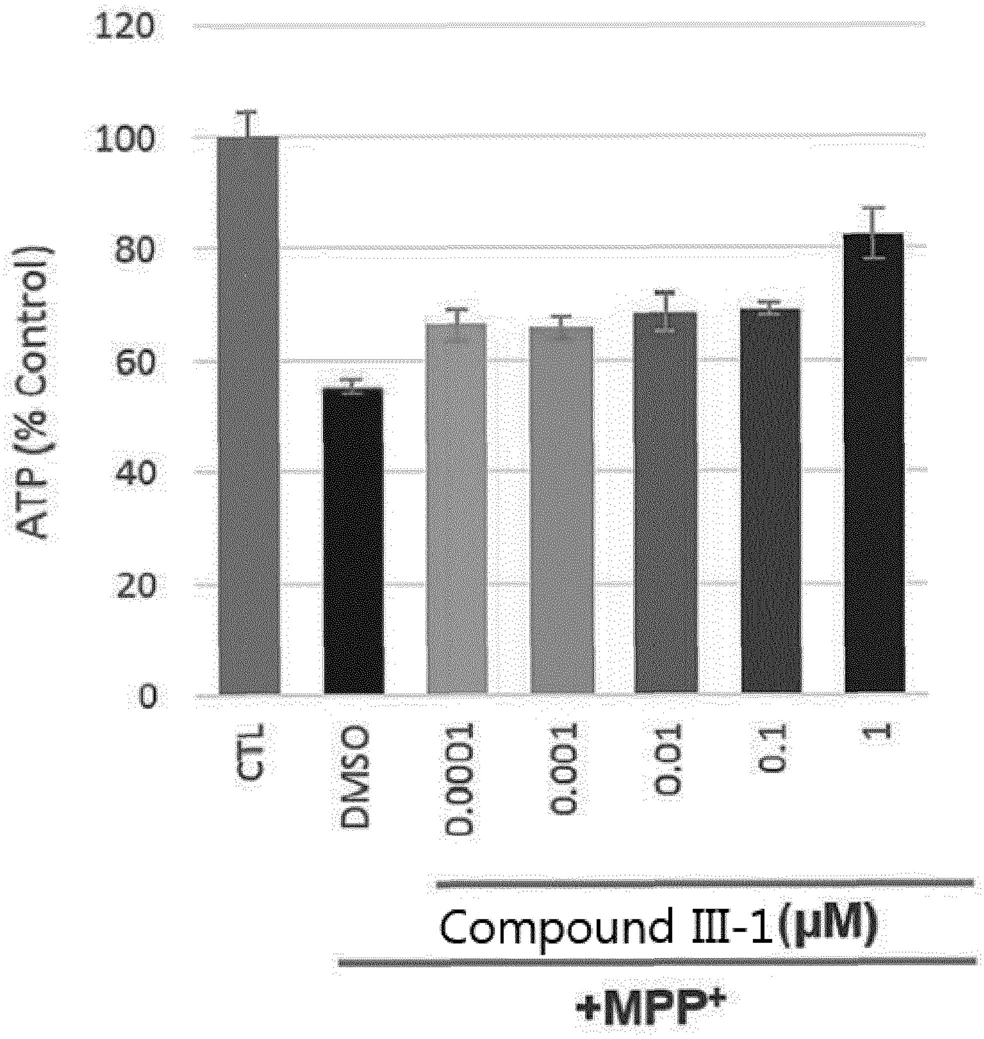
120
100
80
60
40
20
0
ATP (% Control)
CTL
DMSO
0.0001
0.001
0.01
0.1
1
Compound III-1(μM)
+MPP+

[Figure 4]

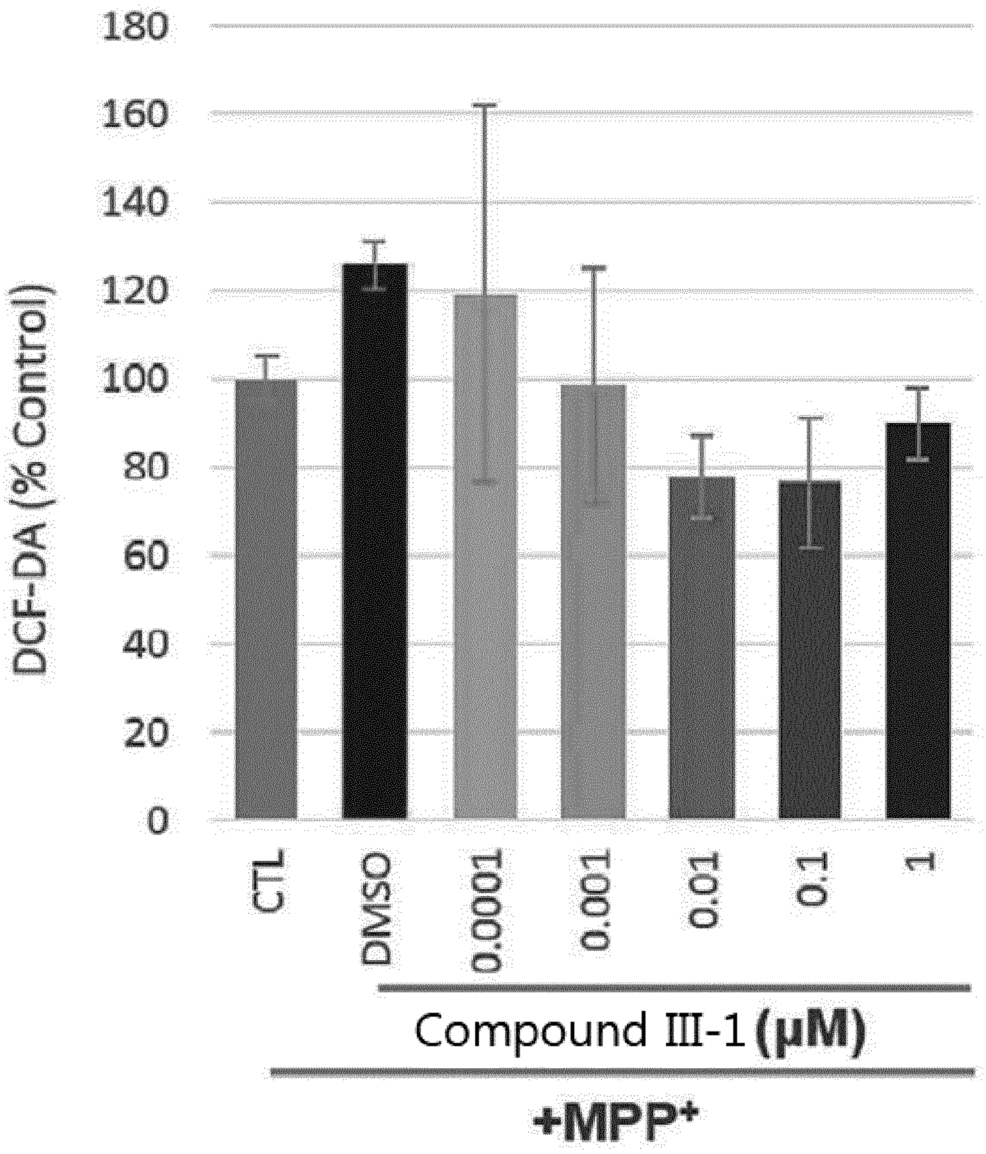

180
160
140
120
100
80
60
40
20
0
DCF-DA (% Control)
CTL
DMSO
0.0001
0.001
0.01
0.1
1
Compound Ⅲ-1 (μM)
+MPP+

[Figure 5]

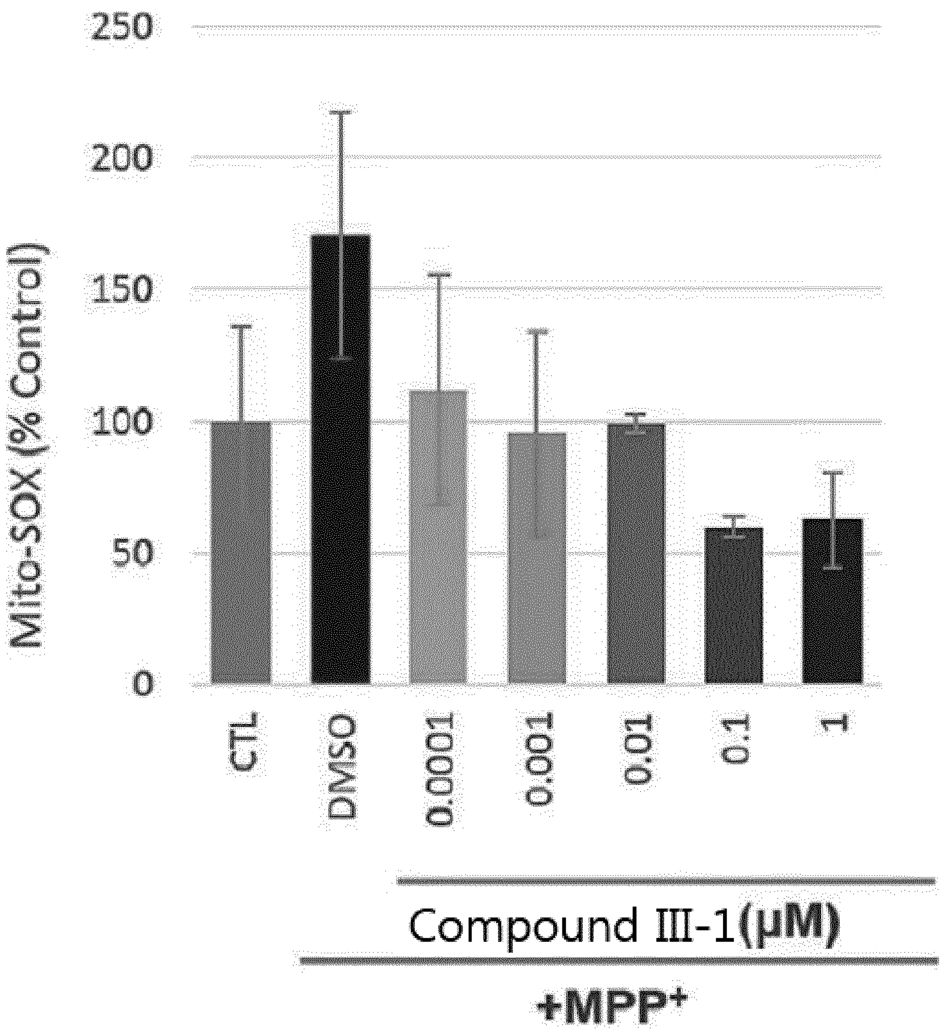
250
200
150
100
50
0
Mito-SOX (% Control)
CTL
DMSO
0.0001
0.001
0.01
0.1
1
Compound Ⅲ-1(μM)
+MPP+

[Figure 6a]

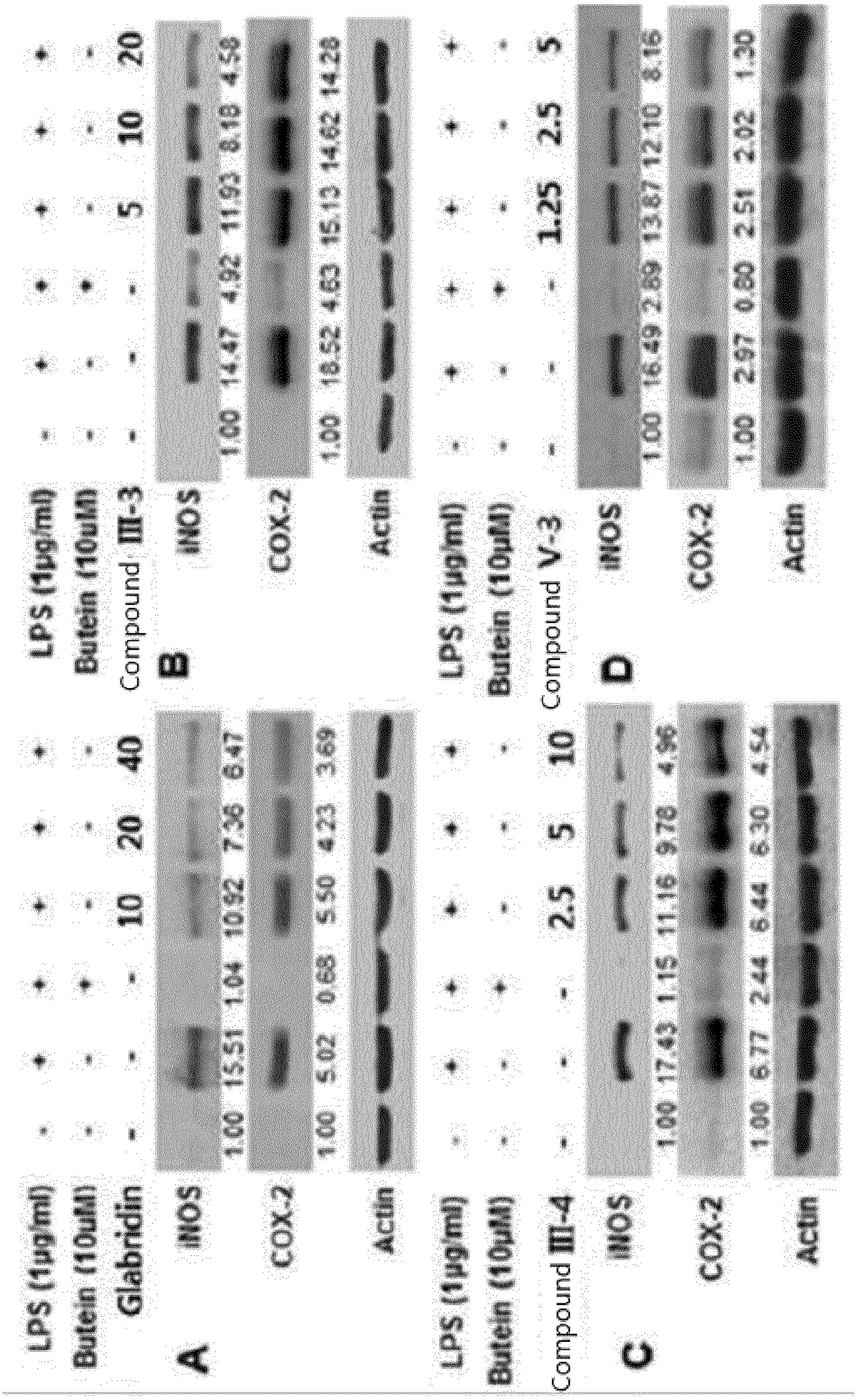
A
LPS (1μg/ml) - + + + + +
Butein (10uM) - - + - - -
Glabridin - - - 10 20 40
iNOS
1.00 15.51 1.04 10.92 7.36 6.47
COX-2
1.00 5.02 0.68 5.50 4.23 3.69
Actin
B
LPS (1μg/ml) - + + + + +
Butein (10uM) - - + - - -
Compound III-3 - - - 5 10 20
iNOS
1.00 14.47 4.92 11.93 8.18 4.58
COX-2
1.00 18.52 4.63 15.13 14.62 14.28
Actin
C
LPS (1μg/ml) - + + + + +
Butein (10uM) - - + - - -
Compound III-4 - - - 2.5 5 10
iNOS
1.00 17.43 1.15 11.16 9.78 4.36
COX-2
1.00 6.77 2.44 6.44 6.30 4.54
Actin
D
LPS (1μg/ml) - + + + + +
Butein (10uM) - - + - - -
Compound V-3 - - - 1.25 2.5 5
iNOS
1.00 16.49 2.89 13.87 12.10 8.16
COX-2
1.00 2.97 0.80 2.51 2.02 1.30
Actin

[Figure 6b]

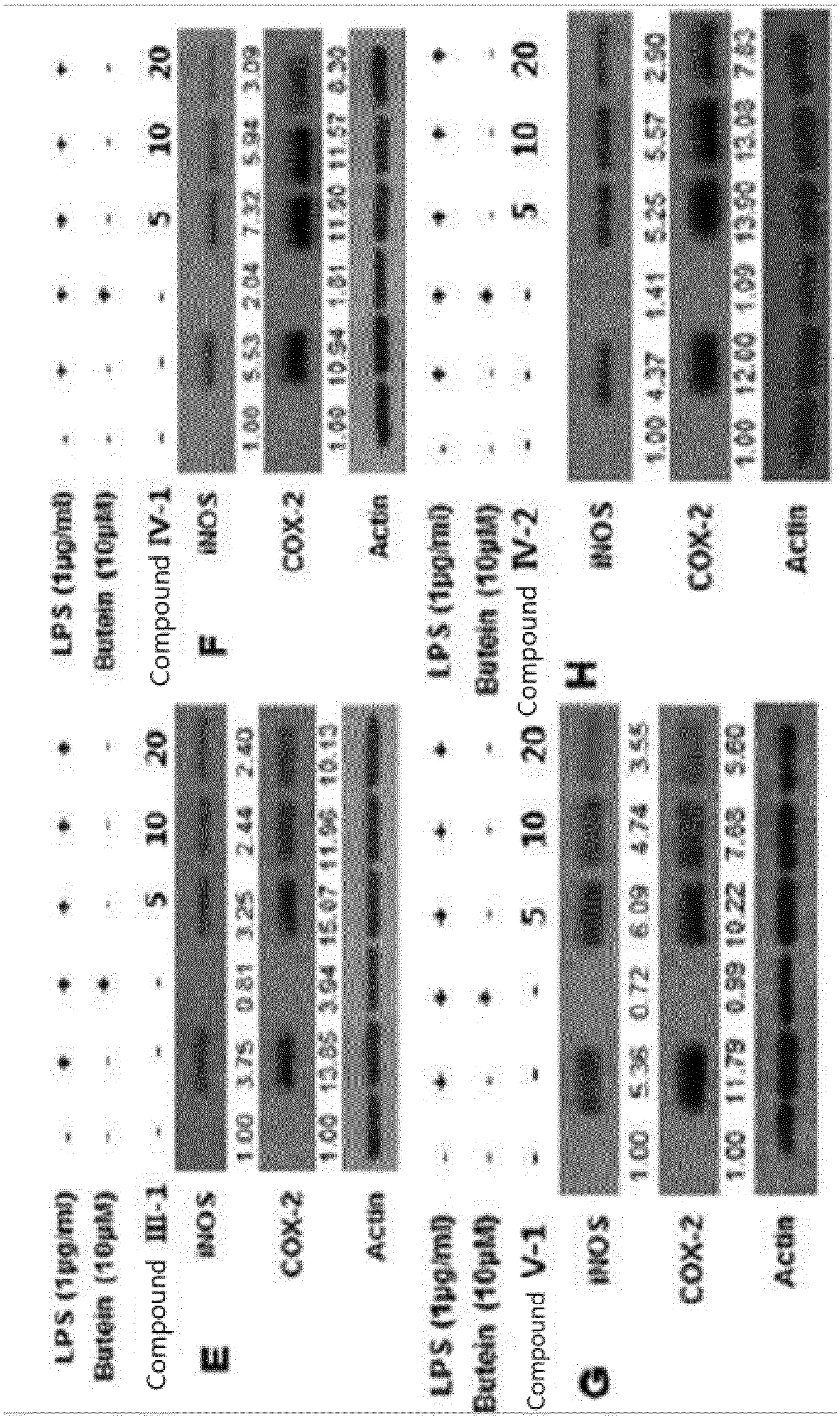
E
LPS (1μg/ml)
Butein (10μM)
Compound III-1
iNOS
1.00 3.75 0.81 3.25 2.44 2.40
COX-2
1.00 13.85 3.94 15.07 11.96 10.13
Actin
5 10 20
F
LPS (1μg/ml)
Butein (10μM)
Compound IV-1
iNOS
1.00 5.53 2.04 7.32 5.94 3.09
COX-2
1.00 10.94 1.81 11.90 11.57 8.30
Actin
5 10 20
G
LPS (1μg/ml)
Butein (10μM)
Compound V-1
iNOS
1.00 5.36 0.72 6.09 4.74 3.55
COX-2
1.00 11.79 0.99 10.22 7.68 5.60
Actin
5 10 20
H
LPS (1μg/ml)
Butein (10μM)
Compound IV-2
iNOS
1.00 4.37 1.41 5.25 5.57 2.50
COX-2
1.00 12.00 1.09 13.90 13.08 7.83
Actin
5 10 20

[Figure 6c]

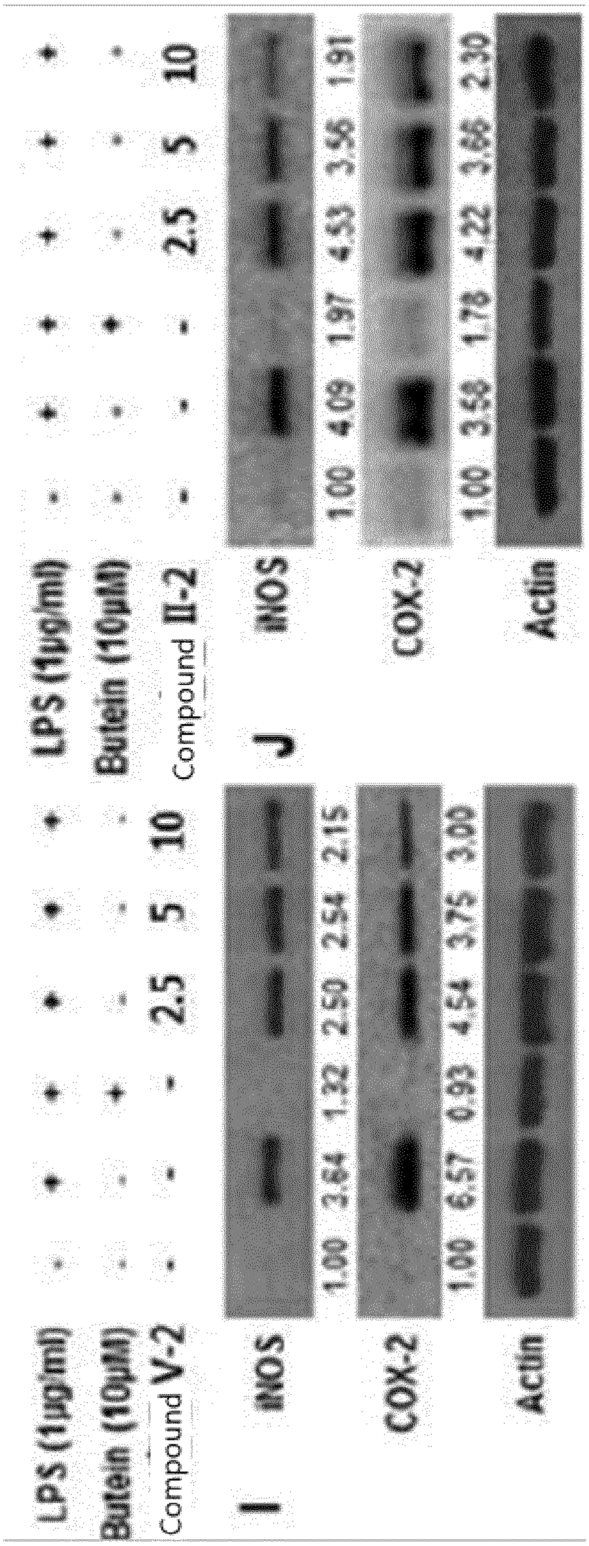
I
LPS (1μg/ml)
Butein (10μM)
Compound V-2
2.5
5
10
iNOS
1.00 3.64 1.32 2.50 2.54 2.15
COX-2
1.00 6.57 0.93 4.54 3.75 3.00
Actin
J
LPS (1μg/ml)
Butein (10μM)
Compound II-2
2.5
5
10
iNOS
1.00 4.09 1.97 4.53 3.56 1.91
COX-2
1.00 3.58 1.78 4.22 3.66 2.30
Actin

[Figure 7a]

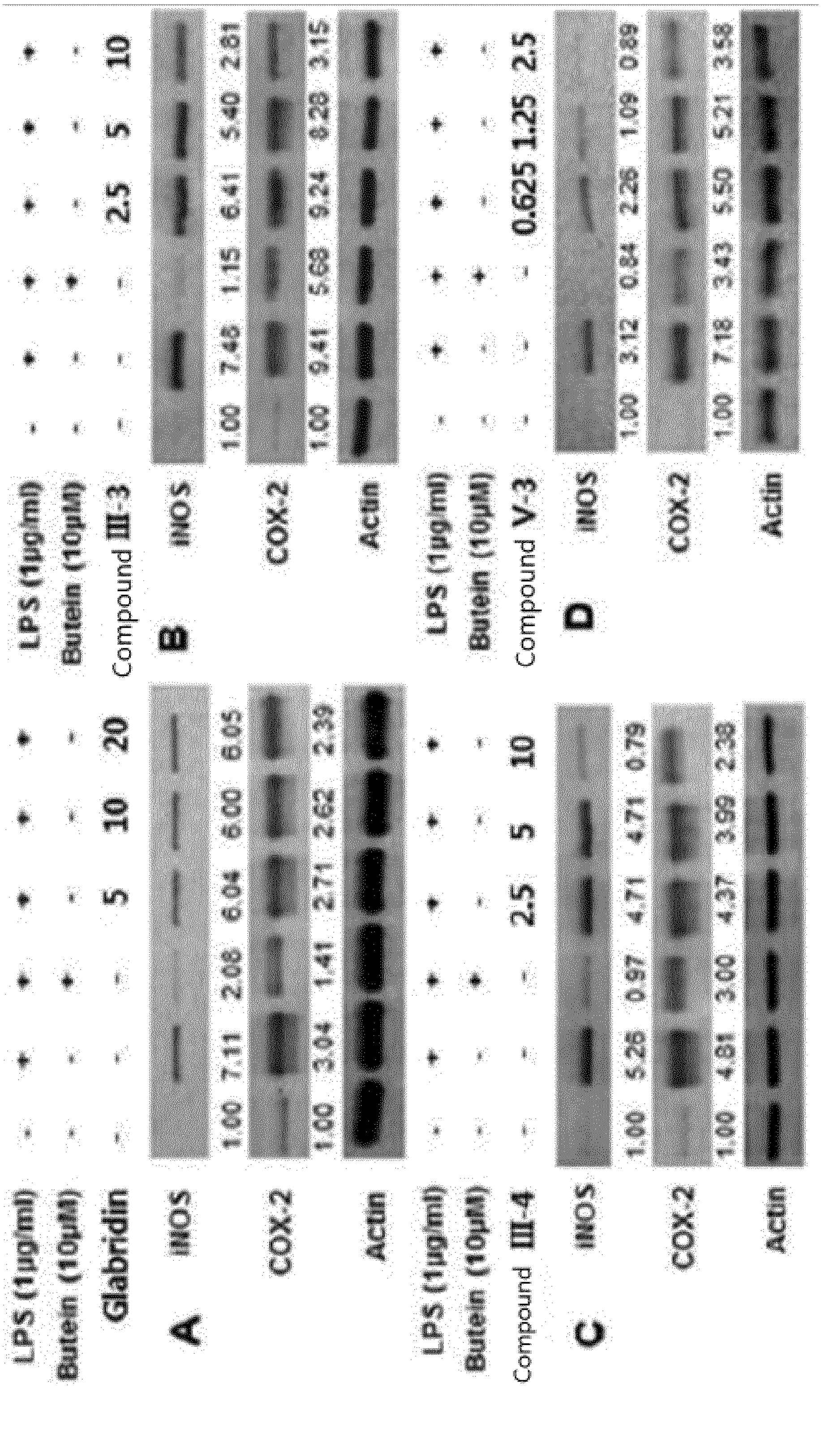
A
LPS (1μg/ml)
Butein (10μM)
Glabridin
5 10 20
iNOS
1.00 7.11 2.08 6.04 6.00 6.05
COX-2
1.00 3.04 1.41 2.71 2.62 2.39
Actin
B
Compound III-3
2.5 5 10
iNOS
1.00 7.48 1.15 6.41 5.40 2.81
COX-2
1.00 9.41 5.68 9.24 8.28 3.15
Actin
C
Compound III-4
2.5 5 10
iNOS
1.00 5.28 0.97 4.71 4.71 0.79
COX-2
1.00 4.81 3.00 4.37 3.99 2.38
Actin
D
Compound V-3
0.625 1.25 2.5
iNOS
1.00 3.12 0.84 2.26 1.09 0.89
COX-2
1.00 7.18 3.43 5.50 5.21 3.58
Actin

[Figure 7b]

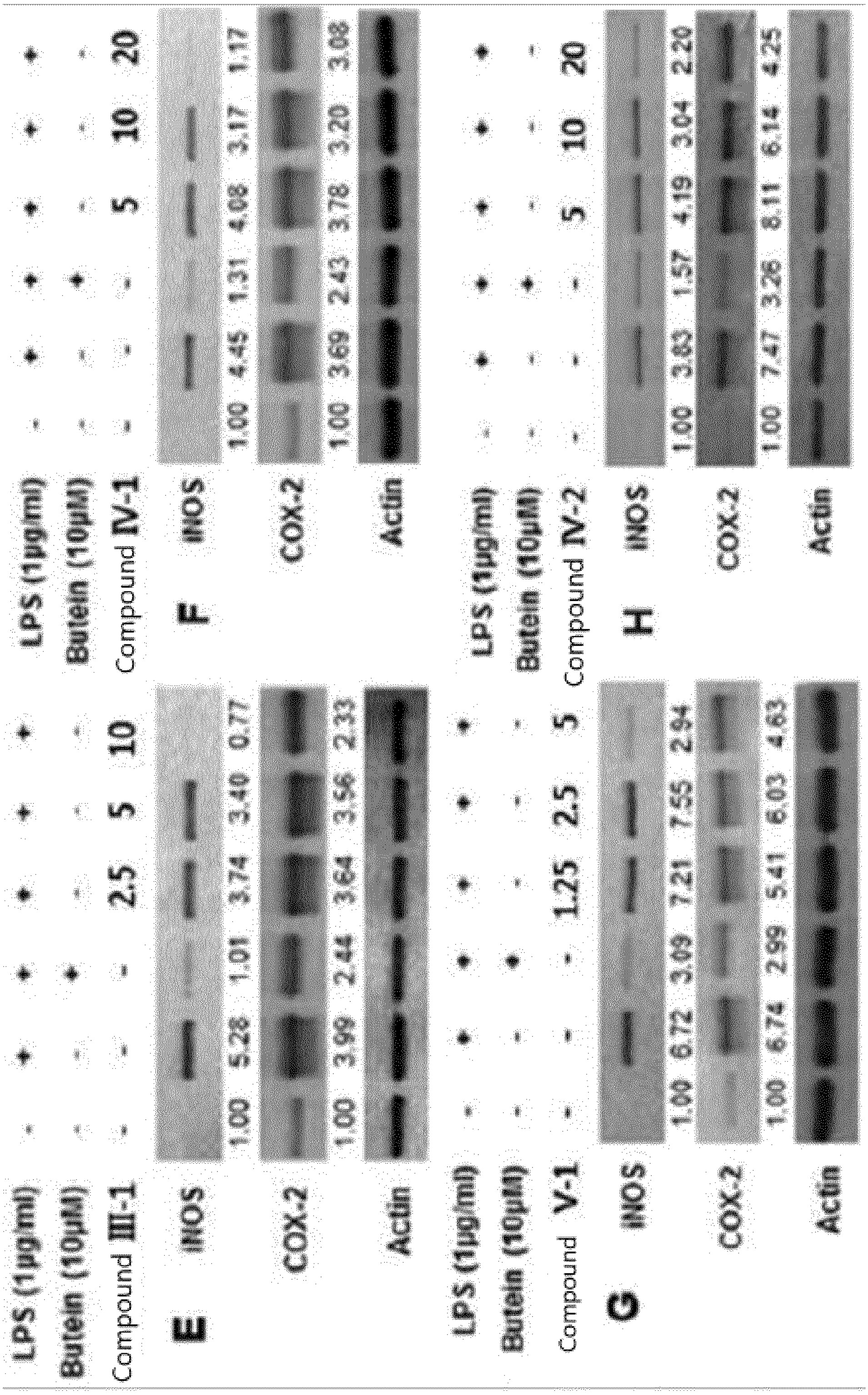
E
LPS (1µg/ml)
Butein (10µM)
Compound III-1
- - - 2.5 5 10
iNOS
1.00 5.28 1.01 3.74 3.40 0.77
COX-2
1.00 3.99 2.44 3.64 3.56 2.33
Actin
F
LPS (1µg/ml)
Butein (10µM)
Compound IV-1
5 10 20
iNOS
1.00 4.45 1.31 4.08 3.17 1.17
COX-2
1.00 3.69 2.43 3.78 3.20 3.08
Actin
G
LPS (1µg/ml)
Butein (10µM)
Compound V-1
- - - 1.25 2.5 5
iNOS
1.00 6.72 3.09 7.21 7.55 2.94
COX-2
1.00 6.74 2.99 5.41 6.03 4.63
Actin
H
LPS (1µg/ml)
Butein (10µM)
Compound IV-2
- - - 5 10 20
iNOS
1.00 3.83 1.57 4.19 3.04 2.20
COX-2
1.00 7.47 3.26 8.11 6.14 4.25
Actin

[Figure 7c]

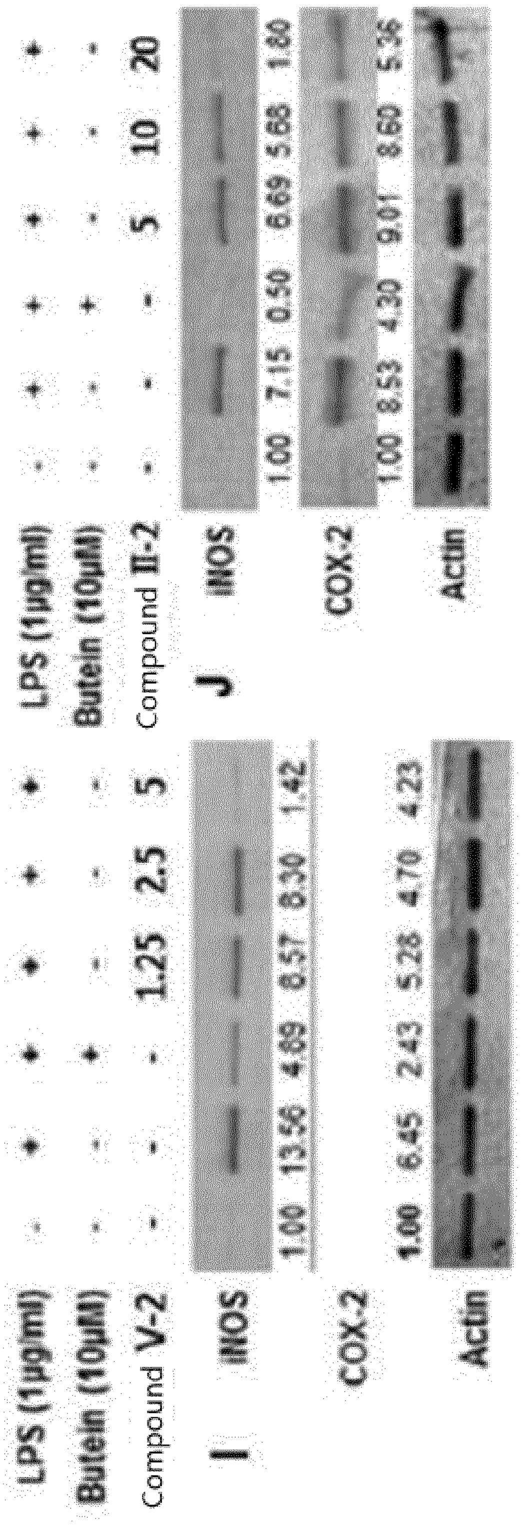
I
LPS (1μg/ml)
Butein (10μM)
Compound V-2
- - - 1.25 2.5 5
iNOS
1.00 13.56 4.69 6.57 8.30 1.42
COX-2
1.00 6.45 2.43 5.28 4.70 4.23
Actin
J
LPS (1μg/ml)
Butein (10μM)
Compound II-2
- - - 5 10 20
iNOS
1.00 7.15 0.50 6.69 5.68 1.80
COX-2
1.00 8.53 4.30 9.01 8.60 5.36
Actin

[Figure 8a]

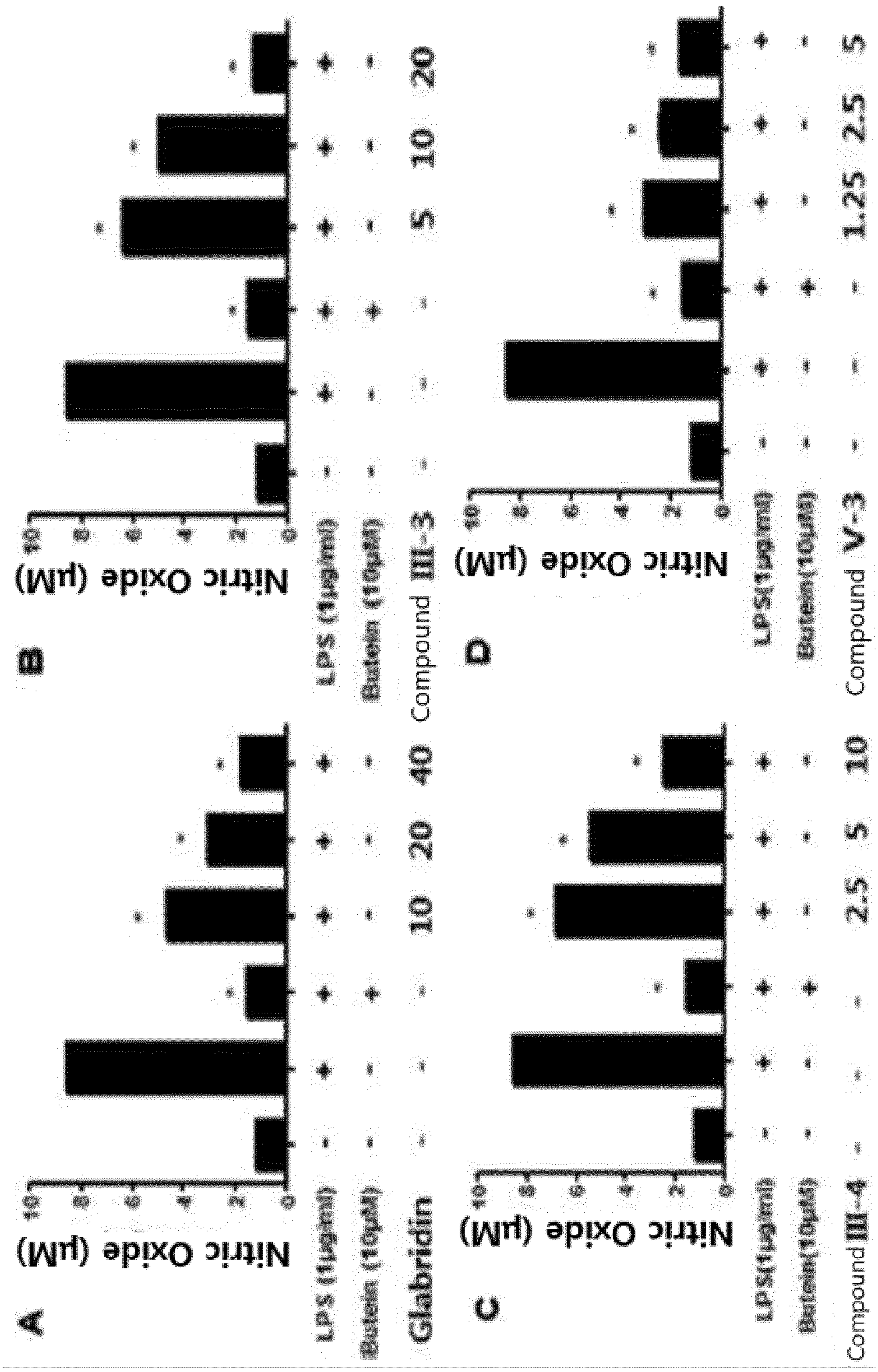
A
B
C
D
Nitric Oxide (μM)
LPS (1μg/ml)
Butein (10μM)
Glabridin
Compound III-3
Compound III-4
Compound V-3
LPS(1μg/ml)
Butein(10μM)

[Figure 8b]

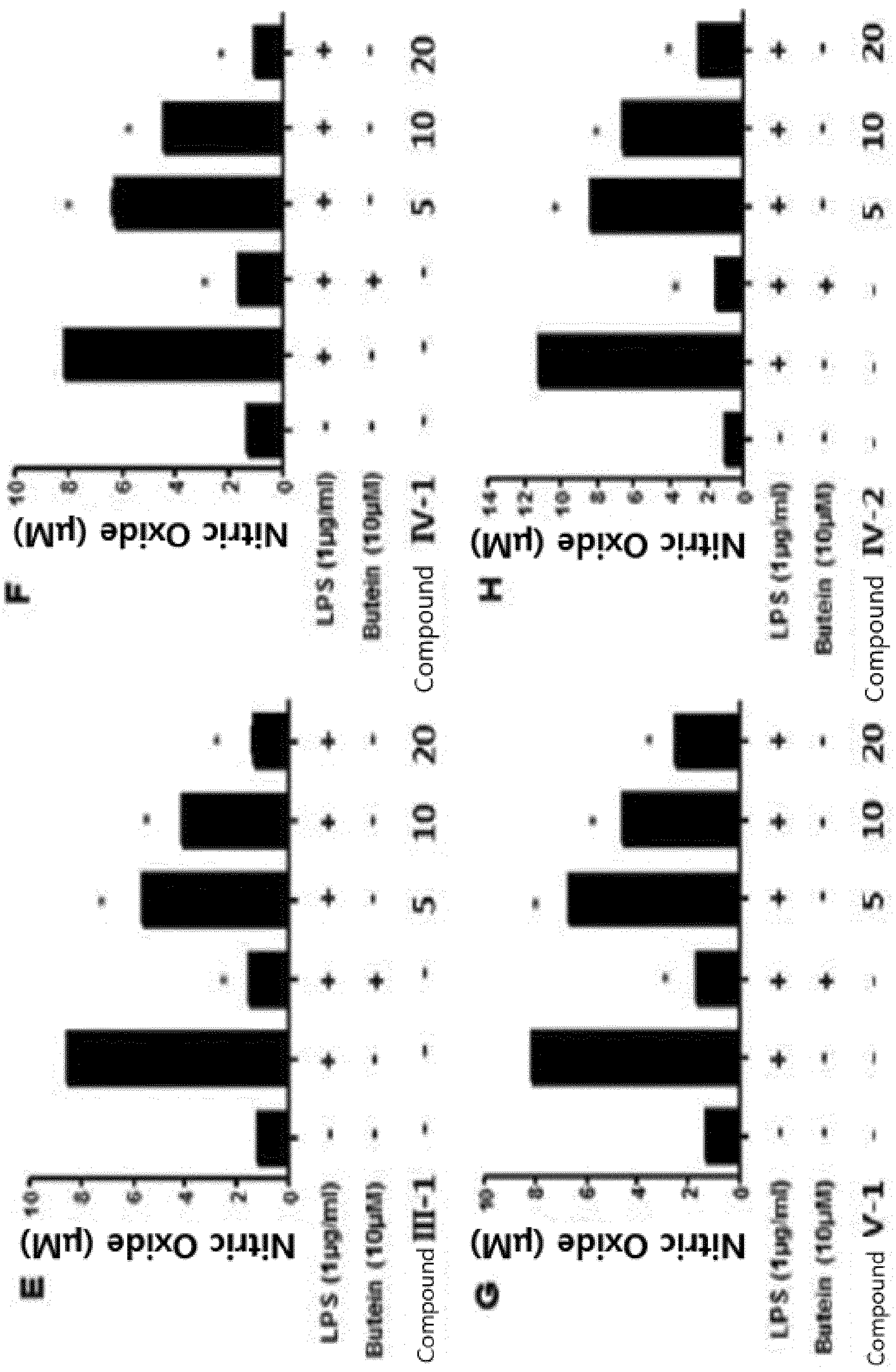
E
Nitric Oxide (μM)
LPS (1μg/ml)
Butein (10μM)
Compound III-1
5
10
20
F
Nitric Oxide (μM)
LPS (1μg/ml)
Butein (10μM)
Compound IV-1
5
10
20
G
Nitric Oxide (μM)
LPS (1μg/ml)
Butein (10μM)
Compound V-1
5
10
20
H
Nitric Oxide (μM)
LPS (1μg/ml)
Butein (10μM)
Compound IV-2
5
10
20

[Figure 8c]

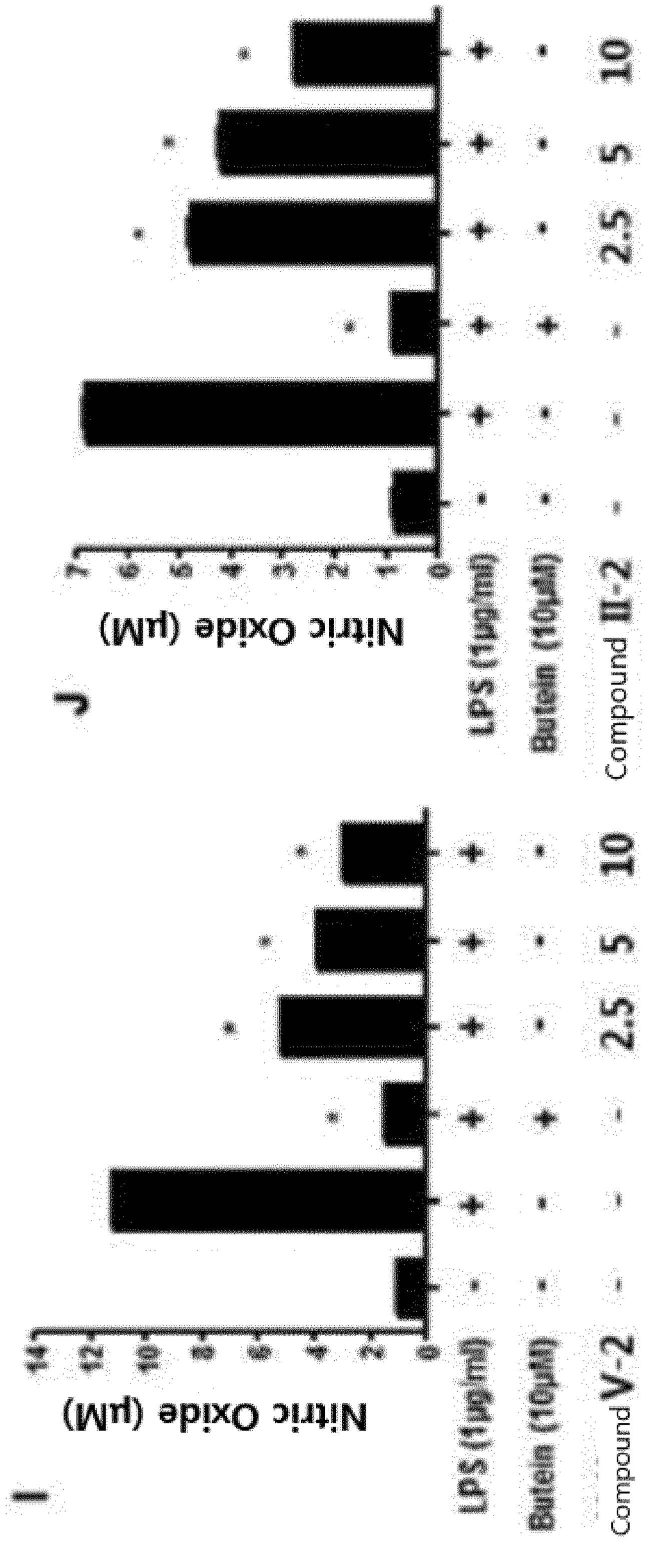

I
Nitric Oxide (μM)
LPS (1μg/ml)
Butein (10μM)
Compound V-2
J
Nitric Oxide (μM)
LPS (1μg/ml)
Butein (10μM)
Compound II-2

[Figure 9a]

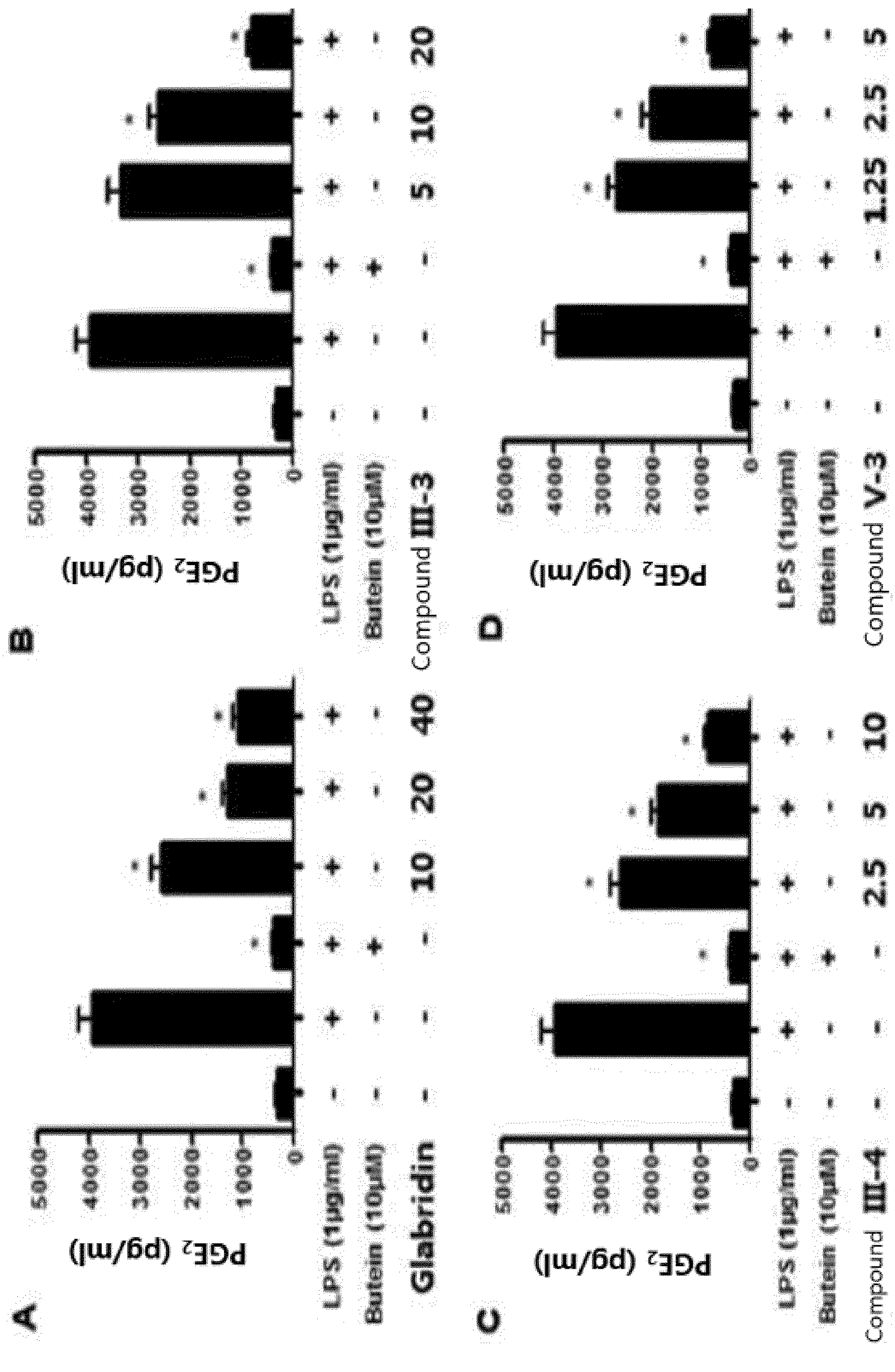
A
PGE2 (pg/ml)
5000
4000
3000
2000
1000
0
LPS (1μg/ml)
Butein (10μM)
Glabridin
10
20
40
B
Compound III-3
5
10
20
C
Compound III-4
2.5
5
10
D
Compound V-3
1.25
2.5
5

[Figure 9b]

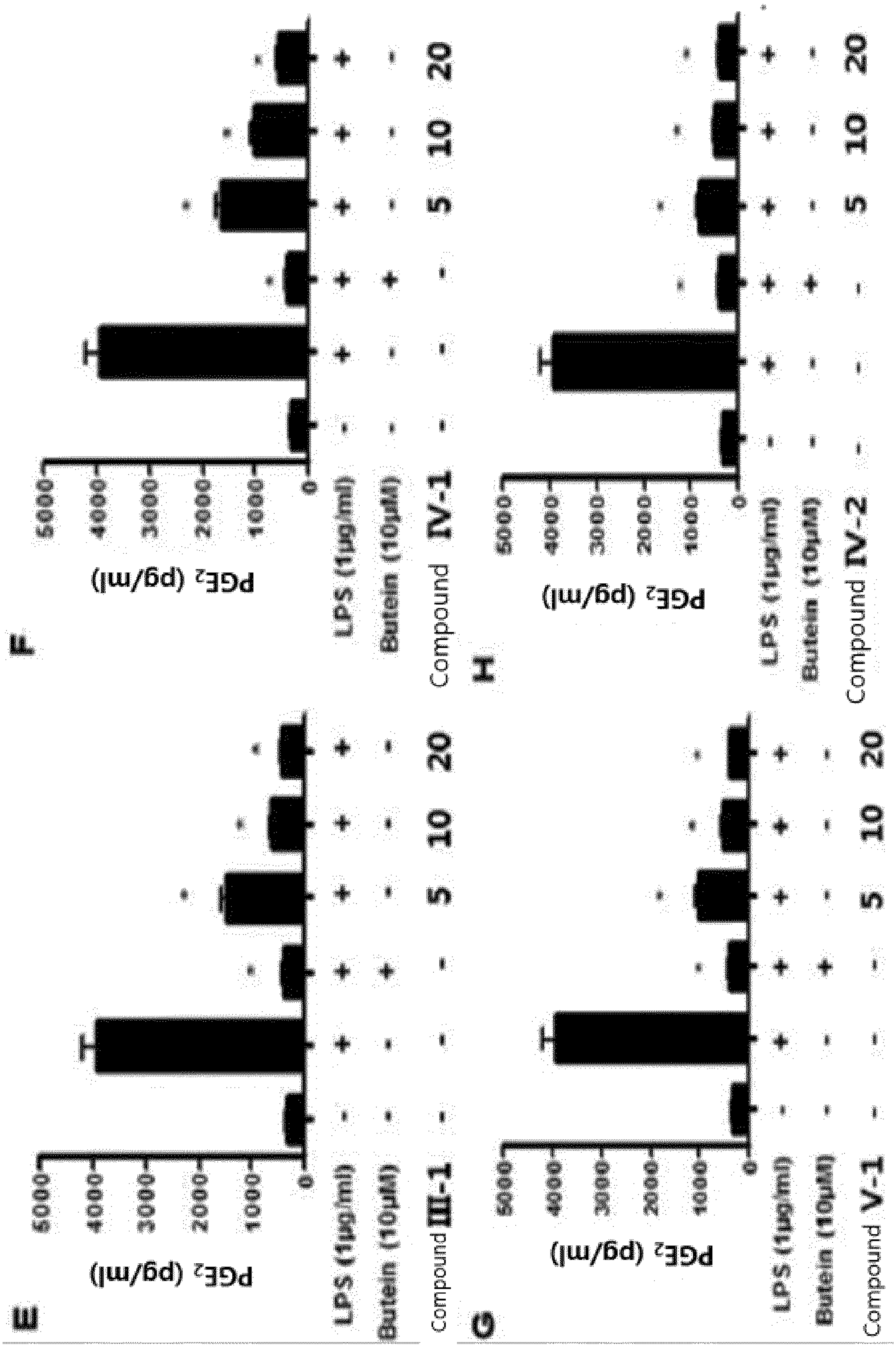
E
PGE2 (pg/ml)
LPS (1μg/ml)
Butein (10μM)
Compound III-1
F
Compound IV-1
G
Compound V-1
H
Compound IV-2

[Figure 9c]

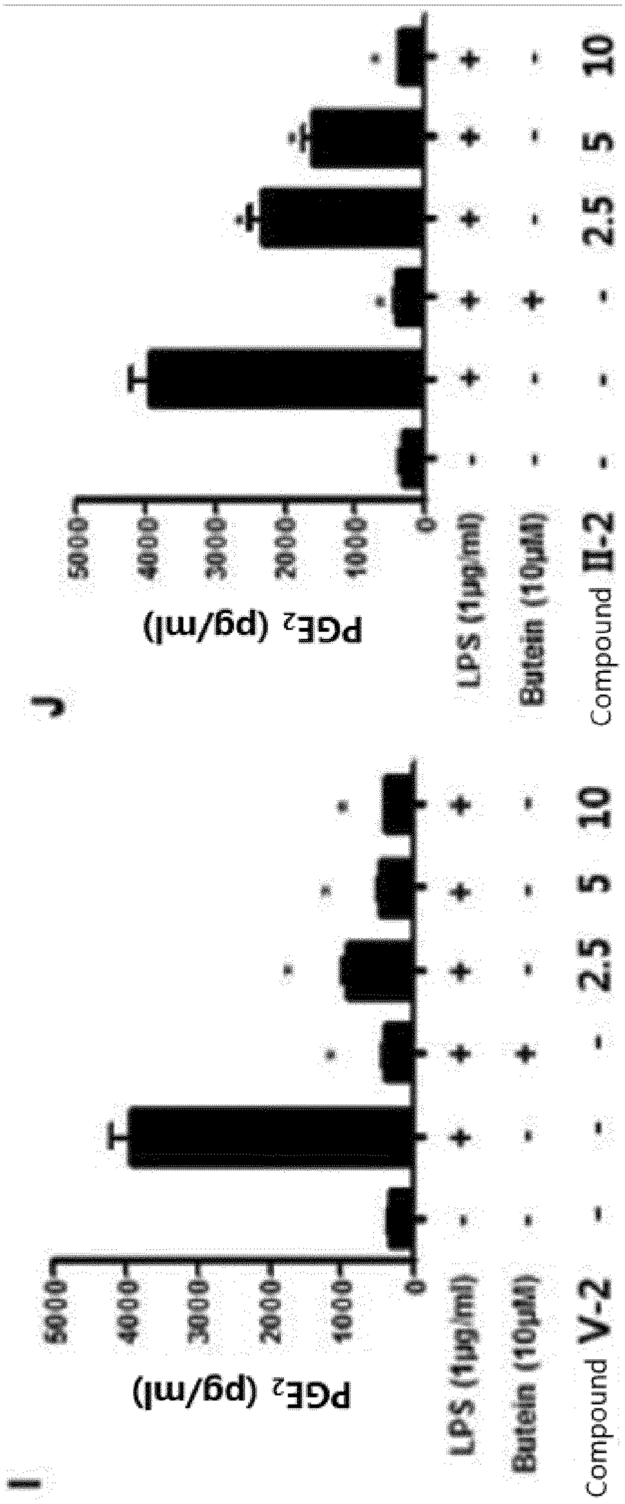
I
PGE2 (pg/ml)
LPS (1μg/ml)
Butein (10μM)
Compound V-2
2.5 5 10
J
PGE2 (pg/ml)
LPS (1μg/ml)
Butein (10μM)
Compound II-2
2.5 5 10

[Figure 10a]

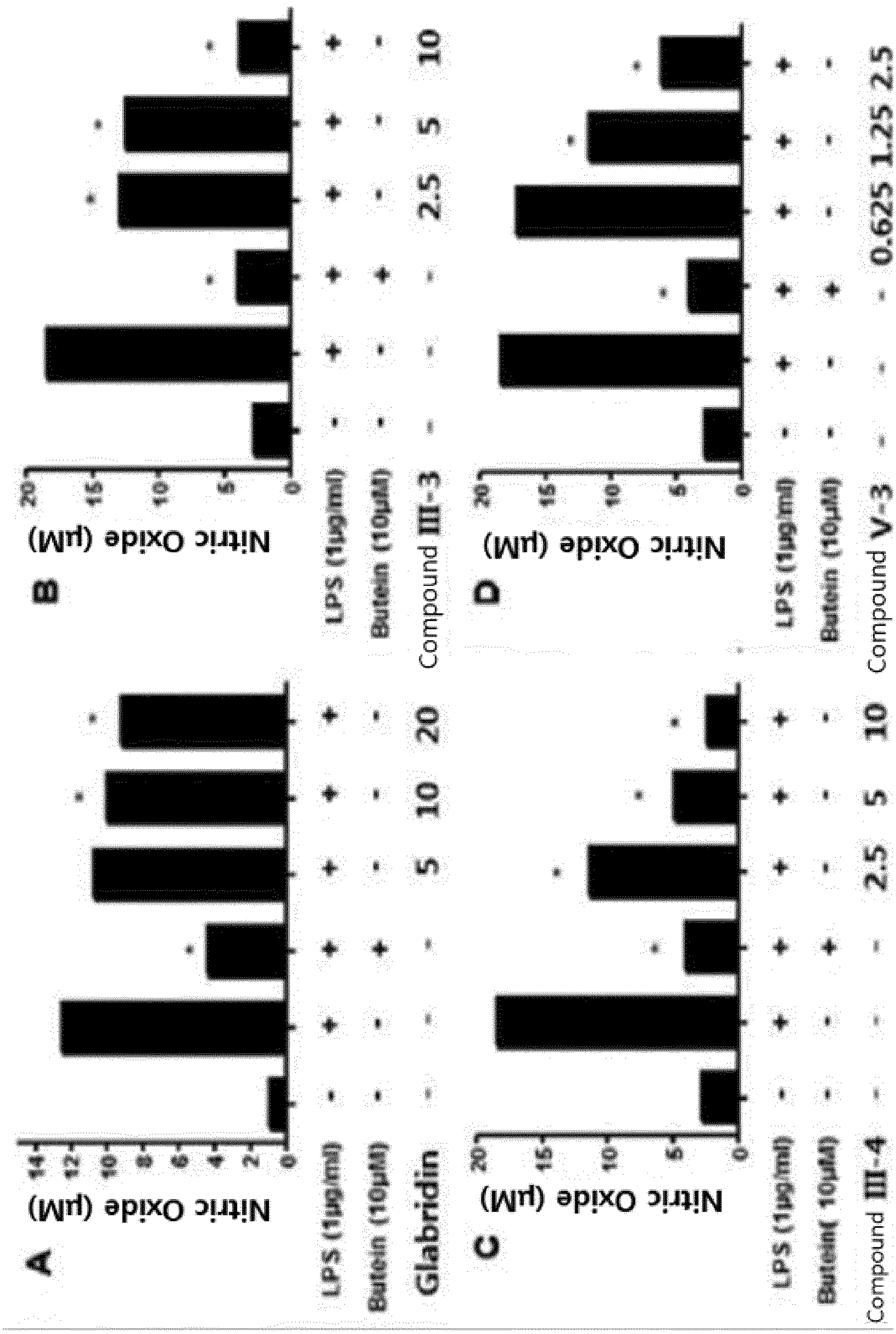
A
Nitric Oxide (μM)
LPS (1μg/ml)
Butein (10μM)
Glabridin
5
10
20
B
Nitric Oxide (μM)
LPS (1μg/ml)
Butein (10μM)
Compound III-3
2.5
5
10
C
Nitric Oxide (μM)
LPS (1μg/ml)
Butein( 10μM)
Compound III-4
2.5
5
10
D
Nitric Oxide (μM)
LPS (1μg/ml)
Butein (10μM)
Compound V-3
0.625
1.25
2.5

[Figure 10b]

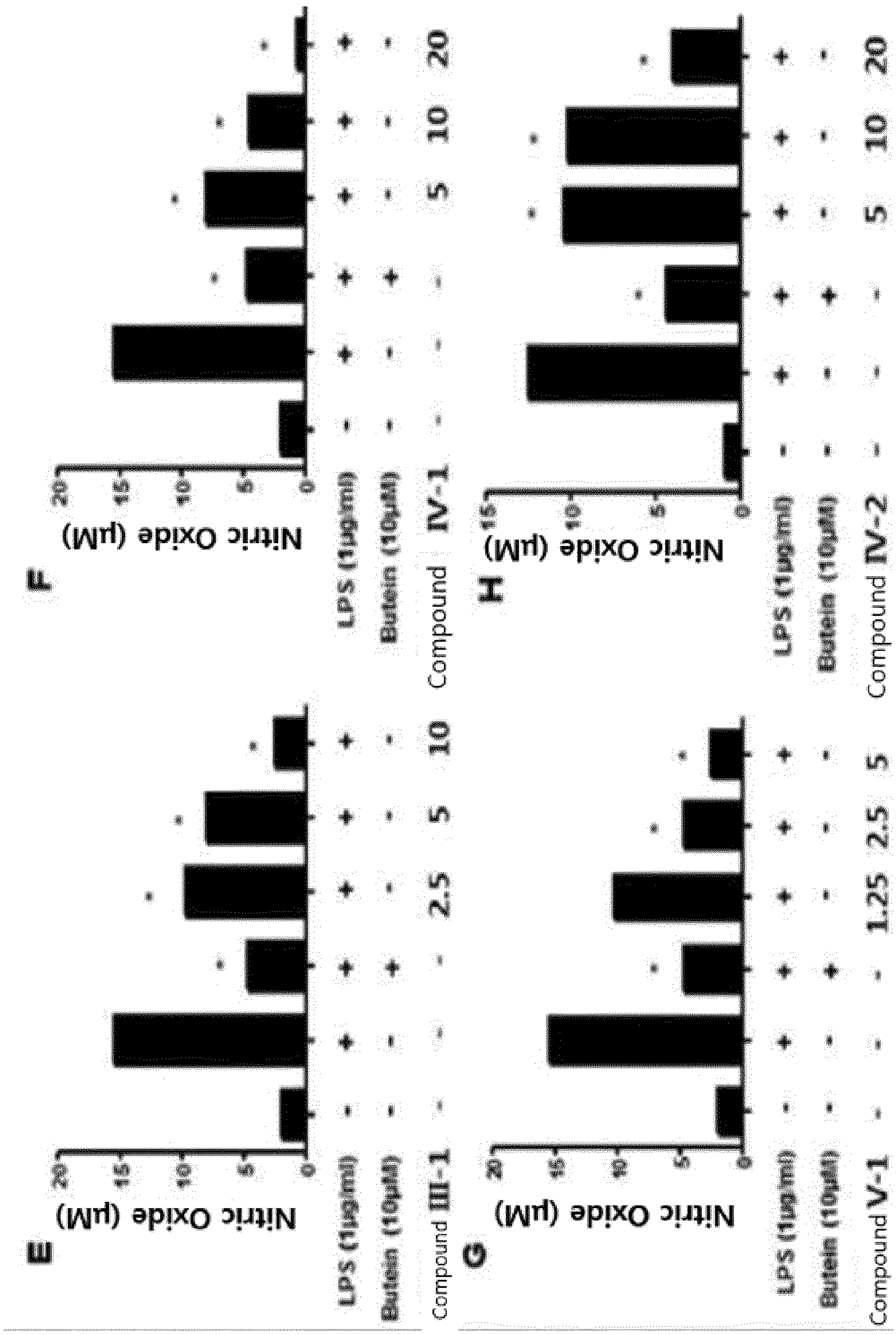
E
Nitric Oxide (μM)
LPS (1μg/ml)
Butein (10μM)
Compound III-1
F
Nitric Oxide (μM)
LPS (1μg/ml)
Butein (10μM)
Compound IV-1
G
Nitric Oxide (μM)
LPS (1μg/ml)
Butein (10μM)
Compound V-1
H
Nitric Oxide (μM)
LPS (1μg/ml)
Butein (10μM)
Compound IV-2

[Figure 10c]

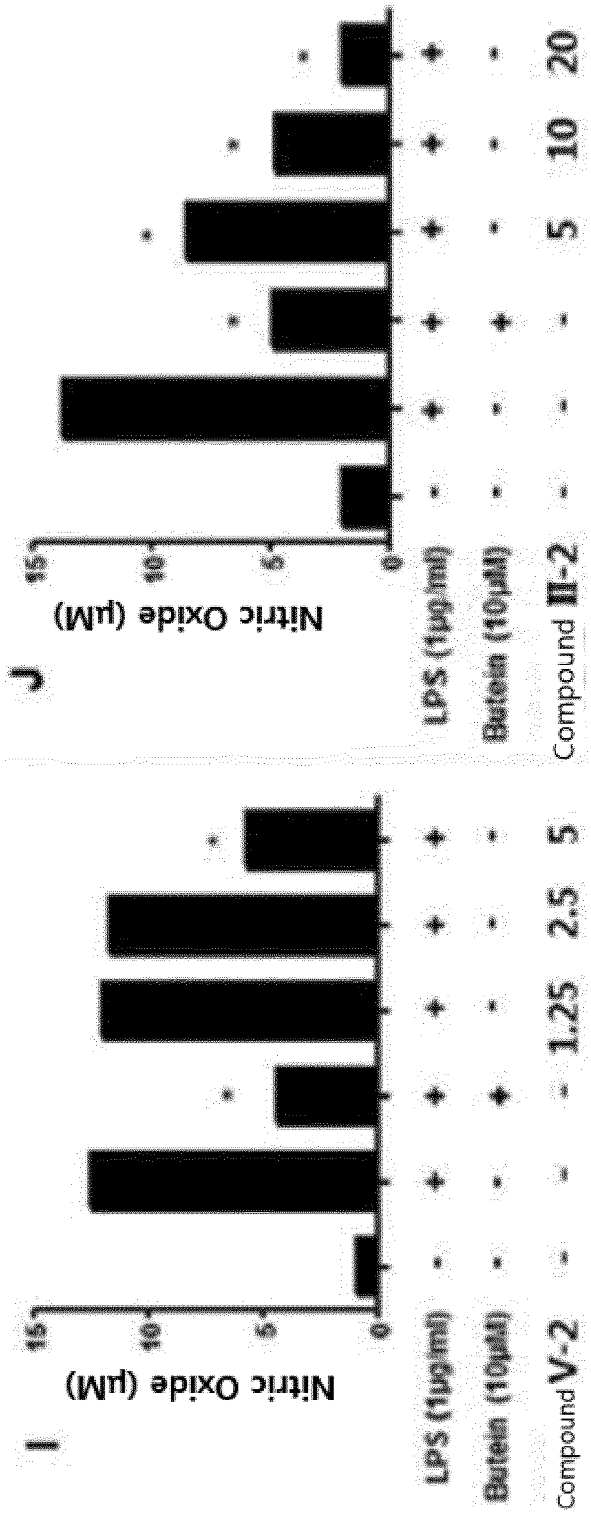
I
J
Nitric Oxide (μM)
0
5
10
15
LPS (1μg/ml)
Butein (10μM)
Compound V-2
1.25
2.5
5
Compound II-2
5
10
20

[Figure 11a]

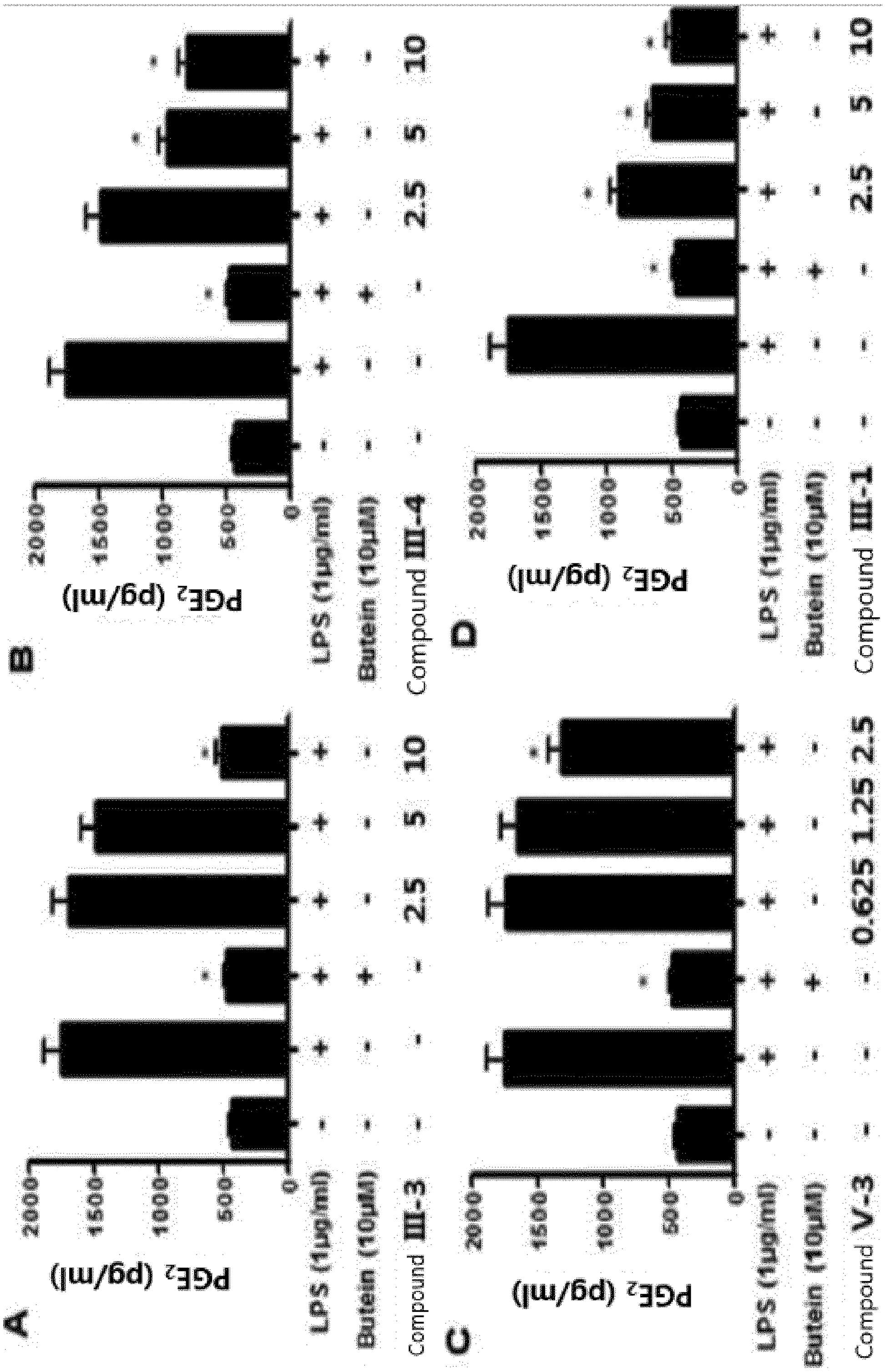
A
PGE2 (pg/ml)
LPS (1μg/ml)
Butein (10μM)
Compound III-3
B
Compound III-4
C
Compound V-3
0.625 1.25 2.5
D
Compound III-1

[Figure 11b]

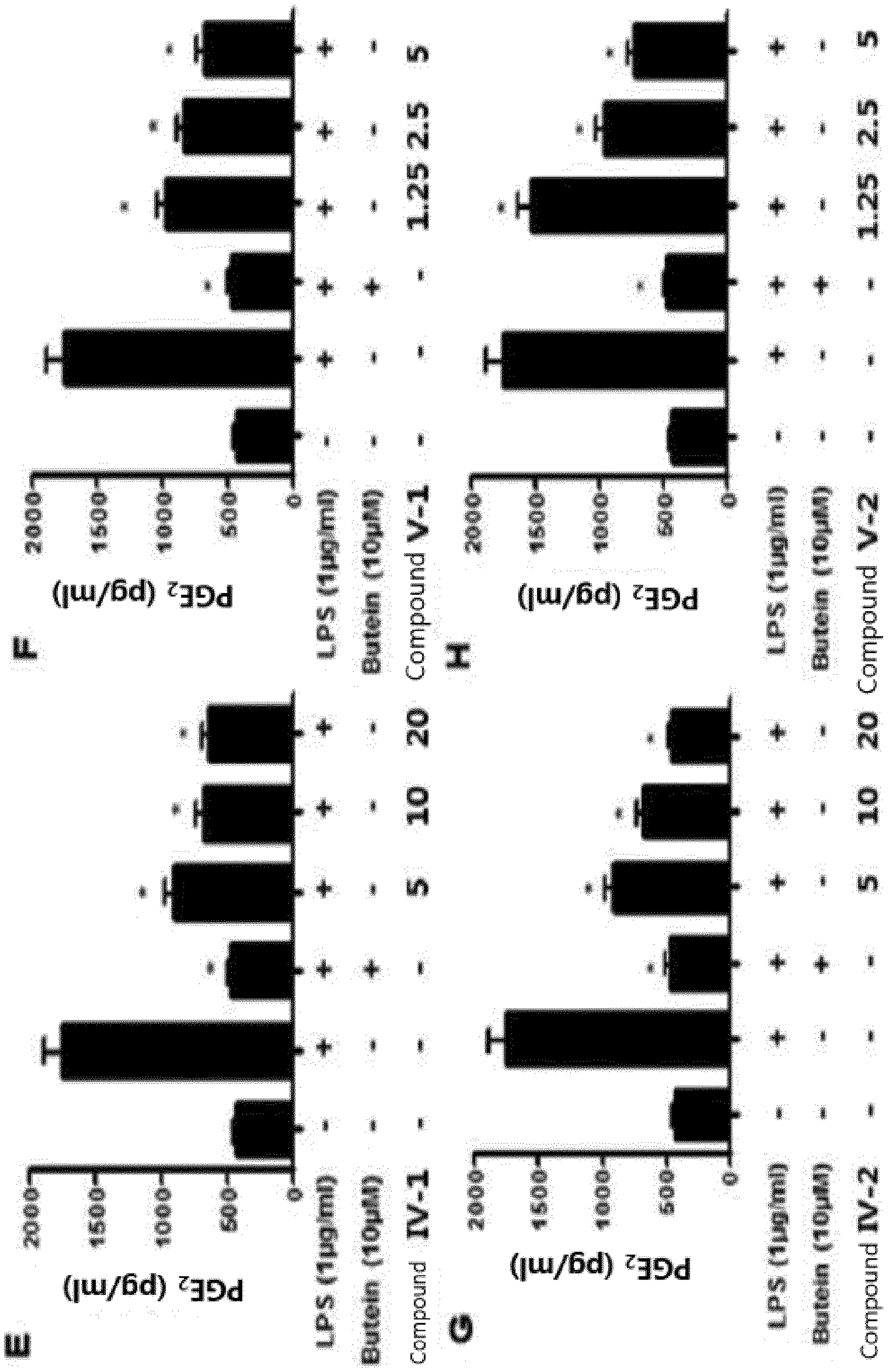

E
PGE2 (pg/ml)
LPS (1μg/ml)
Butein (10μM)
Compound IV-1
F
Compound V-1
G
Compound IV-2
H
Compound V-2

[Figure 11c]

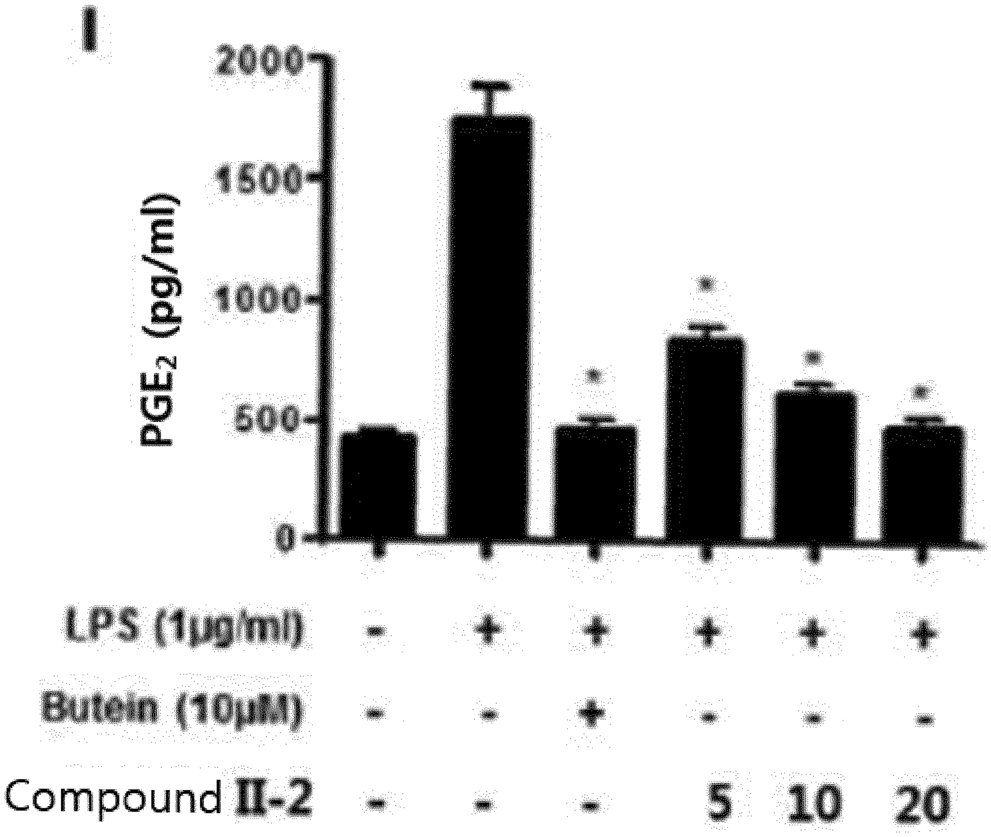
I
2000
1500
1000
500
0
PGE2 (pg/ml)
*
*
*
*
LPS (1μg/ml) - + + + + +
Butein (10μM) - - + - - -
Compound II-2 - - - 5 10 20

[Figure 12a]

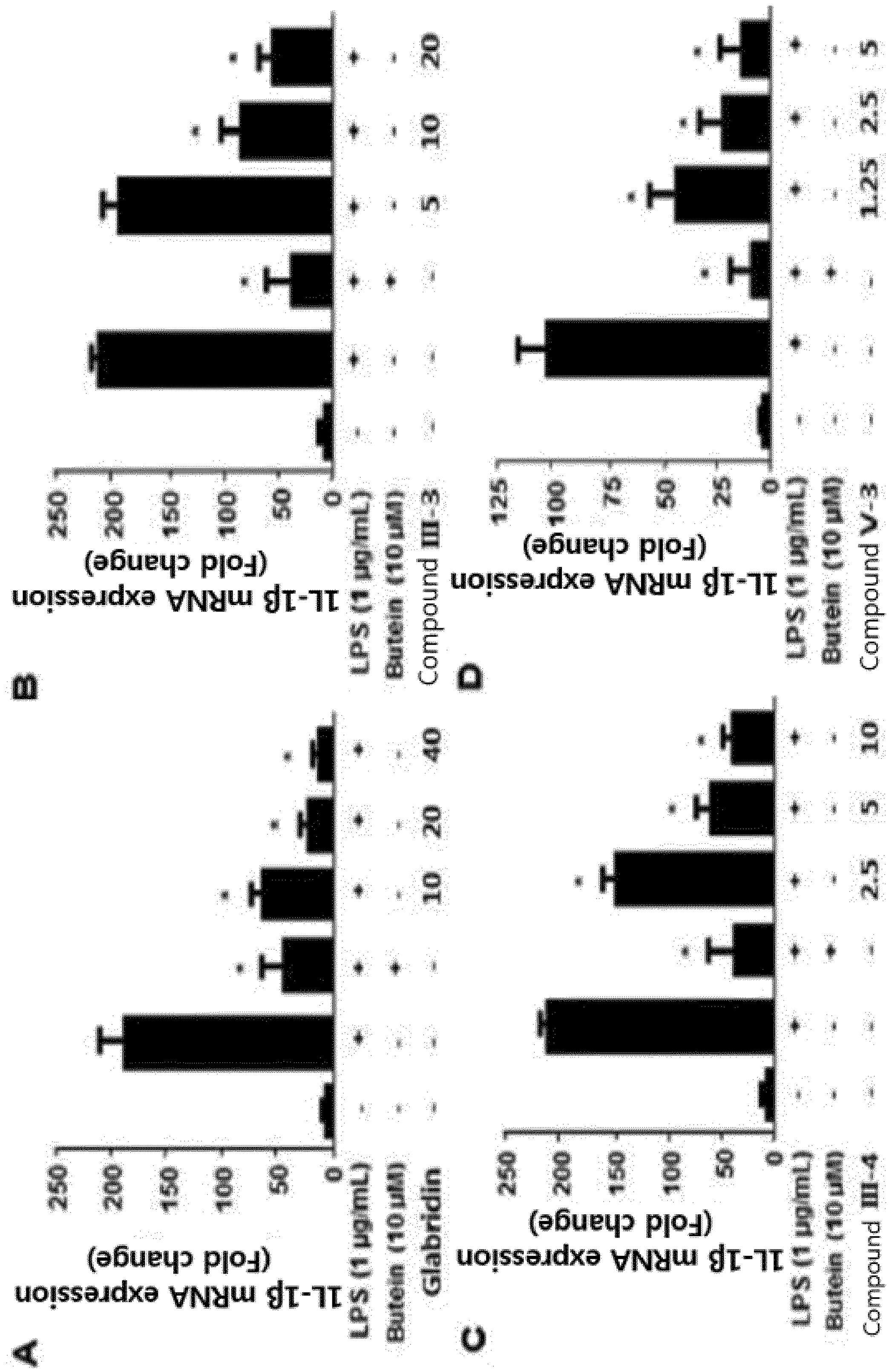
A
1L-1β mRNA expression (Fold change)
LPS (1 μg/mL)
Butein (10 μM)
Glabridin
B
1L-1β mRNA expression (Fold change)
LPS (1 μg/mL)
Butein (10 μM)
Compound III-3
C
1L-1β mRNA expression (Fold change)
LPS (1 μg/mL)
Butein (10 μM)
Compound III-4
D
1L-1β mRNA expression (Fold change)
LPS (1 μg/mL)
Butein (10 μM)
Compound V-3

[Figure 12b]

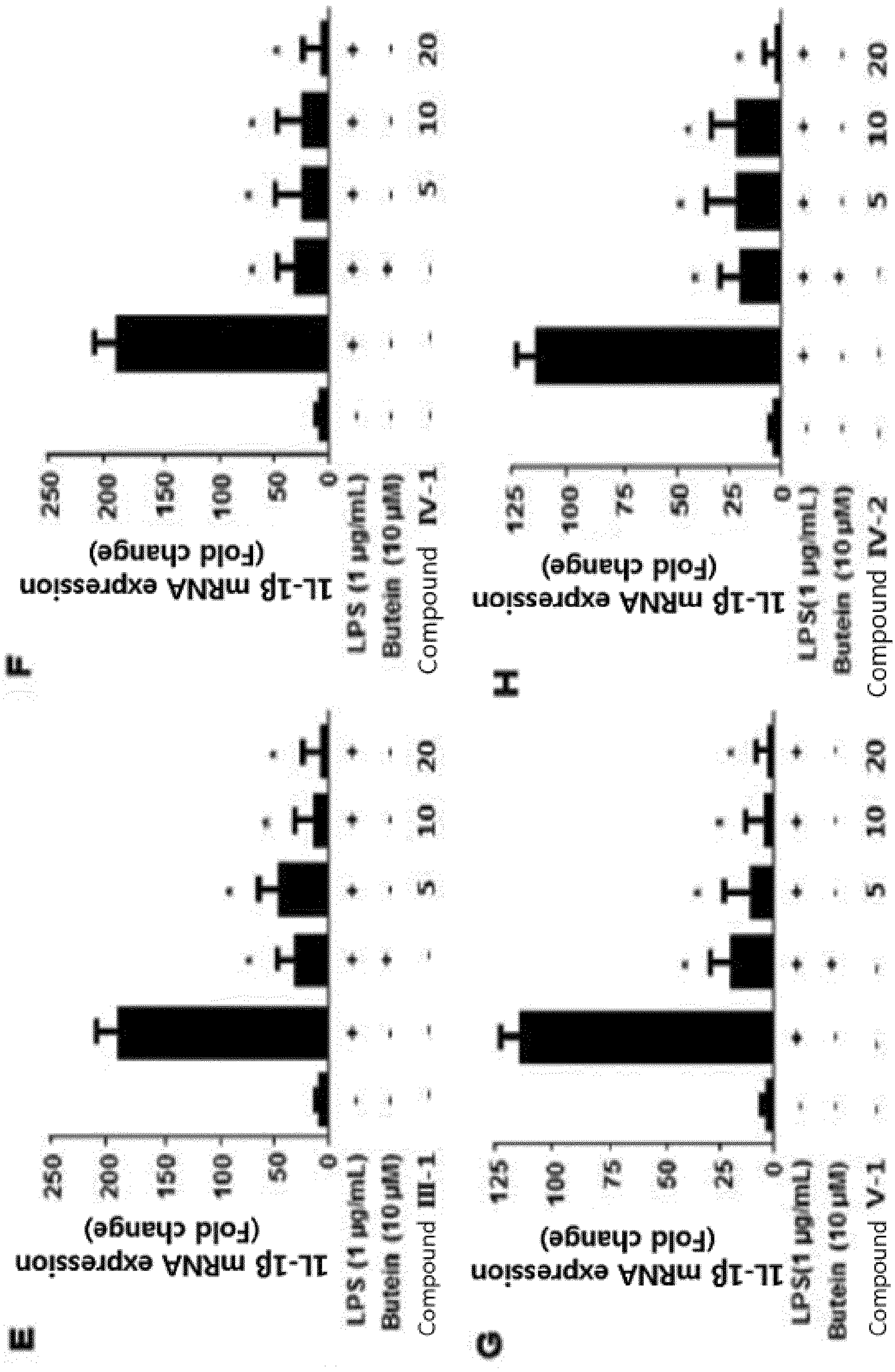
E
1L-1β mRNA expression
(Fold change)
LPS (1 μg/mL)
Butein (10 μM)
Compound III-1
F
1L-1β mRNA expression
(Fold change)
LPS (1 μg/mL)
Butein (10 μM)
Compound IV-1
G
1L-1β mRNA expression
(Fold change)
LPS(1 μg/mL)
Butein (10 μM)
Compound V-1
H
1L-1β mRNA expression
(Fold change)
LPS(1 μg/mL)
Butein (10 μM)
Compound IV-2

[Figure 12c]

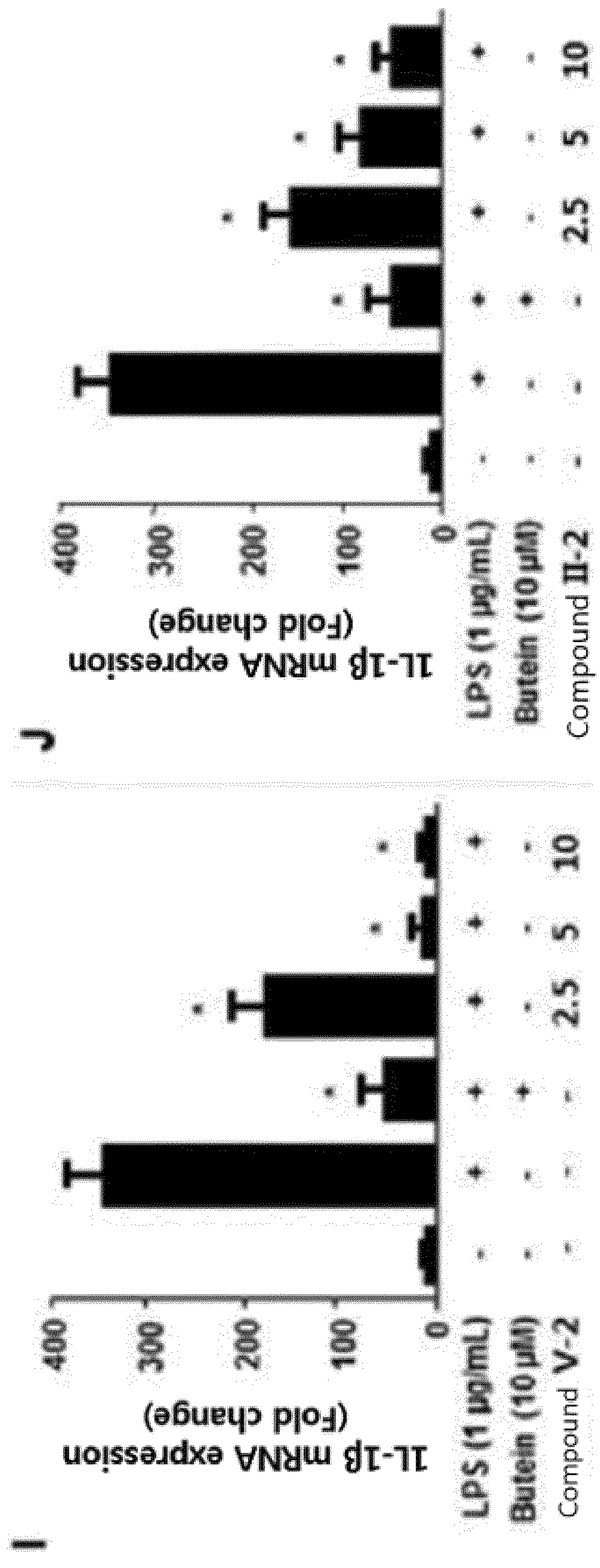

I
1L-1β mRNA expression (Fold change)
400
300
200
100
0
LPS (1 μg/mL)
Butein (10 μM)
Compound V-2
2.5
5
10
J
1L-1β mRNA expression (Fold change)
400
300
200
100
0
LPS (1 μg/mL)
Butein (10 μM)
Compound II-2
2.5
5
10

[Figure 13a]

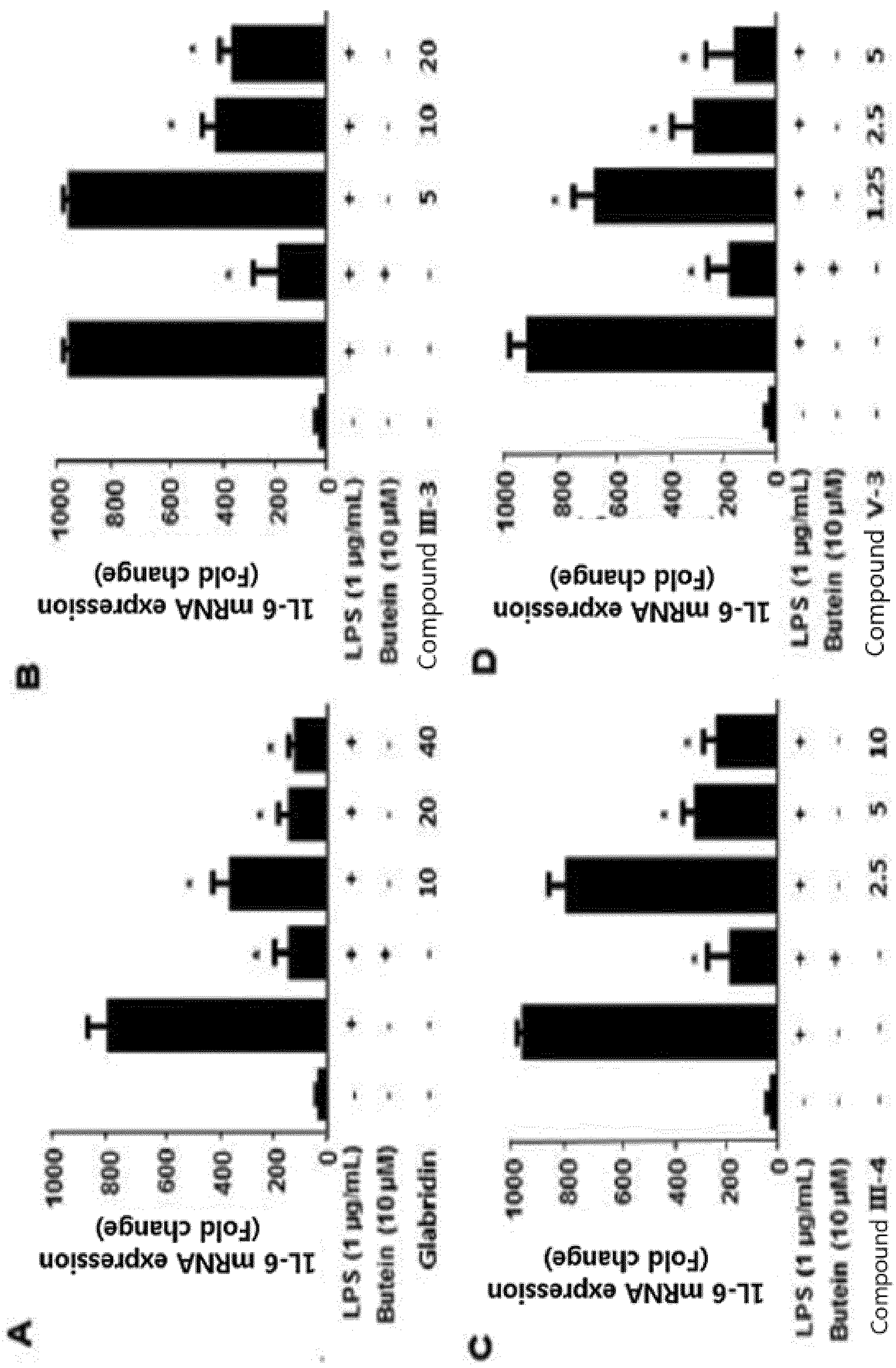
A
IL-6 mRNA expression (Fold change)
1000
800
600
400
200
0
LPS (1 μg/mL)
Butein (10 μM)
Glabridin
10
20
40
B
IL-6 mRNA expression (Fold change)
LPS (1 μg/mL)
Butein (10 μM)
Compound Ⅲ-3
5
10
20
C
IL-6 mRNA expression (Fold change)
LPS (1 μg/mL)
Butein (10 μM)
Compound Ⅲ-4
2.5
5
10
D
IL-6 mRNA expression (Fold change)
LPS (1 μg/mL)
Butein (10 μM)
Compound V-3
1.25
2.5
5

[Figure 13b]

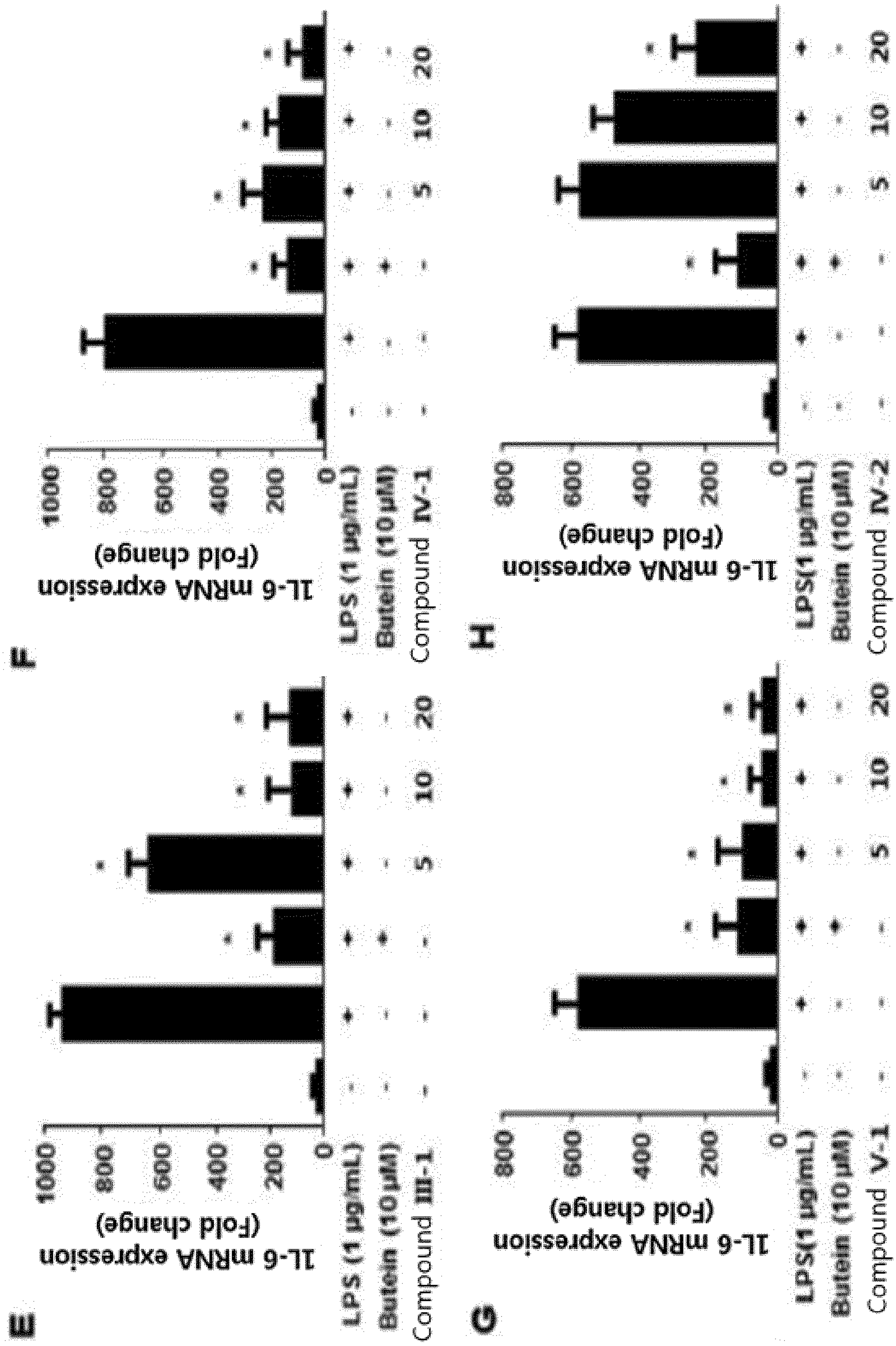
E
IL-6 mRNA expression (Fold change)
LPS (1 μg/mL)
Butein (10 μM)
Compound III-1
F
IL-6 mRNA expression (Fold change)
LPS (1 μg/mL)
Butein (10 μM)
Compound IV-1
G
IL-6 mRNA expression (Fold change)
LPS(1 μg/mL)
Butein (10 μM)
Compound V-1
H
IL-6 mRNA expression (Fold change)
LPS(1 μg/mL)
Butein (10 μM)
Compound IV-2

[Figure 13c]

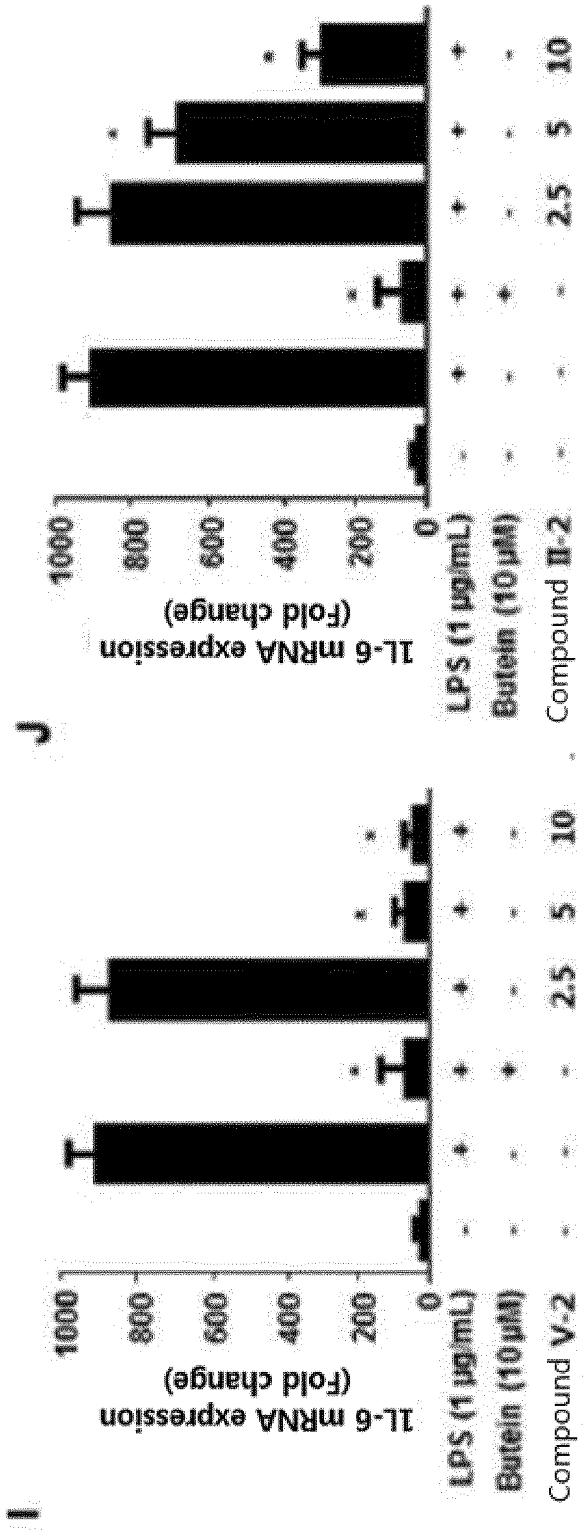
I
IL-6 mRNA expression (Fold change)
1000
800
600
400
200
0
LPS (1 μg/mL)
Butein (10 μM)
Compound V-2
2.5
5
10
J
IL-6 mRNA expression (Fold change)
1000
800
600
400
200
0
LPS (1 μg/mL)
Butein (10 μM)
Compound II-2
2.5
5
10

[Figure 14a]

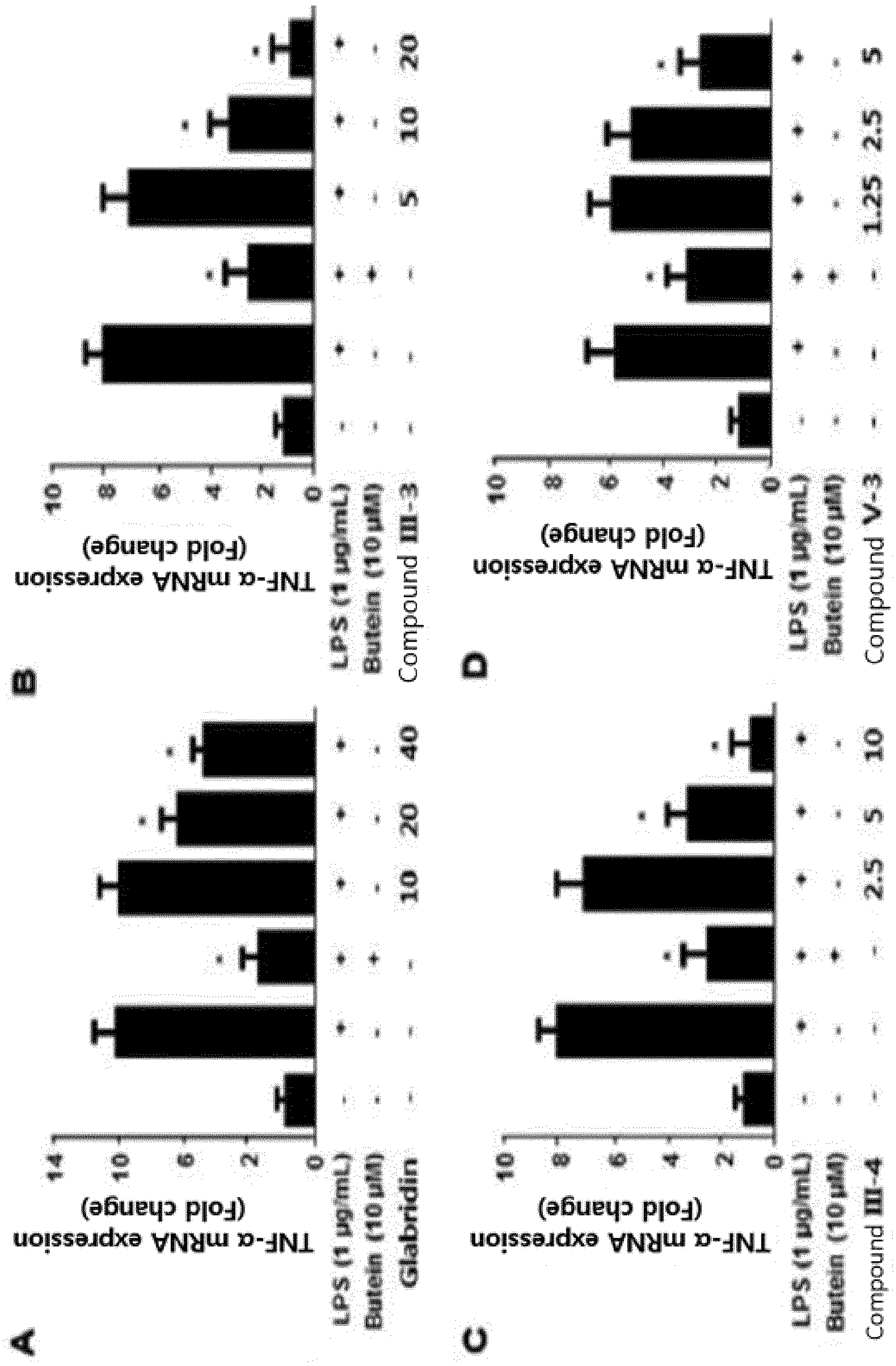
A
B
C
D
TNF-α mRNA expression (Fold change)
LPS (1 μg/mL)
Butein (10 μM)
Glabridin
Compound III-3
Compound III-4
Compound V-3
10
20
40
5
10
20
2.5
5
10
1.25
2.5
5

[Figure 14b]

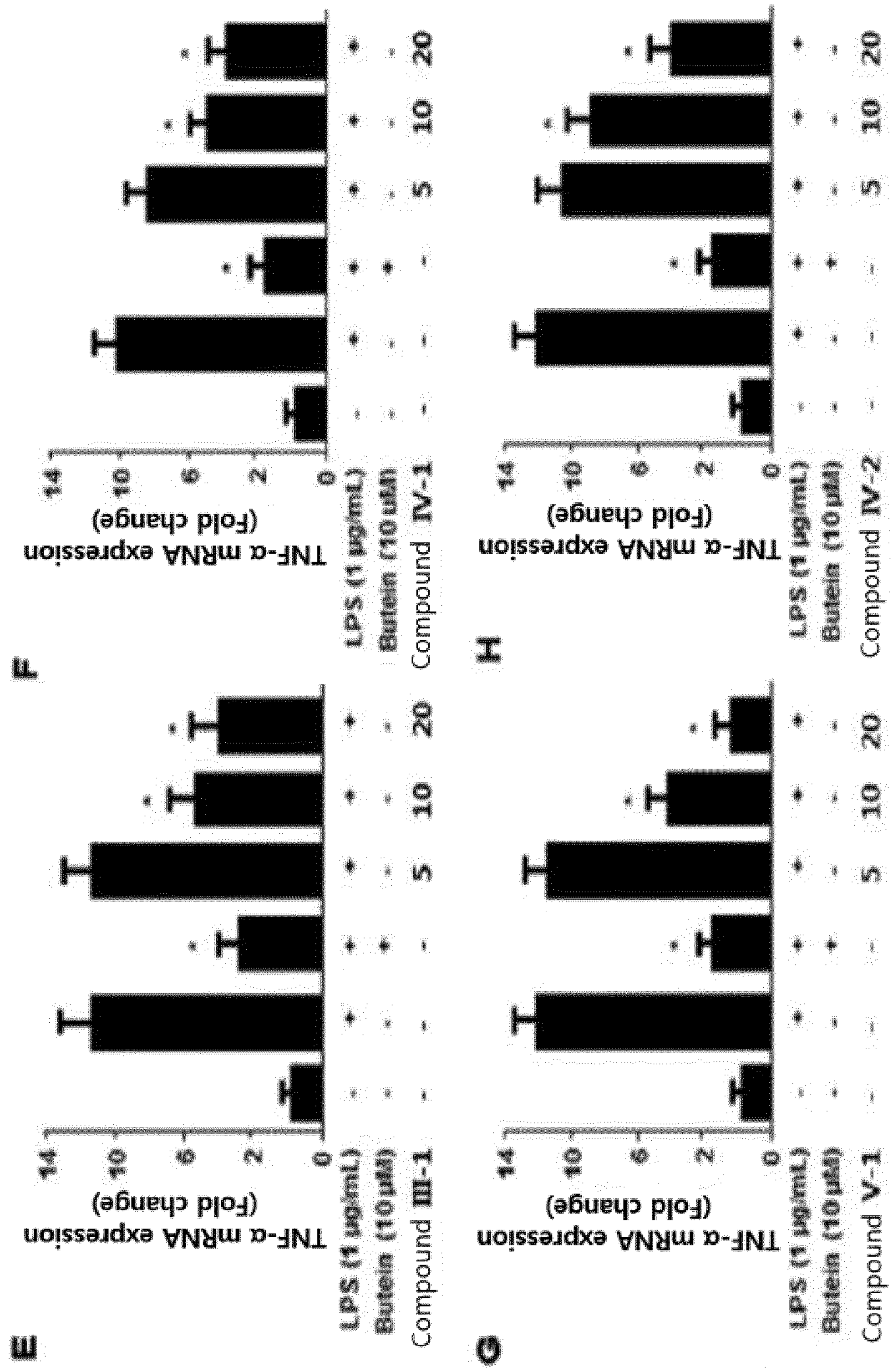
E
TNF-α mRNA expression (Fold change)
14
10
6
2
0
LPS (1 μg/mL)
Butein (10 μM)
Compound III-1
5
10
20
F
TNF-α mRNA expression (Fold change)
LPS (1 μg/mL)
Butein (10 μM)
Compound IV-1
G
TNF-α mRNA expression (Fold change)
LPS (1 μg/mL)
Butein (10 μM)
Compound V-1
H
TNF-α mRNA expression (Fold change)
LPS (1 μg/mL)
Butein (10 μM)
Compound IV-2

[Figure 14c]

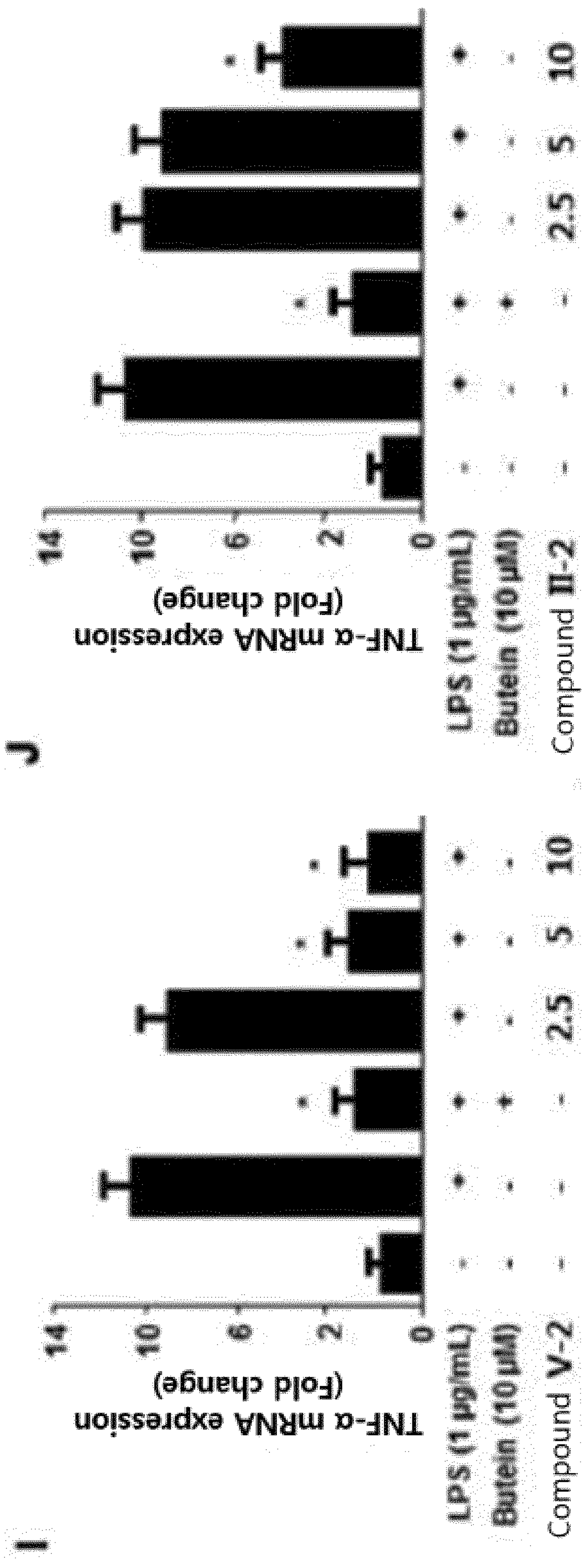
I
J
TNF-α mRNA expression
(Fold change)
0
2
6
10
14
LPS (1 μg/mL)
Butein (10 μM)
Compound V-2
Compound II-2
2.5
5
10

[Figure 15a]

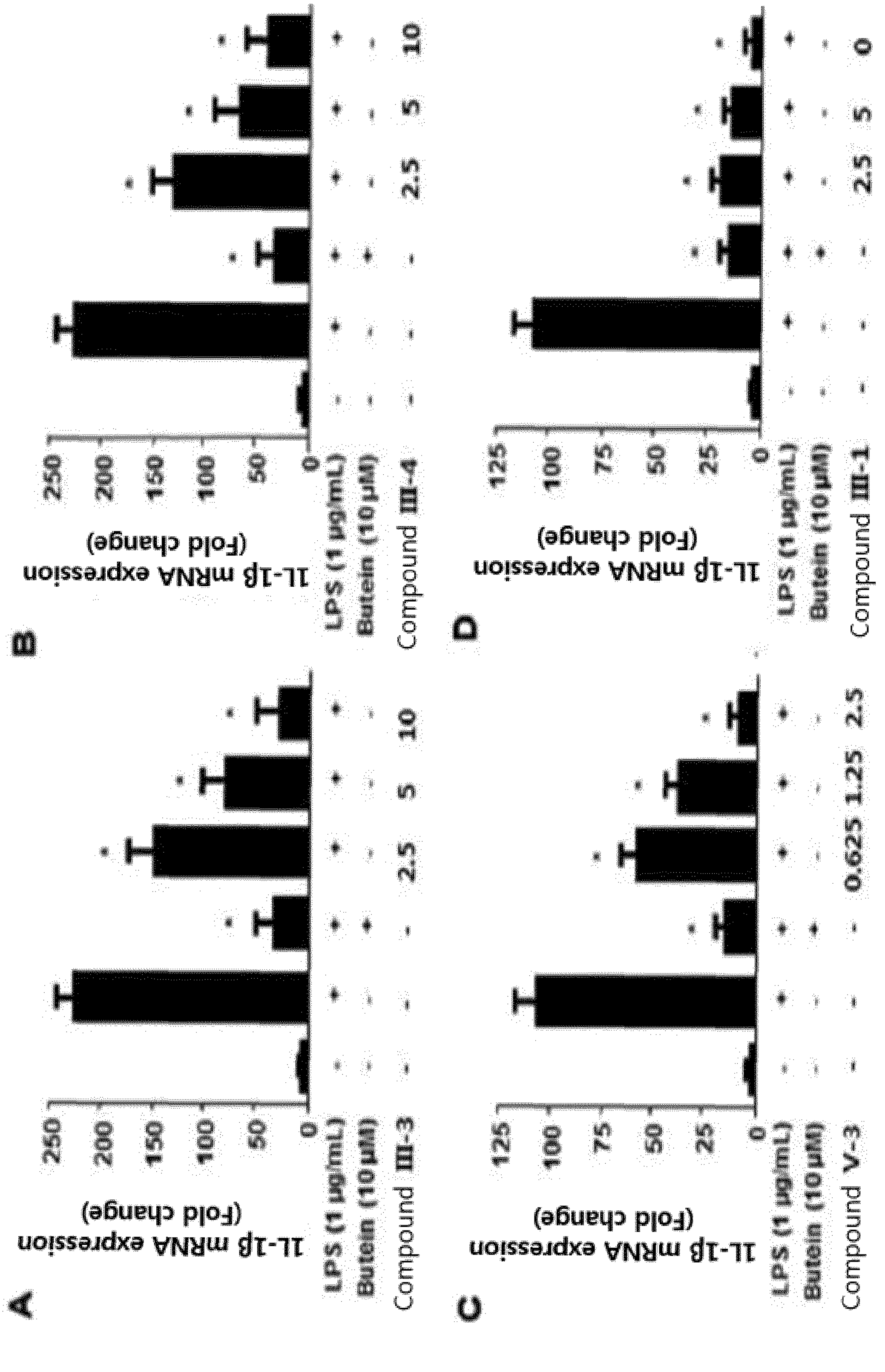
A
1L-1β mRNA expression (Fold change)
LPS (1 μg/mL)
Butein (10 μM)
Compound Ⅲ-3
B
1L-1β mRNA expression (Fold change)
LPS (1 μg/mL)
Butein (10 μM)
Compound Ⅲ-4
C
1L-1β mRNA expression (Fold change)
LPS (1 μg/mL)
Butein (10 μM)
Compound V-3
D
1L-1β mRNA expression (Fold change)
LPS (1 μg/mL)
Butein (10 μM)
Compound Ⅲ-1

[Figure 15b]

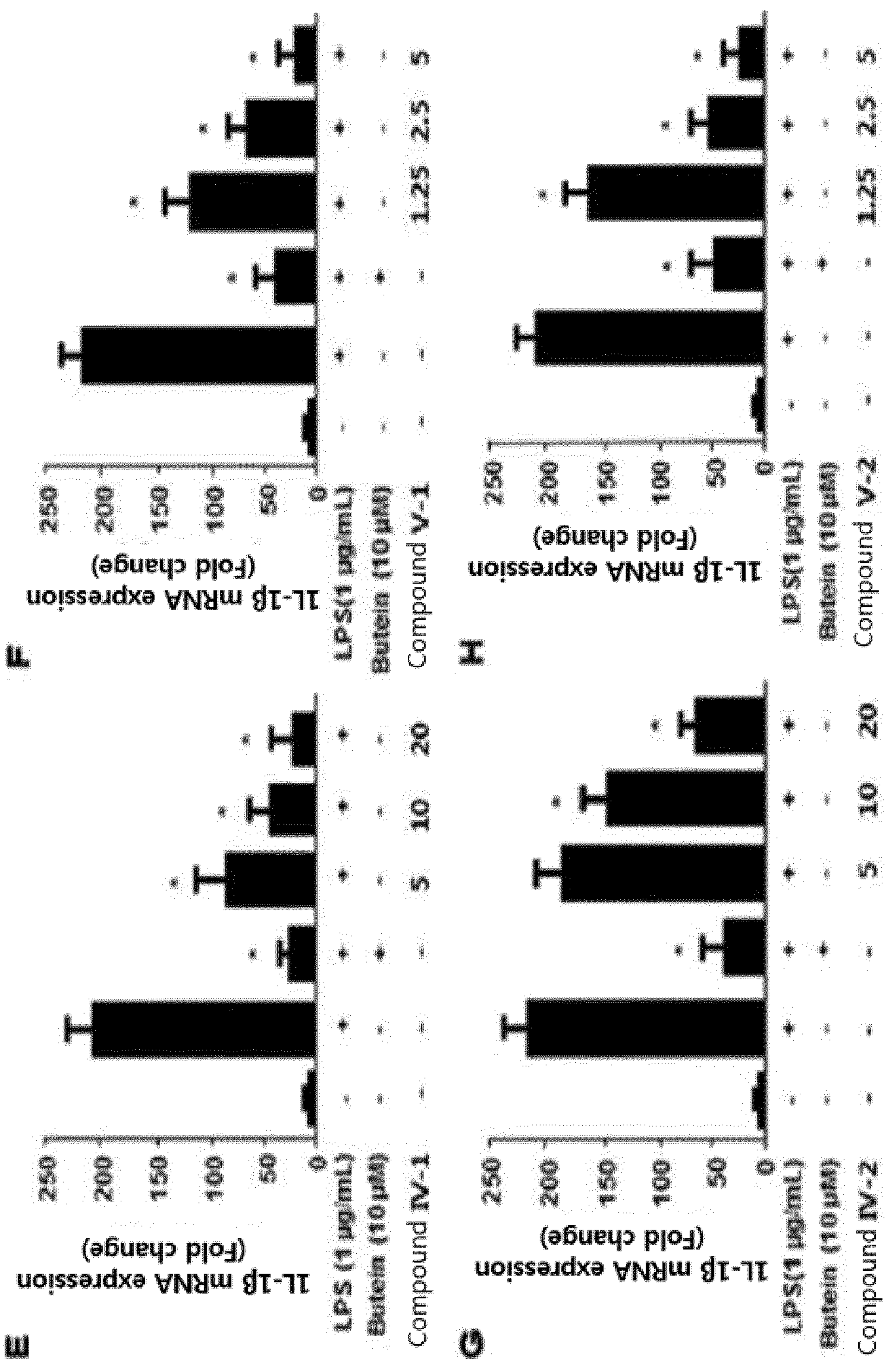
E
1L-1β mRNA expression
(Fold change)
250
200
150
100
50
0
LPS (1 μg/mL)
Butein (10 μM)
Compound IV-1
5
10
20
F
1L-1β mRNA expression
(Fold change)
LPS(1 μg/mL)
Butein (10 μM)
Compound V-1
1.25
2.5
5
G
1L-1β mRNA expression
(Fold change)
LPS(1 μg/mL)
Butein (10 μM)
Compound IV-2
5
10
20
H
1L-1β mRNA expression
(Fold change)
LPS(1 μg/mL)
Butein (10 μM)
Compound V-2
1.25
2.5
5

[Figure 15c]

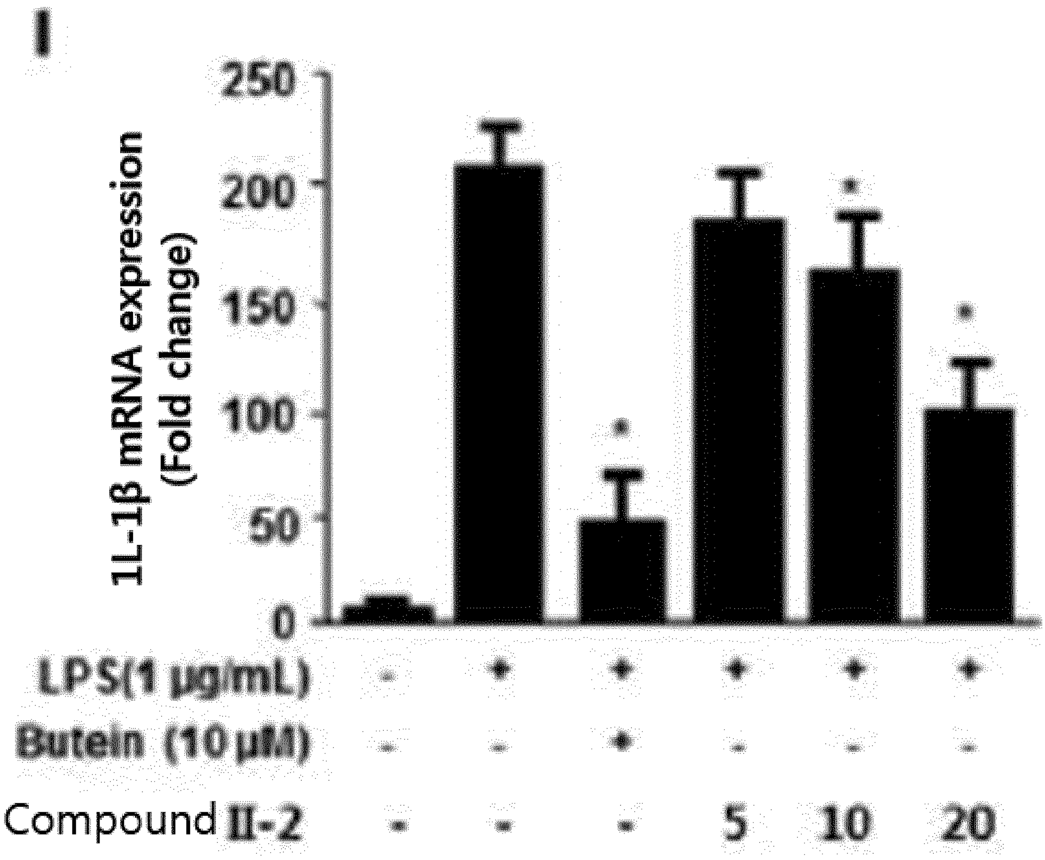
I
1L-1β mRNA expression
(Fold change)
250
200
150
100
50
0
LPS(1 μg/mL) - + + + + +
Butein (10 μM) - - + - - -
Compound II-2 - - - 5 10 20

[Figure 16a]

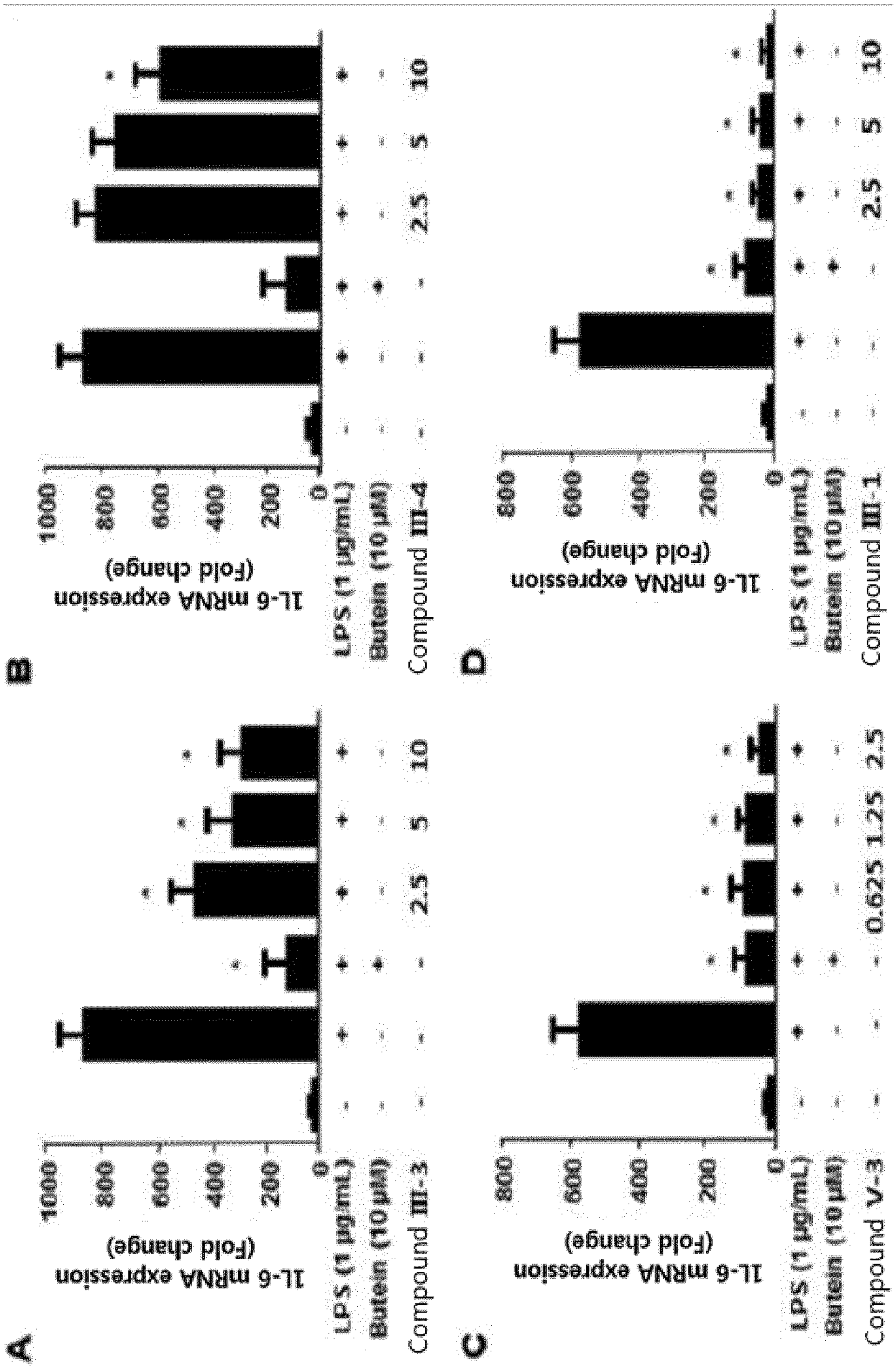
A
B
C
D
IL-6 mRNA expression
(Fold change)
LPS (1 μg/mL)
Butein (10 μM)
Compound III-3
Compound III-4
Compound V-3
Compound III-1
2.5
5
10
0.625
1.25

[Figure 16b]

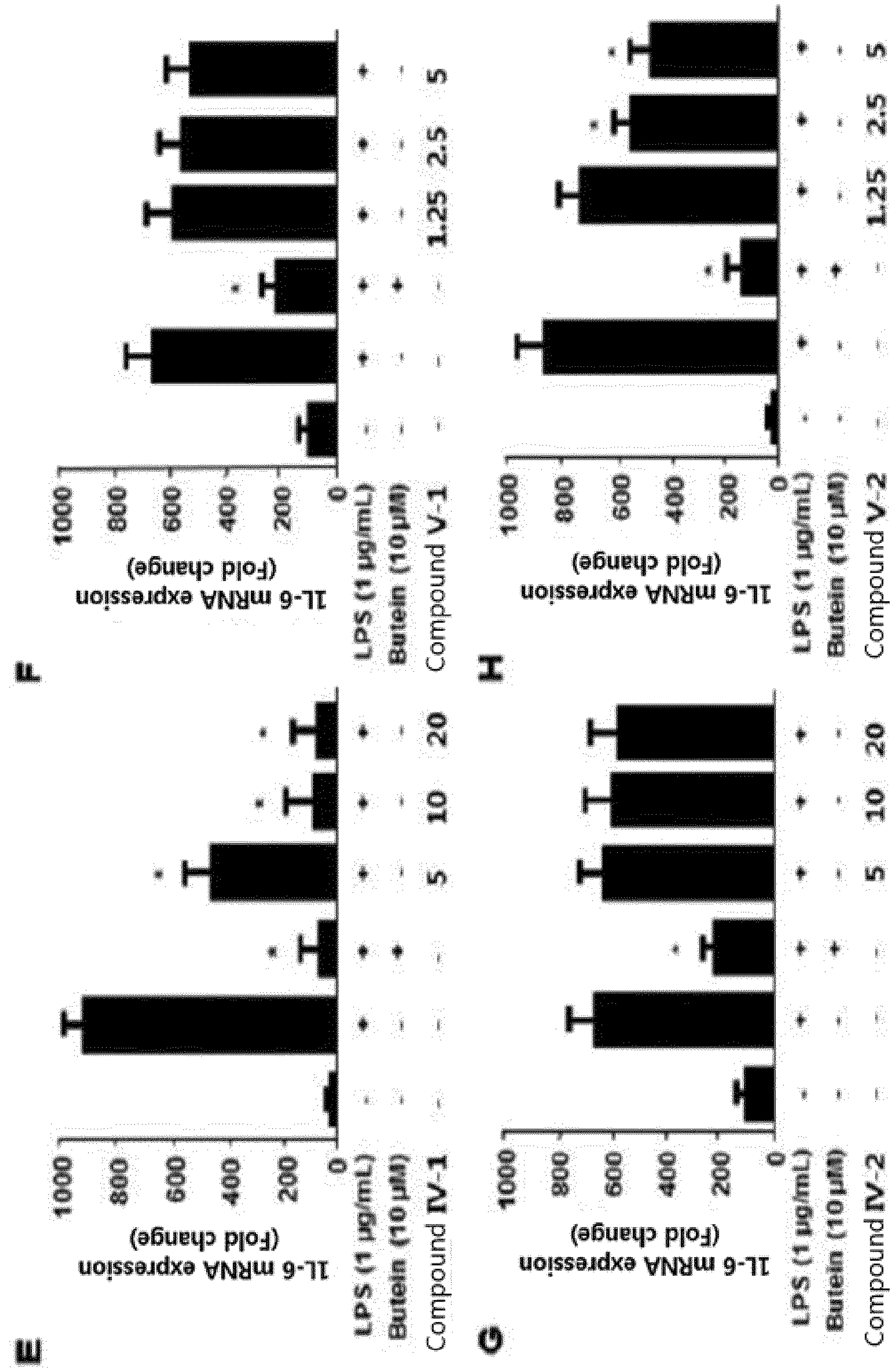
E
F
G
H
IL-6 mRNA expression (Fold change)
0
200
400
600
800
1000
LPS (1 μg/mL)
Butein (10 μM)
Compound IV-1
Compound V-1
Compound IV-2
Compound V-2
5
10
20
1.25
2.5

[Figure 16c]

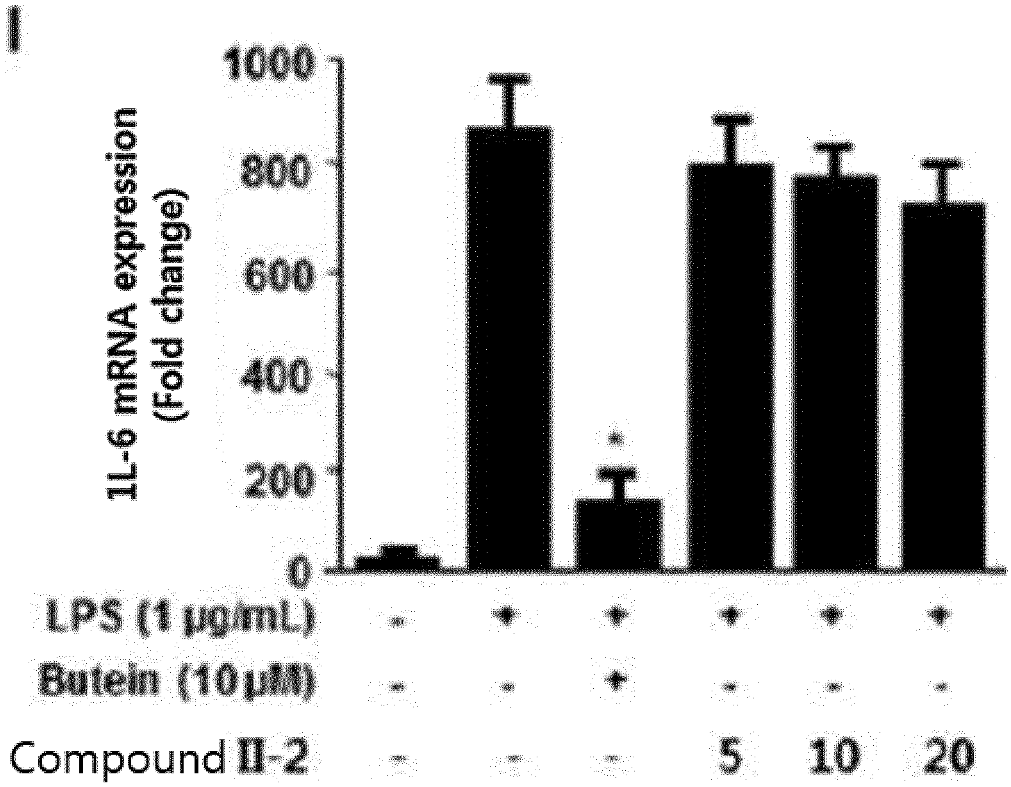
I
1000
800
600
400
200
0
1L-6 mRNA expression
(Fold change)
LPS (1 µg/mL) - + + + + +
Butein (10 µM) - - + - - -
Compound II-2 - - - 5 10 20

[Figure 17a]

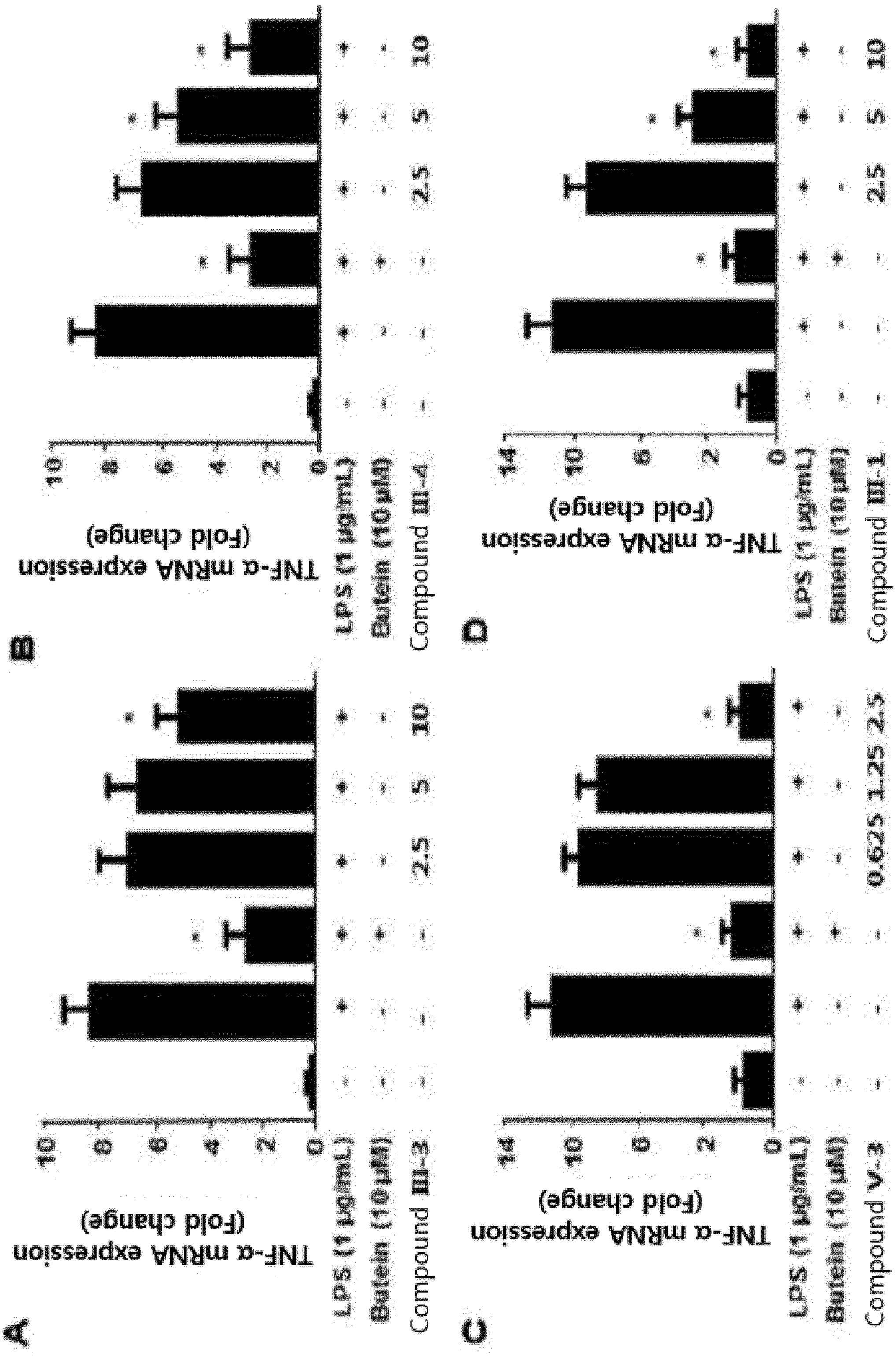
A
TNF-α mRNA expression (Fold change)
LPS (1 µg/mL)
Butein (10 µM)
Compound III-3
B
TNF-α mRNA expression (Fold change)
LPS (1 µg/mL)
Butein (10 µM)
Compound III-4
C
TNF-α mRNA expression (Fold change)
LPS (1 µg/mL)
Butein (10 µM)
Compound V-3
D
TNF-α mRNA expression (Fold change)
LPS (1 µg/mL)
Butein (10 µM)
Compound III-1

[Figure 17b]

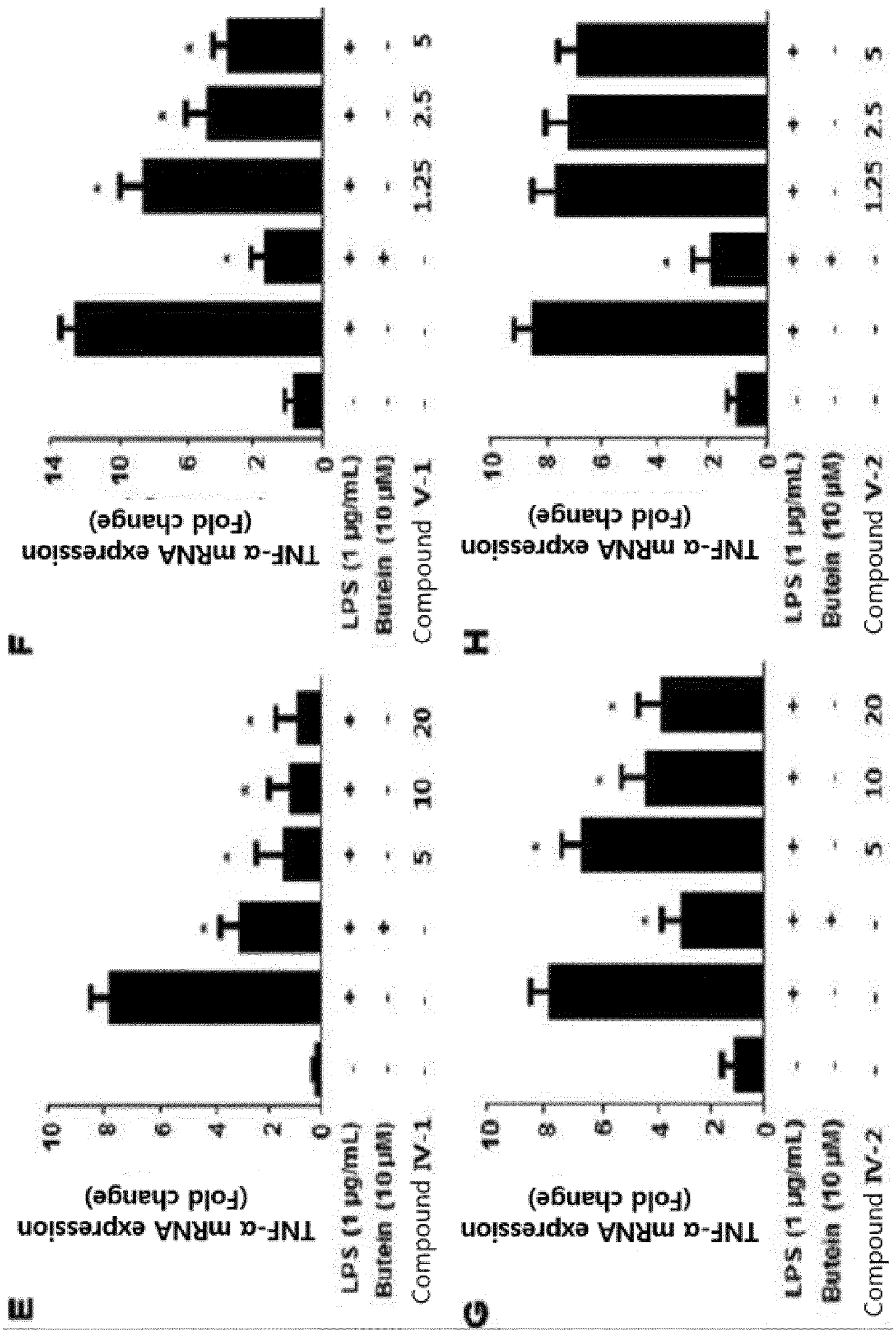
E
TNF-α mRNA expression (Fold change)
LPS (1 μg/mL)
Butein (10 μM)
Compound IV-1
F
TNF-α mRNA expression (Fold change)
LPS (1 μg/mL)
Butein (10 μM)
Compound V-1
G
TNF-α mRNA expression (Fold change)
LPS (1 μg/mL)
Butein (10 μM)
Compound IV-2
H
TNF-α mRNA expression (Fold change)
LPS (1 μg/mL)
Butein (10 μM)
Compound V-2

[Figure 17c]

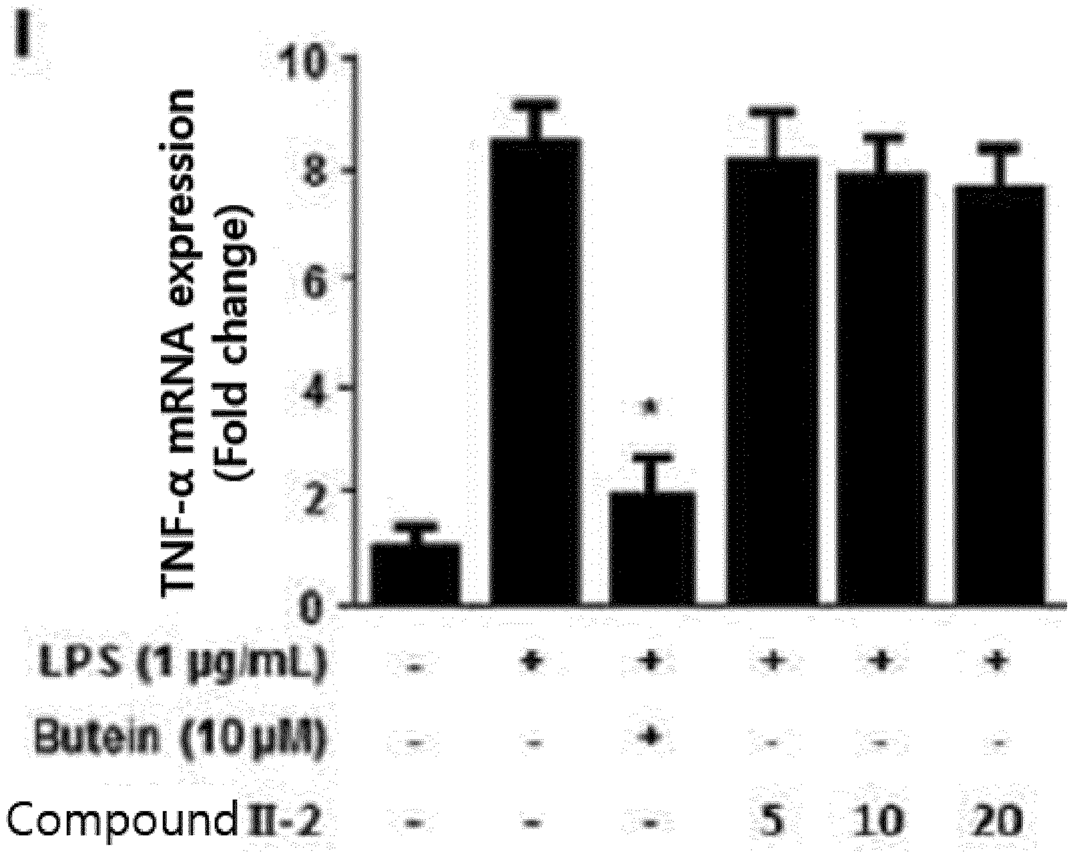
I
TNF-α mRNA expression
(Fold change)
0
2
4
6
8
10
*
LPS (1 μg/mL) - + + + + +
Butein (10 μM) - - + - - -
Compound II-2 - - - 5 10 20

[Figure 18a]

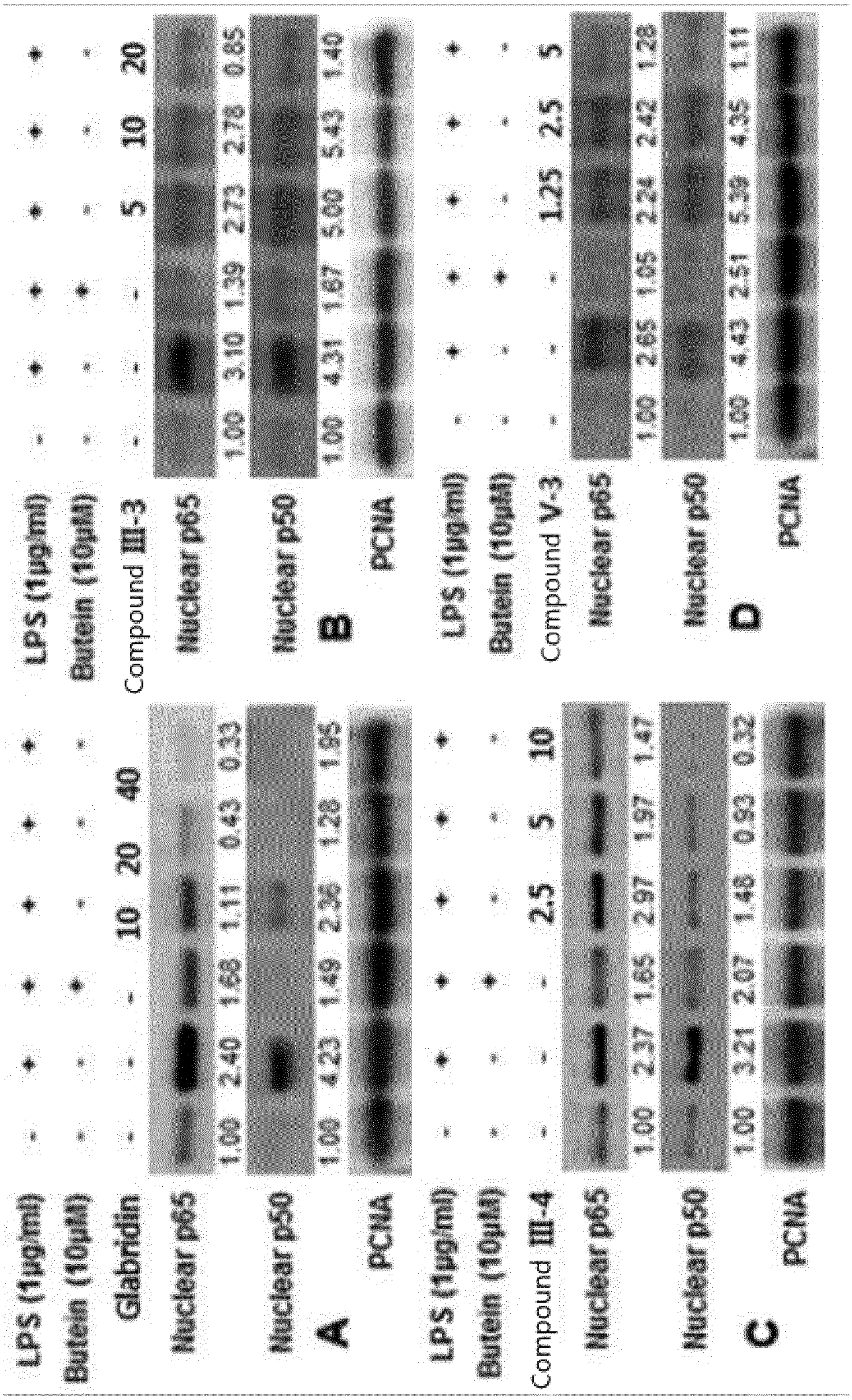
A
LPS (1μg/ml) - + + + + +
Butein (10μM) - - + - - -
Glabridin - - - 10 20 40
Nuclear p65
1.00 2.40 1.68 1.11 0.43 0.33
Nuclear p50
1.00 4.23 1.49 2.36 1.28 1.95
PCNA
B
LPS (1μg/ml) - + + + + +
Butein (10μM) - - + - - -
Compound III-3 - - - 5 10 20
Nuclear p65
1.00 3.10 1.39 2.73 2.78 0.85
Nuclear p50
1.00 4.31 1.67 5.00 5.43 1.40
PCNA
C
LPS (1μg/ml) - + + + + +
Butein (10μM) - - + - - -
Compound III-4 - - - 2.5 5 10
Nuclear p65
1.00 2.37 1.65 2.97 1.97 1.47
Nuclear p50
1.00 3.21 2.07 1.48 0.93 0.32
PCNA
D
LPS (1μg/ml) - + + + + +
Butein (10μM) - - + - - -
Compound V-3 - - - 1.25 2.5 5
Nuclear p65
1.00 2.65 1.05 2.24 2.42 1.28
Nuclear p50
1.00 4.43 2.51 5.39 4.35 1.11
PCNA

[Figure 18b]

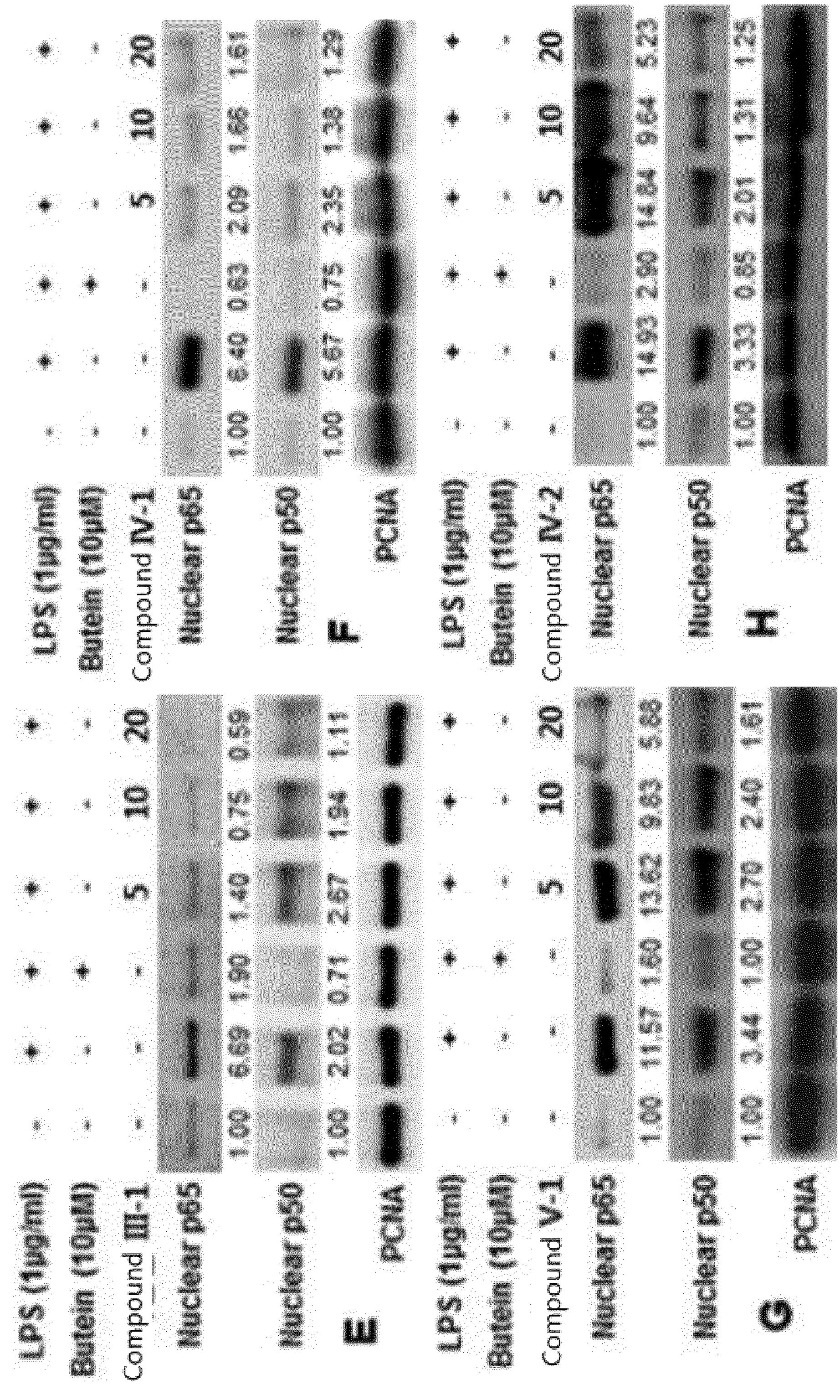
E
LPS (1μg/ml)
Butein (10μM)
Compound Ⅲ-1
- 5 10 20
Nuclear p65
1.00 6.69 1.90 1.40 0.75 0.59
Nuclear p50
1.00 2.02 0.71 2.67 1.94 1.11
PCNA
F
LPS (1μg/ml)
Butein (10μM)
Compound Ⅳ-1
- 5 10 20
Nuclear p65
1.00 6.40 0.63 2.09 1.66 1.61
Nuclear p50
1.00 5.67 0.75 2.35 1.38 1.29
PCNA
G
LPS (1μg/ml)
Butein (10μM)
Compound Ⅴ-1
- 5 10 20
Nuclear p65
1.00 11.57 1.60 13.62 9.83 5.88
Nuclear p50
1.00 3.44 1.00 2.70 2.40 1.61
PCNA
H
LPS (1μg/ml)
Butein (10μM)
Compound Ⅳ-2
- 5 10 20
Nuclear p65
1.00 14.93 2.90 14.84 9.64 5.23
Nuclear p50
1.00 3.33 0.85 2.01 1.31 1.25
PCNA

[Figure 18c]

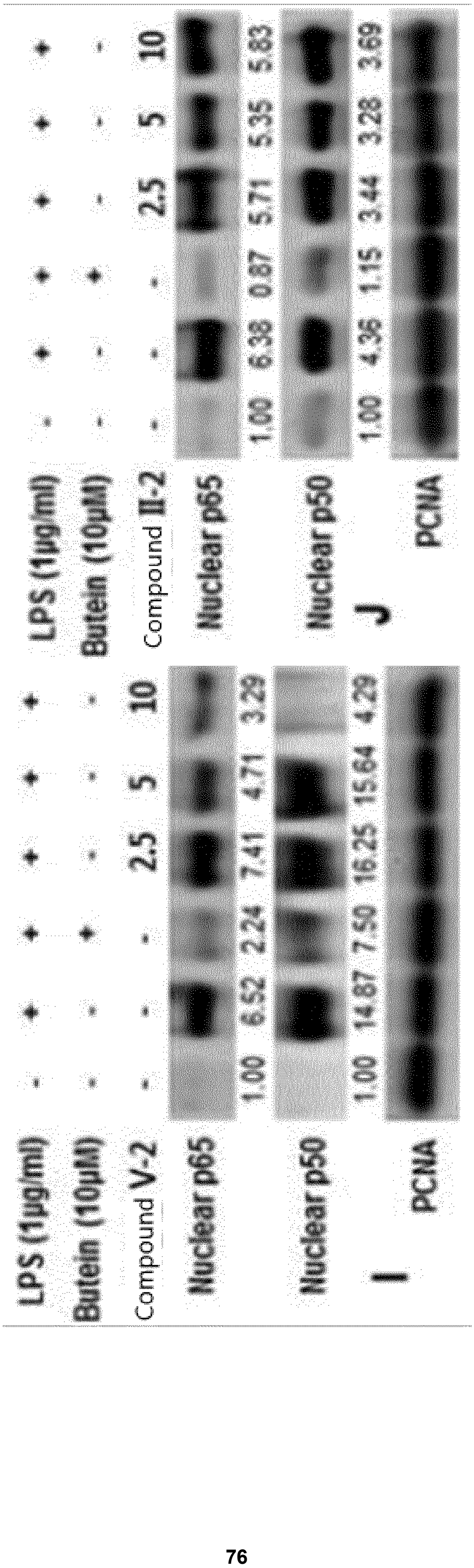
I
LPS (1μg/ml) - + + + + +
Butein (10μM) - - + - - -
Compound V-2 - - - 2.5 5 10
Nuclear p65
1.00 6.52 2.24 7.41 4.71 3.29
Nuclear p50
1.00 14.87 7.50 16.25 15.64 4.29
PCNA
J
LPS (1μg/ml) - + + + + +
Butein (10μM) - - + - - -
Compound II-2 - - - 2.5 5 10
Nuclear p65
1.00 6.38 0.87 5.71 5.35 5.83
Nuclear p50
1.00 4.36 1.15 3.44 3.28 3.69
PCNA

[Figure 19a]

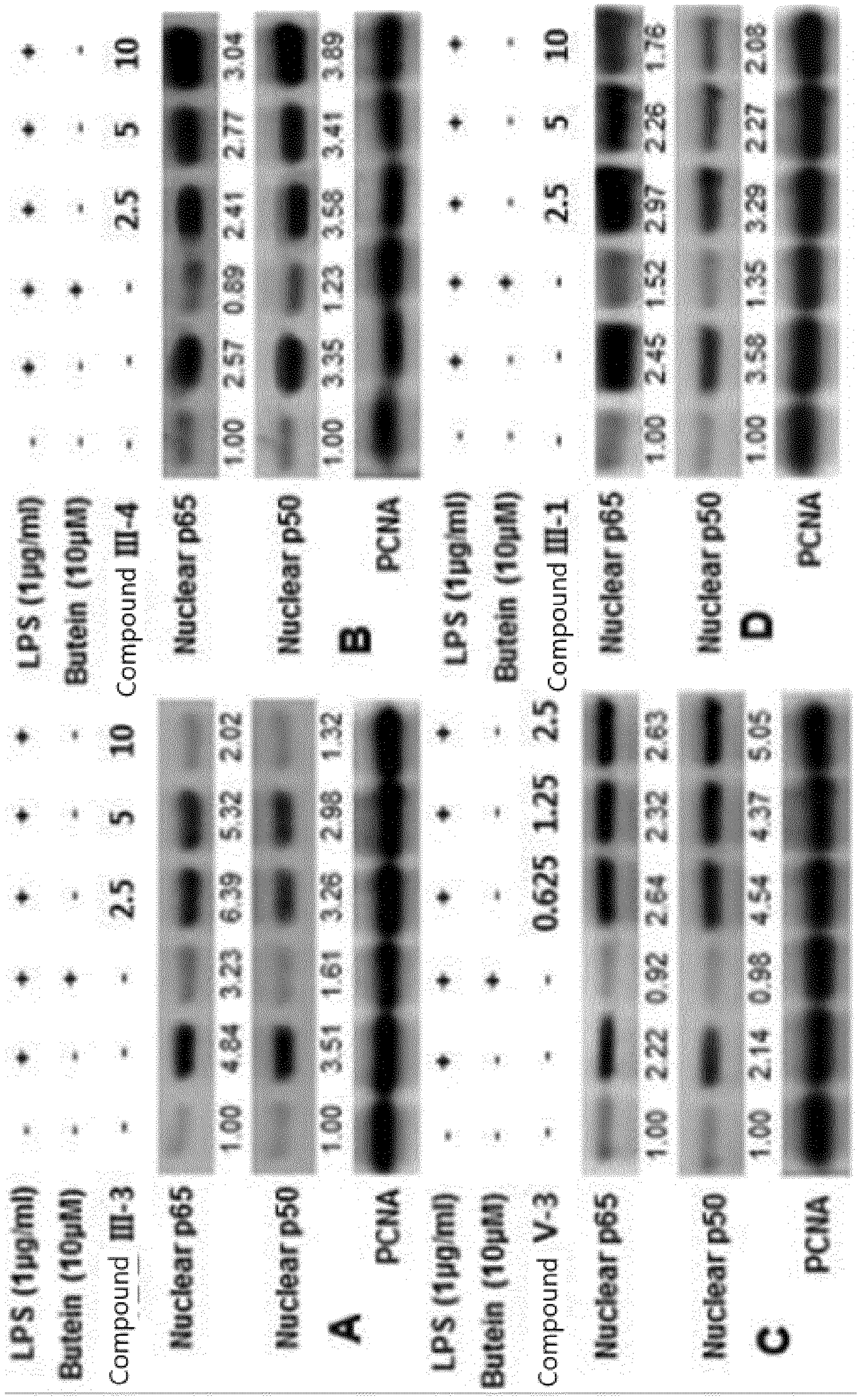
A
LPS (1μg/ml)
Butein (10μM)
Compound Ⅲ-3
2.5 5 10
Nuclear p65
1.00 4.84 3.23 6.39 5.32 2.02
Nuclear p50
1.00 3.51 1.61 3.26 2.98 1.32
PCNA
B
LPS (1μg/ml)
Butein (10μM)
Compound Ⅲ-4
2.5 5 10
Nuclear p65
1.00 2.57 0.89 2.41 2.77 3.04
Nuclear p50
1.00 3.35 1.23 3.58 3.41 3.89
PCNA
C
LPS (1μg/ml)
Butein (10μM)
Compound V-3
0.625 1.25 2.5
Nuclear p65
1.00 2.22 0.92 2.64 2.32 2.63
Nuclear p50
1.00 2.14 0.98 4.54 4.37 5.05
PCNA
D
LPS (1μg/ml)
Butein (10μM)
Compound Ⅲ-1
2.5 5 10
Nuclear p65
1.00 2.45 1.52 2.97 2.26 1.76
Nuclear p50
1.00 3.58 1.35 3.29 2.27 2.08
PCNA

[Figure 19b]

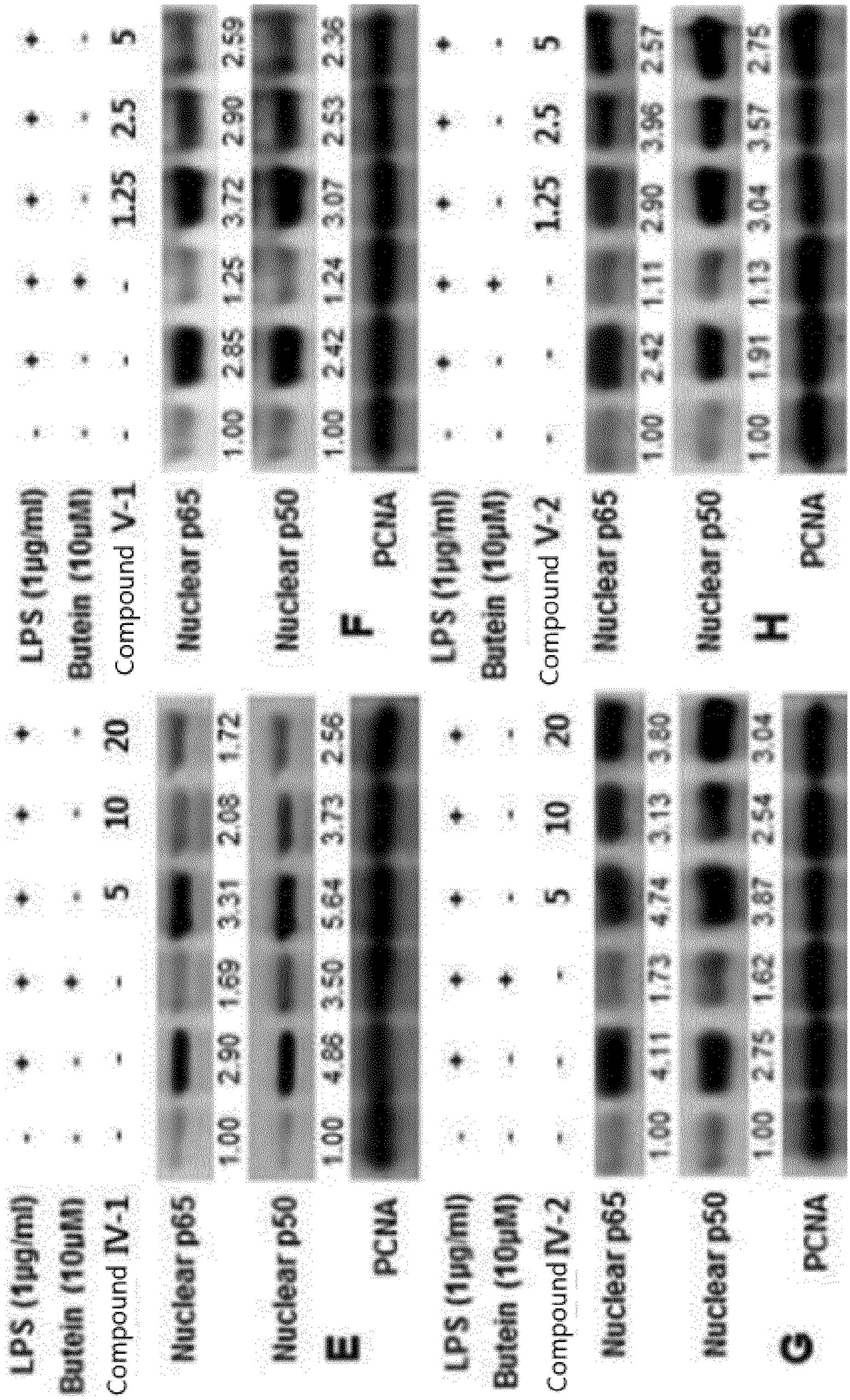

E
LPS (1μg/ml)
Butein (10uM)
Compound IV-1
Nuclear p65
Nuclear p50
PCNA
5
10
20
1.00 2.90 1.69 3.31 2.08 1.72
1.00 4.86 3.50 5.64 3.73 2.56
F
LPS (1μg/ml)
Butein (10uM)
Compound V-1
Nuclear p65
Nuclear p50
PCNA
1.25
2.5
5
1.00 2.85 1.25 3.72 2.90 2.59
1.00 2.42 1.24 3.07 2.53 2.36
G
LPS (1μg/ml)
Butein (10μM)
Compound IV-2
Nuclear p65
Nuclear p50
PCNA
5
10
20
1.00 4.11 1.73 4.74 3.13 3.80
1.00 2.75 1.62 3.87 2.54 3.04
H
LPS (1μg/ml)
Butein (10μM)
Compound V-2
Nuclear p65
Nuclear p50
PCNA
1.25
2.5
5
1.00 2.42 1.11 2.90 3.96 2.57
1.00 1.91 1.13 3.04 3.57 2.75

[Figure 19c]

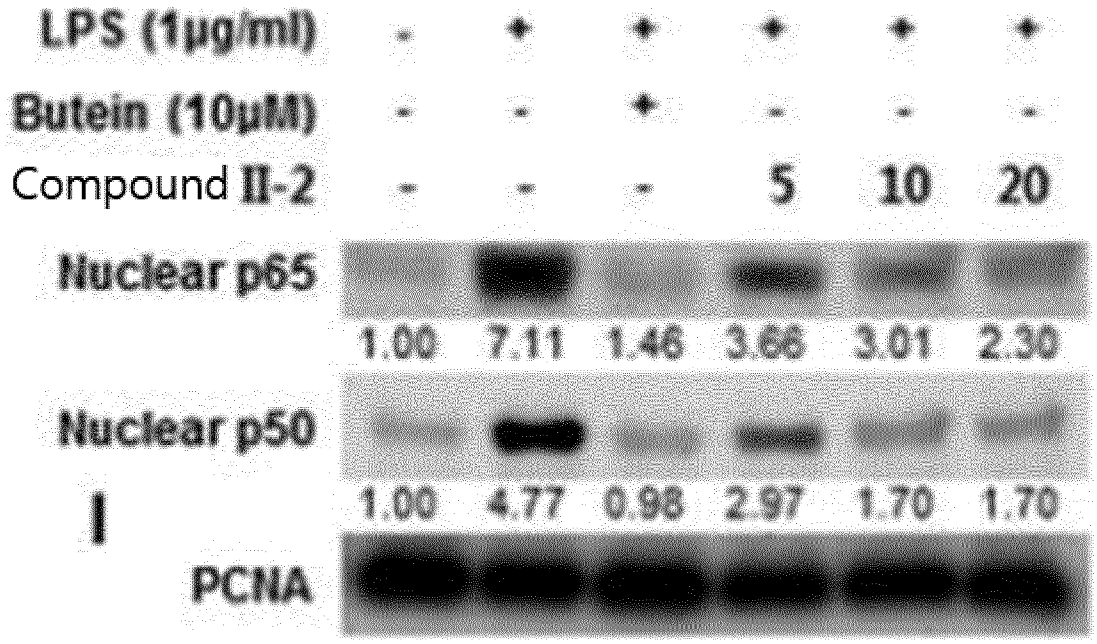
LPS (1μg/ml) - + + + + +
Butein (10μM) - - + - - -
Compound II-2 - - - 5 10 20
Nuclear p65
1.00 7.11 1.46 3.66 3.01 2.30
Nuclear p50
1.00 4.77 0.98 2.97 1.70 1.70
PCNA

**INTERNATIONAL SEARCH REPORT**

International application No.
**PCT/KR2020/002907**

A. CLASSIFICATION OF SUBJECT MATTER

***A61K 31/352(2006.01)i, A61P 25/00(2006.01)i***

According to International Patent Classification (IPC) or to both national classification and IPC

B. FIELDS SEARCHED

Minimum documentation searched (classification system followed by classification symbols)
A61K 31/352; A61K 31/353; A61P 39/00; C07D 493/04; A61P 25/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: neurological disorder, pyrano[2,3-f]chromene derivative, paraoxonase (PON)

C. DOCUMENTS CONSIDERED TO BE RELEVANT

| Category* | Citation of document, with indication, where appropriate, of the relevant passages | Relevant to claim No. |
|---|---|---|
| X | KR 10-2015-0075030 A (ERUM BIOTECHNOLOGIES INC.) 02 July 2015<br>See claims 1, 4; paragraphs [0009], [0010], [0416]-[0419]. | 1-11 |
| A | YU, X.-Q. et al. In vitro and in vivo neuroprotective effect and mechanisms of glabridin, a major active isoflavan from glycyrrhiza glabra (licorice). Life sciences. 2008, vol. 82, pages 68-78<br>See abstract; figure 1. | 1-11 |
| A | CUI, Y.-M. et al. Effect of glabridin from glycyrrhiza glabra on learning and memory in mice. Planta medica. 2008, vol. 74, pages 377-380<br>See abstract; figure 1. | 1-11 |
| A | JP 2006-008604 A (KURARAY CO., LTD.) 12 January 2006<br>See the entire document. | 1-11 |
| A | JP 2006-008606 A (KURARAY CO., LTD.) 12 January 2006<br>See the entire document. | 1-11 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 JUNE 2020 (09.06.2020) | **09 JUNE 2020 (09.06.2020)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**
Information on patent family members

International application No.
**PCT/KR2020/002907**

| Patent document cited in search report | Publication date | Patent family member | Publication date |
|---|---|---|---|
| KR 10-2015-0075030 A | 02/07/2015 | AU 2014-370697 A1 | 16/06/2016 |
| | | AU 2014-370697 B2 | 19/01/2017 |
| | | CA 2929001 A1 | 02/07/2015 |
| | | CA 2929001 C | 20/02/2018 |
| | | CN 105849111 A | 10/08/2016 |
| | | CN 105849111 B | 04/05/2018 |
| | | DK 3098224 T3 | 13/05/2019 |
| | | EP 3098224 A1 | 30/11/2016 |
| | | EP 3098224 B1 | 30/01/2019 |
| | | ES 2718878 T3 | 05/07/2019 |
| | | HR P20190573 T1 | 17/05/2019 |
| | | HU E043902 T2 | 28/10/2019 |
| | | IL 245908 A | 02/08/2016 |
| | | JP 2017-501163 A | 12/01/2017 |
| | | JP 2018-135343 A | 30/08/2018 |
| | | JP 6620096 B2 | 11/12/2019 |
| | | MX 2016008429 A | 23/02/2017 |
| | | NZ 720328 A | 23/02/2018 |
| | | PL 3098224 T3 | 31/07/2019 |
| | | SG 11201604371 A | 28/07/2016 |
| | | TW 201607949 A | 01/03/2016 |
| | | TW I585093 B | 01/06/2017 |
| | | US 2016-0272650 A1 | 22/09/2016 |
| | | US 9783551 B2 | 10/10/2017 |
| | | WO 2015-099392 A1 | 02/07/2015 |
| JP 2006-008604 A | 12/01/2006 | None | |
| JP 2006-008606 A | 12/01/2006 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020180002539
- KR 1020150075030


### Non-patent literature cited in the description


- *Neurochem Research,* 2013, vol. 38, 1809-1818
- *Neurotoxicology,* 2014, vol. 43, 3-9
- *PLOS one,* 2014, vol. 9, e106601
- *Toxicology and Applied Pharmacology,* 2011, vol. 256, 369-378
- *Neurochemical Research,* 2013, vol. 38, 1809-1818

<110> Glaceum, Inc.

<120> PHARMACEUTICAL COMPOSITION FOR THE PREVENTION OR TREATMENT OF NEUROLOGICAL DISEASES

<130> PN190034

<160> 4

<170> KoPatentIn 3.0

<210> 1
<211> 22
<212> DNA
<213> Artificial Sequence

<220>
<223> PON2 Forward

<400> 1
ggtaaacagg tctgcggcct cg 22

<210> 2
<211> 23
<212> DNA
<213> Artificial Sequence

<220>
<223> PON2 Reverse

<400> 2
cctaggagtc acttcccgcc tca 23

<210> 3
<211> 20
<212> DNA
<213> Artificial Sequence

<220>
<223> GAPDH Forward

<400> 3
tgtgtccgtc gtggatctga 20

<210> 4
<211> 22
<212> DNA
<213> Artificial Sequence

<220>
<223> GAPDH Reverse

<400> 4
cctgcttcac caccttcttg at 22